# EUROPEAN PATENT SPECIFICATION

(11) **EP 1 663 303 B1**
(45) Date of publication and mention of the grant of the patent: **04.05.2011**
(21) Application number: 04784029.3
(22) Date of filing: 15.09.2004
(51) Int. Cl.: A61K 39/385, A61K 39/116, A61K 39/00, A61K 39/02, A61K 39/38, A61K 39/09

(54) **IMMUNOGENIC COMPOSITIONS FOR STREPTOCOCCUS AGALACTIAE**
IMMUNOGENE ZUSAMMENSETZUNGEN FÜR STREPTOCOCCUS AGALACTIAE
COMPOSITIONS IMMUNOGENES POUR STREPTOCOCCUS AGALACTIAE

(30) Priority: 15.09.2003 WO PCT/US03/29167; 26.02.2004 US 548789 P
(43) Date of publication of application: 07.06.2006
(73) Proprietor: Novartis Vaccines and Diagnostics, Inc., Emeryville, CA 94608-2916 (US)
(72) Inventor: Telford, John L. Novartis Vaccines & Diagnostics, SRL, 53100 Siena (IT); Grandi, Guido Novartis Vaccines & Diagnostics, SRL, 53100 Siena (IT); Margarit Y Ros, Immaculada Novartis Vaccines & Diagnostics, SRL, 53100 Siena (IT); Maione, Domenico Novartis Vaccines & Diagnostics, SRL, 53100 Siena (IT)
(74) Representative: Marshall, Cameron John
(86) International application number: PCT/US2004/030032
(87) International publication number: WO 2005/028618

(56) References cited:
- WO-A2-2004/041157
- DATABASE EPO Proteins [Online] 17 February 2003 (2003-02-17), "Sequence 4310 from Patent WO02092818." XP002411767 retrieved from EBI accession no. EPOP:AX606381 Database accession no. AX606381 & WO 02/092818 A2 (PASTEUR INSTITUT [FR]; CENTRE NAT RECH SCIENT [FR]; GLASER PHILIPPE [F) 21 November 2002 (2002-11-21)
- DATABASE EPO Proteins [Online] 2 February 2004 (2004-02-02), "Sequence 8780 from Patent WO0234771." XP002411768 retrieved from EBI accession no. EPOP:CQ651823 Database accession no. CQ651823 & WO 02/34771 A2 (CHIRON SPA [IT]; INST GENOMIC RES [US]; TELFORD JOHN [IT]; MASIGNANI V) 2 May 2002 (2002-05-02)
- DATABASE Geneseq [Online] 20 May 2004 (2004-05-20), "Streptococcus agalactiae ORF SAG0645-related protein 1." XP002411769 retrieved from EBI accession no. GSP:ADL00034 Database accession no. ADL00034 & WO 2004/018646 A (CHIRON CORP [US]; INST GENOMIC RES [US]; TETTELIN HERVE [US]; MASIGNAN) 4 March 2004 (2004-03-04)

## Description

### FIELD OF THE INVENTION

The invention relates to an immunogenic antigen derived from *Streptococcus agalactiae* ("GBS") and its use in combinations with other GBS antigens to provide for broader coverage among different GBS strains. In particular, the invention relates to a composition as recited in the claims comprising a combination of two or more GBS antigens, wherein the combination includes GBS 80 or a fragment thereof. The combination includes GBS 80 and at least one other GBS antigen. For example, the combination may include GBS 80 and up to thirteen GBS antigens. In a preferred embodiment, the combination may include GBS 80 and up to ten GBS antigens. In a more preferred embodiment, the combination may include GBS 80 and up to five GBS antigens. In one embodiment, the combination may consist of two to thirteen GBS antigens selected from an antigen group consisting of GBS 80, GBS 91, GBS 104, GBS 184, GBS 276, GBS 305, GBS 322, GBS 330, GBS 338, GBS 361, GBS 404, GBS 690, and GBS 691. Preferably, the combination includes GBS 80 in combination with one or more of GBS 104 and GBS 322.

### BACKGROUND OF THE INVENTION

GBS has emerged in the last 20 years as the major cause of neonatal sepsis and meningitis that affect 0.5 - 3 per 1000 live births, and an important cause of morbidity among the older age group affecting 5 - 8 per 100,000 of the population. Current disease management strategies rely on intrapartum antibiotics and neonatal monitoring which have reduced neonatal case mortality from >50% in the 1970's to less than 10% in the 1990's. Nevertheless, there is still considerable morbidity and mortality and the management is expensive. 15 - 35% of pregnant women are asymptomatic carriers and at high risk of transmitting the disease to their babies. Risk of neonatal infection is associated with low serotypes specific maternal antibodies and high titers are believed to be protective. In addition, invasive GBS disease is increasingly recognized in elderly adults with underlying disease such as diabetes and cancer.

The "B" in "GBS" refers to the Lancefield classification, which is based on the antigenicity of a carbohydrate which is soluble in dilute acid and called the C carbohydrate. Lancefield identified 13 types of C carbohydrate, designated A to O, that could be serologically differentiated. The organisms that most commonly infect humans are found in groups A, B, D, and G. Within group B, strains can be divided into at least 9 serotypes (Ia, Ib, Ia/c, II, III, IV, V, VI, VII and VIII) based on the structure of their polysaccharide capsule. In the past, serotypes Ia, Ib, II, and III were equally prevalent in normal vaginal carriage and early onset sepsis in newborns. Type V GBS has emerged as an important cause of GBS infection in the USA, however, and strains of types VI and VIII have become prevalent among Japanese women.

The genome sequence of a serotype V strain 2603 V/R has been published (Ref. 1) and various polypeptides for use a vaccine antigens have been identified (Ref. 2). The vaccines currently in clinical trials, however, are based on polysaccharide antigens. These suffer from serotype-specificity and poor immunogenicity, and so there is a need for effective vaccines against *S.agalactiae* infection.

It is an object of the invention to provide further and improved compositions for providing immunity against GBS disease and/or infection. The compositions are based on a combination of two or more (e.g., three or more) GBS antigens.

### SUMMARY OF THE INVENTION

Applicants have discovered that an immunogenic GBS antigen, GBS 80, is particularly suitable for immunization purposes, especially when used in combination with other GBS antigens. The combination may include GBS 80 and at least one other GBS antigen or up to thirteen other GBS antigens as recited in the claims. In a preferred embodiment, the combination may include GBS 80 and up to 10 GBS antigens. In a more preferred embodiment, the combination includes GBS 80 and up to five GBS antigens. In particular, the invention relates to a composition comprising a combination of two or more GBS antigens, wherein the combination includes GBS 80 or a fragment thereof. In one embodiment, the combination may consist of two to thirteen GBS antigens selected from the group consisting of GBS 80, GBS 91, GBS 104, GBS 184, GBS 276, GBS 305, GBS 322, GBS 330, GBS 338, GBS 361, GBS 404, GBS 690, and GBS 691. Preferably, the combination consists of GBS 80, GBS 104 and GBS 322.

Instead of the full length antigen, the combination may comprise an immunogenic fragment of the selected GBS antigen and/or a polypeptide sequence having sequence identity to the selected antigen.

Preferably, the combination of GBS antigens consists of three, four, five, six, seven, eight, nine, or ten GBS antigens. Still more preferably, the combination of GBS antigens consists of three, four, or five GBS antigens.

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, Mack Publishing Company, Easton, Pa., 19th Edition (1995); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S. ed., CRC Press, 1997); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

All publications, patents and patent applications cited herein, are hereby incorporated by reference in their entireties.

### GBS Antigens

As discussed above, the invention provides an immunogenic composition as recited in the claims comprising a combination of two or more GBS antigens, wherein said combination includes GBS 80 or a fragment thereof.

The combinations of GBS antigens may include polypeptide fragments of the identified GBS antigens. The length of the fragment may vary depending on the amino acid sequence of the specific GBS antigen, but the fragment is preferably at least 7 consecutive amino acids, (*e.g*. 8, 10, 12, 14, 16, 18, 20, 25, 30, 35, 40, 50, 60, 70, 80, 90, 100, 150, 200 or more). Preferably the fragment comprises one or more epitopes from the sequence. Other preferred fragments include (1) the N-terminal signal peptides of each identified GBS antigen, (2) the identified GBS antigens without their N-terminal signal peptides, and (3) each identified GBS antigen wherein up to 10 amino acid residues (e.g. 1, 2, 3, 4, 5, 6, 7, 8, 9, 10, 15, 20, 25 or more) are deleted from the N-terminus and/or the C-terminus *e.g*. the N-terminal amino acid residue may be deleted. Other fragments omit one or more domains of the protein (*e.g*. omission of a signal peptide, of a cytoplasmic domain, of a transmembrane domain, or of an extracellular domain).

The combinations of GBS antigens may include polypeptide sequences having sequence identity to the identified GBS antigens. The degree of sequence identity may vary depending on the amino acid sequence (a) in question, but is preferably greater than 80% (*e.g*. 85%, 90%, 91%, 92%, 93%, 94%, 95%, 96%, 97%, 98%, 99%, 99.5% or more). Polypeptides having sequence identity include homologs, orthologs, allelic variants and functional mutants of the identified GBS antigens. Typically, 50% identity or more between two proteins is considered to be an indication of functional equivalence. Identity between proteins is preferably determined by the Smith-Watennan homology search algorithm as implemented in the MPSRCH program (Oxford Molecular), using an affinity gap search with parameters *gap open penalty=12* and *gap extension penalty=1.*

The polypeptides can, of course, be prepared by various means (*e.g*. recombinant expression, purification from GBS, chemical synthesis *etc*.) and in various forms (*e.g.* native, fusions, glycosylated, non-glycosylated *etc.*)*.* They are preferably prepared in substantially pure form (*i.e*. substantially free from other streptococcal or host cell proteins) or substantially isolated form.

### GBS 80

As discussed above, the invention relates to the use of GBS 80 in synergistic combination with other GBS antigens. GBS 80 refers to a putative cell wall surface anchor family protein. Nucleotide and amino acid sequence of GBS 80 sequenced from serotype V isolated strain 2603 V/R are set forth in Ref. 2 as SEQ ID 8779 and SEQ lD 8780. These sequences are also set forth below as SEQ ID NOS 1 and 2:
**SEQ ID NO.1** 
ATGAAATTATCGAAGAAGTTATTGTTTTCGGCTGCTGTTTTAACAATGGTGGCGGGGTCAACTGTTGA
ACCAGTAGCTCAGTTTGCGACTGGAATGAGTATTGTAAGAGCTGCAGAAGTGTCACAAGAACGCCCAG
CGAAAACAACAGTAAATATCTATAAATTACAAGCTGATAGTTATAAATCGGAAATTACTTCTAATGGT
GGTATCGAGAATAAAGACGGCGAAGTAATATCTAACTATGCTAAACTTGGTGACAATGTAAAAGGTTT
GCAAGGTGTACAGTTTAAACGTTATAAAGTCAAGACGGATATTTCTGTTGATGAATTGAAAAAATTGA
CAACAGTTGAAGCAGCAGATGCAAAAGTTGGAACGATTCTTGAAGAAGGTGTCAGTCTACCTCAAAAA
ACTAATGCTCAAGGTTTGGTCGTCGATGCTCTGGATTCAAAAAGTAATGTGAGATACTTGTATGTAGA
AGATTTAAAGAATTCACCTTCAAACATTACCAAAGCTTATGCTGTACCGTTTGTGTTGGAATTACCAG
TTGCTAACTCTACAGGTACAGGTTTCCTTTCTGAAATTAATATTTACCCTAAAAACGTTGTAACTGAT
GAACCAAAAACAGATAAAGATGTTAAAAAATTAGGTCAGGACGATGCAGGTTATACGATTGGTGAAGA
ATTCAAATGGTTCTTGAAATCTACAATCCCTGCCAATTTAGGTGACTATGAAAAATTTGAAATTACTG
ATAAATTTGCAGATGGCTTGACTTATAAATCTGTTGGAAAAATCAAGATTGGTTCGAAAACACTGAAT
AGAGATGAGCACTACACTATTGATGAACCAACAGTTGATAACCAAAATACATTAAAAATTACGTTTAA
ACCAGAGAAATTTAAAGAAATTGCTGAGCTACTTAAAGGAATGACCCTTGTTAAAAATCAAGATGCTC
TTGATAAAGCTACTGCAAATACAGATGATGCGGCATTTTTGGAAATTCCAGTTGCATCAACTATTAAT
GAAAAAGCAGTTTTAGGAAAAGCAATTGAAAATACTTTTGAACTTCAATATGACCATACTCCTGATAA
AGCTGACAATCCAAAACCATCTAATCCTCCAAGAAAACCAGAAGTTCATACTGGTGGGAAACGATTTG
TAAAGAAAGACTCAACAGAAACACAAACACTAGGTGGTGCTGAGTTTGATTTGTTGGCTTCTGATGGG
ACAGCAGTAAAATGGACAGATGCTCTTATTAAAGCGAATACTAATAAAAACTATATTGCTGGAGAAGC
TGTTACTGGGCAACCAATCAAATTGAAATCACATACAGACGGTACGTTTGAGATTAAAGGTTTGGCTT
ATGCAGTTGATGCGAATGCAGAGGGTACAGCAGTAACTTACAAATTAAAAGAAACAAAAGCACCAGAA
GGTTATGTAATCCCTGATAAAGAAATCGAGTTTACAGTATCACAAACATCTTATAATACAAAACCAAC
TGACATCACGGTTGATAGTGCTGATGCAACACCTGATACAATTAAAAACAACAAACGTCCTTCAATCC
CTAATACTGGTGGTATTGGTACGGCTATCTTTGTCGCTATCGGTGCTGCGGTGATGGCTTTTGCTGTT
AAGGGGATGAAGCGTCGTACAAAAGATAAC
**SEQ ID NO: 2**

MKLSKKLLFSAAVLTMVAGSTVEPVAQFATGMSIVRAAEVSQERPAKTTVNIYKLQADSYKSEITSNG
GIENKDGEVISNYAKLGDNVKGLQGVQFKRYKVKTDISVDELKKLTTVEAADAKVGTILEEGVSLPQK
TNAQGLVVDALDSKSNVRYLYVEDLKNSPSNITKAYAVPFVLELPVANSTGTGFLSEINIYPKNVVTD
EPKTDKDVKKLGQDDAGYTIGEEFKWFLKSTIPANLGDYEKFEITDKFADGLTYKSVGKIKIGSKTLN
RDEHYTIDEPTVDNQNTLKITFKPEKFKEIAELLKGMTLVKNQDALDKATANTDDAAFLEIPVASTIN
EKAVLGKAIENTFELQYDHTPDKADNPKPSNPPRKPEVHTGGKRFVKKDSTETQTLGGAEFDLLASDG
TAVKWTDALIKANTNKNYIAGEAVTGQPIKLKSHTDGTFEIKGLAYAVDANAEGTAVTYKLKETKAPE
GYVIPDKEIEFTVSQTSYNTKPTDITVDSADATPDTIKNNKRPSIPNTGGIGTAIFVAIGAAVMAFAV
KGMKRRTKDN

As described above, the combinations of the invention may include a fragment of a GBS antigen. In some instances, removal of one or more domains, such as a leader or signal sequence region, a transmembrane region, a cytoplasmic region or a cell wall anchoring motif, may facilitate cloning of the gene encoding the antigen and/or recombinant expression of the GBS protein. In addition, fragments comprising immunogenic epitopes of the cited GBS antigens may be used in the compositions of the invention.

GBS 80 contains an N-terminal leader or signal sequence region which is indicated by the underlined sequence at the beginning of SEQ ID NO: 2 above. In one embodiment, one or more amino acids from the leader or signal sequence region of GBS 80 are removed. An example of such a GBS 80 fragment is set forth below as SEQ ID NO: 3:
**SEQ ID NO: 3**

AEVSQERPAKTTVNIYKLQADSYKSEITSNGGIENKDGEVISNYAKLGDNVKGLQGVQFKRYKVKTDI
SVDELKKLTTVEAADAKVGTILEEGVSLPQKTNAQGLVVDALDSKSNVRYLYVEDLKNSPSNITKAYA
VPFVLELPVANSTGTGFLSEINIYPKNVVTDEPKTDKDVKKLGQDDAGYTIGEEFKWFLKSTIPANLG
DYEKFEITDKFADGLTYKSVGKIKIGSKTLNRDEHYTIDEPTVDNQNTLKITFKPEKFKEIAELLKGM
TLVKNQDALDKATANTDDAAFLEIPVASTINEKAVLGKAIENTFELQYDHTPDKADNPKPSNPPRKPE
VHTGGKRFVKKDSTETQTLGGAEFDLLASDGTAVKWTDALIKANTNKNYIAGEAVTGQPIKLKSHTDG
TFEIKGLAYAVDANAEGTAVTYKLKETKAPEGYVIPDKEIEFTVSQTSYNTKPTDITVDSADATPDTI
KNNKRPSIPNTGGIGTAIFVAIGAAVMAFAVKGMKRRTKDN

GBS 80 contains a C-terminal transmembrane region which is indicated by the underlined sequence near the end of SEQ ID NO: 2 above. In one embodiment, one or more amino acids from the transmembrane region and/or a cytoplasmic region are removed. An example of such a GBS 80 fragment is set forth below as SEQ ID NO: 4:
**SEQ ID NO: 4**

MKLSKKLLFSAAVLTMVAGSTVEPVAQFATGMSIVRAAEVSQERPAKTTVNIYKLQADSYKSEITSNG
GIENKDGEVISNYAKLGDNVKGLQGVQFKRYKVKTDISVDELKKLTTVEAADAKVGTILEEGVSLPQK
TNAQGLVVDALDSKSNVRYLYVEDLKNSPSNITKAYAVPFVLELPVANSTGTGFLSEINIYPKNVVTD
EPKTDKDVKKLGQDDAGYTIGEEFKWFLKSTIPANLGDYEKFEITDKFADGLTYKSVGKIKIGSKTLN
RDEHYTIDEPTVDNQNTLKITFKPEKFKEIAELLKGMTLVKNQDALDKATANTDDAAFLEIPVASTIN
EKAVLGKAIENTFELQYDHTPDKADNPKPSNPPRKPEVHTGGKRFVKKDSTETQTLGGAEFDLLASDG

TAVKWTDALIKANTNKNYIAGEAVTGQPIKLKSHTDGTFEIKGLAYAVDANAEGTAVTYKLKETKAPE
GYVIPDKEIEFTVSQTSYNTKPTDITVDSADATPDTIKNNKRPSIPNTG

GBS 80 contains an amino acid motif indicative of a cell wall anchor: **SEQ ID NO: 5** IPNTG (shown in italics in SEQ ID NO: 2 above). In some recombinant host cell systems, it may be preferable to remove this motif to facilitate secretion of a recombinant GBS 80 protein from the host cell. Accordingly, in one preferred fragment of GBS 80 for use in the invention, the transmembrane and/or cytoplasmic regions and the cell wall anchor motif are removed from GBS 80. An example of such a GBS 80 fragment is set forth below as SEQ ID NO: 6.
**SEQ ID NO: 6**

MKLSKKLLFSAAVLTMVAGSTVEPVAQFATGMSIVRAAEVSQERPAKTTVNIYKLQADSYKSEITSNG
GIENKDGEVISNYAKLGDNVKGLQGVQFKRYKVKTDISVDELKKLTTVEAADAKVGTILEEGVSLPQK
TNAQGLVVDALDSKSNVRYLYVEDLKNSPSNITKAYAVPFVLELPVANSTGTGFLSEINIYPKNVVTD
EPKTDKDVKKLGQDDAGYTIGEEFKWFLKSTIPANLGDYEKFEITDKFADGLTYKSVGKIKIGSKTLN
RDEHYTIDEPTVDNQNTLKITFKPEKFKEIAELLKGMTLVKNQDALDKATANTDDAAFLEIPVASTIN
EKAVLGKAIENTFELQYDHTPDKADNPKPSNPPRKPEVHTGGKRFVKKDSTETQTLGGAEFDLLASDG
TAVKWTDALIKANTNKNYIAGEAVTGQPIKLKSHTDGTFEIKGLAYAVDANAEGTAVTYKLKETKAPE
GYVIPDKEIEFTVSQTSYNTKPTDITVDSADATPDTIKNNKRPS

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

In one embodiment, the leader or signal sequence region, the transmembrane and cytoplasmic regions and the cell wall anchor motif are removed from the GBS 80 sequence. An example of such a GBS 80 fragment is set forth below as SEQ ID NO: 7.
**SEQ ID NO: 7**

AEVSQERPAKTTVNIYKLQADSYKSEITSNGGIENKDGEVISNYAKLGDNVKGLQGVQFKRYKVKTDI
SVDELKKLTTVEAADAKVGTILEEGVSLPQKTNAQGLVVDALDSKSNVRYLYVEDLKNSPSNITKAYA
VPFVLELPVANSTGTGFLSEINIYPKNVVTDEPKTDKDVKKLGQDDAGYTIGEEFKWFLKSTIPANLG
DYEKFEITDKFADGLTYKSVGKIKIGSKTLNRDEHYTIDEPTVDNQNTLKITFKPEKFKEIAELLKGM
TLVKNQDALDKATANTDDAAFLEIPVASTINEKAVLGKAIENTFELQYDHTPDKADNPKPSNPPRKPE
VHTGGKRFVKKDSTETQTLGGAEFDLLASDGTAVKWTDALIKANTNKNYIAGEAVTGQPIKLKSHTDG
TFEIKGLAYAVDANAEGTAVTYKLKETKAPEGYVIPDKEIEFTVSQTSYNTKPTDITVDSADATPDTI
KNNKRPS

Applicants have identified a particularly immunogenic fragment of the GBS 80 protein. This immunogenic fragment is located towards the N-terminus of the protein and is underlined in the GBS 80 SEQ ID NO: 2 sequence below. The underlined fragment is set forth below as SEQ ID NO: 8.
**SEQ ID NO: 2** 
MKLSKKLLFSAAVLTMVAGSTVEPVAQFATGMSIVRAAEVSQERPAKTTVNIYKLQADSYKSEITSNG
GIENKDGEVISNYAKLGDNVKGLQGVQFKRYKVKTDISVDELKKLTTVEAADAKVGTILEEGVSLPQK
TNAQGLVVDALDSKSNVRYLYVEDLKNSPSNITKAYAVPFVLELPVANSTGTGFLSEINIYPKNVVTD
EPKTDKDVKKLGQDDAGYTIGEEFKWFLKSTIPANLGDYEKFEITDKFADGLTYKSVGKIKIGSKTLN
RDEHYTIDEPTVDNQNTLKITFKPEKFKEIAELLKGMTLVKNQDALDKATANTDDAAFLEIPVASTIN
EKAVLGKAIENTFELQYDHTPDKADNPKPSNPPRKPEVHTGGKRFVKKDSTETQTLGGAEFDLLASDG
TAVKWTDALIKANTNKNYIAGEAVTGQPIKLKSHTDGTFEIKGLAYAVDANAEGTAVTYKLKETKAPE
GYVIPDKEIEFTVSQTSYNTKPTDITVDSADATPDTIKNNKRPSIPNTGGIGTAIFVAIGAAVMAFAV
KGMKRRTKDN
**SEQ ID NO: 8**

AEVSQERPAKTTVNIYKLQADSYKSEITSNGGIENKDGEVISNYAKLGDNVKGLQGVQFKRYKVKTDI
SVDELKKLTTVEAADAKVGTILEEGVSLPQKTNAQGLVVDALDSKSNVRYLYVEDLKNSPSNITKAYA
VPFVLELPVANSTGTGFLSEINIYPKNVVTDEPKTDKDVKKLGQDDAGYTIGEEFKWFLKSTIPANLG
DYEKFEITDKFADGLTYKSVGKIKIGSKTLNRDEHYTIDEPTVDNQNTLKITFKPEKFKEIAELLKG

The immunogenicity of the protein encoded by SEQ ID NO: 7 was compared against PBS, GBS whole cell, GBS 80 (full length) and another fragment of GBS 80, located closer to the C-terminus of the peptide (SEQ ID NO: 9, below).
**SEQ ID NO: 9**

MTLVKNQDALDKATANTDDAAFLEIPVASTINEKAVLGKAIENTFELQYDHTPDKADNPKPSNPPRKPEVHTGGK
RFVKKDSTETQTLGGAEFDLLASDGTAVKWTDALIKANTNKNYIAGEAVTGQPIKLKSHTDGTFEIKGLAYAVDA
NAEGTAVTYKLKETKAPEGYVIPDKEIEFTVSQTSYNTKPTDITVDSADATPDTIKNNKRPS

Both an Active Maternal Immunization Assay and a Passive Maternal Immunization Assay were conducted on this collection of proteins.

As used herein, an Active Maternal Immunization assay refers to an *in vivo* protection assay where female mice are immunized with the test antigen composition. The female mice are then bred and their pups are challenged with a lethal dose of GBS. Serum titers of the female mice during the immunization schedule are measured as well as the survival time of the pups after challenge.

Specifically, the Active Maternal Immunization assays referred to herein used groups of four CD-1 female mice (Charles River Laboratories, Calco Italy). These mice were immunized intraperitoneally with the selected proteins in Freund's adjuvant at days 1, 21 and 35, prior to breeding. 6-8 weeks old mice received 20 µg protein/dose when immunized with a single antigen, 30-45 µg protein/dose (15 µg each antigen) when immunized with combination of antigens. The immune response of the dams was monitored by using serum samples taken on day 0 and 49. The female mice were bred 2-7 days after the last immunization (at approximately t= 36 - 37), and typically had a gestation period of 21 days. Within 48 hours of birth, the pups were challenged via I.P. with GBS in a dose approximately equal to an amount which would be sufficient to kill 70 - 90 % of unimmunized pups (as determined by empirical data gathered from PBS control groups). The GBS challenge dose is preferably administered in 50µl of THB medium. Preferably, the pup challenge takes place at 56 to 61 days after the first immunization. The challenge inocula were prepared starting from frozen cultures diluted to the appropriate concentration with THB prior to use. Survival of pups was monitored for 5 days after challenge.

As used herein, the Passive Maternal Immunization Assay refers to an *in vivo* protection assay where pregnant mice are passively immunized by injecting rabbit immune sera (or control sera) approximately 2 days before delivery. The pups are then challenged with a lethal dose of GBS.

Specifically, the Passive Maternal Immunization Assay referred to herein used groups of pregnant CD1 mice which were passively immunized by injecting 1 ml of rabbit immune sera or control sera via I.P., 2 days before delivery. Newborn mice (24-48 hrs after birth) are challenged via I.P. with a 70 - 90% lethal dose of GBS serotype III COH1. The challenge dose, obtained by diluting a frozen mid log phase culture, was administered in 50µl of THB medium.

For both assays, the number of pups surviving GBS infection was assessed every 12 hrs for 4 days. Statistical significance was estimated by Fisher's exact test.

The results of each assay for immunization with SEQ ID NO: 7, SEQ ID NO: 8, PBS and GBS whole cell are set forth in Tables 1 and 2 below.

| | | | |
|---|---|---|---|
| **TABLE 1: Active Maternal Immunization** | | | |

| Antigen | Alive/total | %Survival | Fisher's exact test |
|---|---|---|---|
| PBS (neg control) | 13/80 | 16% | |
| GBS (whole cell) | 54/65 | 83% | P<0.00000001 |
| GBS80 (intact) | 62/70 | 88% | P<0.00000001 |
| GBS80 (fragment) SEQ ID 7 | 35/64 | 55% | P=0.0000013 |
| GBS80 (fragment) SEQ ID 8 | 13/67 | 19% | P=0.66 |

| **Table 2: Passive Maternal Immunization** | | | |
|---|---|---|---|
| Antigen | Alive/total | %Survival | Fisher's exact test |
| PBS (neg control) | 12/42 | 28% | |
| GBS (whole cell) | 48/52 | 92% | P<0.00000001 |
| GBS80 (intact) | 48/55 | 87% | P<0.00000001 |
| GBS80 (fragment) SEQ ID 7 | 45/57 | 79% | P=0.0000006 |
| GBS80 (fragment) SEQ ID 8 | 13/54 | 24% | P=1 |

As shown in Tables 1 and 2, immunization with the SEQ ID NO: 7 GBS 80 fragment provided a substantially improved survival rate for the challenged pups than the comparison SEQ ID NO: 8 GBS 80 fragment. These results indicate that the SEQ ID NO: 7 GBS 80 fragment may comprise an important immunogenic epitope of GBS 80.

### Combinations including GBS 80

The invention includes combinations of two or more GBS antigens wherein the combination includes GBS 80 or a fragment thereof. Applicants have discovered that GBS 80 is particularly suitable for immunization in combination with other GBS antigens and that these antigen combinations provide for a broader coverage among different GBS strains.

Preferably, the combination of GBS antigens consists of three, four, five, six, seven, eight, nine, or ten GBS antigens. Still more preferably, the combination of GBS antigens consists of three, four, or five GBS antigens.

Preferably, the combinations of the invention provide for improved immunogenicity over the immunogenicity of the antigens when administered alone. Improved immunogenicity may be measured, for example, by the Active Maternal Immunization Assay. As discussed above, this assay may be used to measure serum titers of the female mice during the immunization schedule as well as the survival time of the pups after challenge. Preferably, immunization with the immunogenic compositions of the invention yield an increase of at least 2 percentage points (preferably at least 3, 4 or 5 percentage points) in the percent survival of the challenged pups as compared to the percent survival from maternal immunization with a single antigen of the composition when administered alone. Preferably, the increase is at least 6, 7, 8, 9, 10, 11, 12, 13, 14, 15, 16, 17, 18, 19, 20, 21, 22, 23, 24, 25, 26, 27, 28, 29 or 30 percentage points. Preferably, the GBS combinations of the invention comprising GBS 80 demonstrate an increase in the percent survival as compared to the percent survival from immunization with a non-GBS 80 antigen alone.

According to one embodiment of the invention, combinations of antigens or fusion proteins containing a portion or portions of the antigens will include GBS 80 or a pportion thereof in combination with from one to 10 antigens, preferably one to 10 or less antigens. Such other antigens include by way of example and not limitation, GBS 67, GBS 91, GBS 104, GBS 184, GBS 276, GBS 305, GBS 322, GBS 330, GBS 338, GBS 361, GBS 404, GBS 690, and GBS 691. Still other antigens are identified in U.S. Serial Number 10/415,182, filed April 28, 2003, hereby incorporated in its entirety.

Combinations, for example, can include GBS 80, GBS 104, GBS 322, and GBS 276, ; GBS 80, GBS 338, GBS 330; GBS 80, GBS 330, GBS 104; GBS 80, GBS 104, GBS 404; GBS 80, GBS 338, GBS 104; GBS 80, GBS 338, GBS404; GBS 338, GBS 330, GBS 104; GBS 338, GBS 104, GBS 404; GBS 80, GBS 330, GBS 404; GBS 80, GBS 322, GBS 104; GBS 80, GBS 322, GBS 276; GBS 80, GBS 322, GBS 91; GBS 80, GBS 104, GBS 276; GBS 80, GBS 104, GBS 91; GBS 80, GBS 276, GBS 91; GBS 80, GBS 322, GBS 104; GBS 80, GBS 322, GBS 276; GBS 80, GBS 322, GBS 91; GBS 80, GBS 104, GBS 276; GBS 80, GBS 104, GBS 91; GBS 80, GBS 276, GBS 91; GBS 80, GBS 690, GBS 691; GBS 80, GBS 690, GBS 338; GBS 80, GBS 690, GBS 305; GBS 80, GBS 691, GBS 305; GBS 80, GBS 338, GBS 305; GBS 80, GBS 338, GBS 361; GBS 80, GBS 305, GBS 361; GBS 80, GBS 184, GBS 691; GBS 80, GBS 691, GBS 338; GBS 80, GBS 104, GBS 276, GBS 322; GBS 80, GBS 104, GBS 67, and GBS 322. Examples of combinations of the invention which demonstrate improved immunogenicity are set forth below. A more detailed description of the GBS antigens referred to in these experiments is set forth following the examples.

### EXAMPLE 1: Active Maternal Immunization Assay of GBS 80 alone vs. in combination

In this example, the Active Maternal Immunization Assay was used to measure the percent survival of pups challenged with a Type III serotype of GBS (COH1 isolate), at t=56 days. The maternal mice were immunized according to the Active Maternal Immunization Assay schedule discussed above with GBS 80 alone, combinations of GBS antigens (with and without GBS 80), placebo (PBS) or inactivated whole cell GBS isolate as indicated in Table 3 below. In these experiments, the challenge dose for GBS Type III, strain isolate COH1 sufficient to kill 70 - 90 % of unimmunized pups is approximately equal to 10 x LD 50% (where LD 50% is the statistically derived Median Lethal Dose).

**Table 3: Active Maternal Immunization Assay of GBS 80 alone vs. in combination**

| | **I Challenge t=56 days** | |
|---|---|---|
| | **Type III COH1 10 x LD 50%** | |
| α-GBS | **Alive/treated** | **Survival %** |
| α-PBS | 3/26 | 11 |
| α-GBS III | 9/20 | 45 |
| 80 | 24/34 | 70 |
| 80+338+330 | 39/40 | 97 |
| 80+330+104 | 38/40 | 95 |
| 80+104+404 | 24/24 | 100 |
| 80+338+104 | 33/34 | 97 |
| 80+338+404 | 30/30 | 100 |
| 338+330+104 | 22/30 | 73 |
| 338+104+404 | 24/37 | 65 |
| 80+330+404 | 25/28 | 89 |

As shown in Table 3, combinations of GBS antigens which included GBS 80 demonstrated an improved immunogenicity over the use of the antigens alone. For example, immunization with GBS 80 alone yielded a 70% survival rate among the challenged pups. Immunization with combinations of GBS 80 with GBS 338, GBS 330, GBS 104, and GBS 404 yielded 95 to 100% survival rate among the challenged pups. This is an increase of 25 to 30 percentage points.

By comparison, combinations of these antigens which did not include GBS 80 failed to achieve the % survival of GBS 80 alone. For example, immunization with GBS 338, GBS 104 and GBS 404 yielded a 65% survival rate. Replacement of any one of these antigens with GBS 80 dramatically increased the percent survival rate to between 97 and 100%. This is an increase of 32 to 35 percentage points. (See percent survival rates of GBS 80, 338, 101 (97%); GBS 80, 338, 404 (100%) and GBS 80, 104, 404 (100%)). Similarly, immunization with GBS 338, 330 and 104 yielded a 73% survival rate. Replacement of any one of these antigens with GBS 80 increased the percent survival rate to between 95 - 97%.

These findings indicate that protection from COH1 isolate is increased with use of GBS 80 in combination with other GBS antigens.

### EXAMPLE 2: Active Maternal Immunization Assay of GBS 80, GBS 322, GBS 276, GBS 104 alone vs. in combination

In this example, the Active Maternal Immunization Assay was used to measure the percent survival of pups challenged with a Type III serotype of GBS (COH1 isolate) at t=56 days. The maternal mice were immunized according to the Active Maternal Immunization Assay schedule discussed above with a single GBS antigen, combinations of GBS antigens with GBS 80, and placebo (PBS) as indicated in Table 4 below.

**Table 4: Active Maternal Immunization Assay of GBS 80, GBS 322, GBS 276 or GBS 104 alone vs. in combination with GBS 80**

| | **I Challenge t=56 days** | |
|---|---|---|
| | **Type III COH1** | **10x LD 50%** |
| α**-GBS** | **Alive/treated** | **Survival %** |
| 80 + 322 + 104 | **27/27** | **100** |
| 80 + 322 + 276 | **35/38** | **92** |
| 80 + 322 + 91 | **24/24** | **100** |
| 80 + 104 + 276 | **29/30** | **97** |
| 80 + 104 + 91 | **36/40** | **90** |
| 80 + 276 + 91 | **33/40** | **82** |
| GBS 80 | **24/30** | **80** |
| GBS 322 | **7/40** | **17** |
| GBS 276 | **13/37** | **35** |
| GBS 104 | **28/38** | **74** |
| α-PBS | **2/27** | **7** |

As shown in Table 4, the combinations of the antigens with GBS 80 yielded improved immunogenicity over the use of the antigens alone. For example, immunization with GBS 322 alone yielded a 17 % survival rate among the challenged pups. Immunization with combinations of GBS 322 with GBS 80 and another GBS antigen yielded survival rates of 92 - 100%. As another example, immunization with GBS 104 alone yielded a 74% survival rate. Immunization with combinations of GBS 104 with GBS 80 and another GBS antigen yielded survival rates of 90 -100%. As another example, immunization with GBS 276 alone yielded a 35% survival rate. Immunization with combinations of GBS 276 with GBS 80 and another GBS antigen yielded survival rates of 82 - 97%.

Having demonstrated the immunogenicity of the above-described combinations, the duration of the immune response in the mouse model was further analysed. The maternal mice used in the above described Active Maternal Immunization Assay were mated a second time and the resulting pups challenged with a different GBS serotype (Type V, CJB 111 isolate) at a dramatically higher dose (300x LD 50%) at t=91 days. The parameters of this second, much stronger challenge were outside those of the standard Active Maternal Immunization Assay and were meant to probe the limits of the immunological memory generated from the original maternal immunization in the mouse model. Indication of immunological memory in this model under these conditions is thought to be significant. As shown in Table 5, even under these extreme conditions, increased survival rates were generally achieved, particularly for the combination comprising GBS 80, GBS 322 and GBS 104. It was surprising to note that the percent survival rate for the combination of GBS 80, GBS 233 and GBS 104 was 100% for both the first and second challenges.

**Table 5: Second generation pups challenged with higher dose of different strain**

| | **II Challenge t=91 days** | |
|---|---|---|
| | **Type V CJB111 300x LD 50%** | |
| α**-GBS** | **Alive/treated** | **Survival** % |
| 80 + 322 + 104 | **20/20** | **100** |
| 80 + 322 + 276 | **32/37** | **86** |
| 80 + 322 + 91 | **27/30** | **90** |
| 80 + 104 + 276 | **22/37** | **59** |
| 80 + 104 + 91 | **36/39** | **92** |
| 80 + 276 + 91 | **23/28** | **82** |
| GBS 80 | **13/30** | **43** |
| GBS 322 | **25/30** | **83** |
| GBS 276 | **18/40** | **45** |
| GBS 104 | **21/39** | **54** |
| α-PBS | **9/36** | **25** |

### EXAMPLE 3: Active Maternal Immunization Assay of combinations of GBS 80 with GBS 690, GBS 691, GBS 338, GBS 305, GBS 361 and GBS 184

In this example additional combinations of GBS antigens were used in the Active Maternal Immunization Assay, again with a GBS Type III COH1 isolate challenge. The maternal mice were immunized according to the Active Maternal Immunization Assay schedule described above with the combinations of GBS antigens set forth in Table 6 below.

**Table 6: Active Maternal Immunization Assay using combinations of GBS 80 with GBS 690, GBS 691, GBS 338, GBS 305, GBS 361 and GBS 184**

| | **I Challenge t=56 days** | |
|---|---|---|
| | **Type III COH1 10x LD 50%** | |
| α**-GBS** | **Alive/treated** | **Survival %** |
| 80 + 690 + 691 | **26/29** | **90** |
| 80 + 690 + 338 | **35/40** | **87** |
| 80 + 690 + 305 | **34/35** | **97** |
| 80 + 691 + 305 | **37/40** | **92** |
| 80 + 338 + 305 | ***25*/*30*** | ***83*** |
| 80 + 338 + 361 | **26/30** | **87** |
| 80 + 305 +361 | **23/30** | **77** |
| 80 + 184 + 691 | **32/39** | **82** |
| α-PBS | **10/40** | **25** |

The maternal mice in this model were also mated a second time and the resulting pups challenged with the same GBS isolate at a dramatically higher dose (100x LD 50%) at t=84 days. As in the example above, the parameters of this second, much stronger challenge were outside those of the standard Active Maternal Immunization Assay and were meant to probe the limits of the immunological memory generated from the original maternal immunization in the mouse model. As shown in Table 7, even under these extreme conditions, some of the survival rates remained at or above 70%. Surprisingly, the percent survival rates for the combination of GBS 80, GBS 184 and GBS 691 actually increased.

**Table 7: Second generation pups challenged with higher dose**

| | **II Challenge t=84 days** | |
|---|---|---|
| | **Type III COH1 100x LD 50%** | |
| α**-GBS** | **Alive/treated** | **Survival %** |
| 80 + 690 + 691 | **19/39** | **49** |
| 80 + 690 + 338 | **21/30** | **70** |
| 80 + 690 + 305 | **23/40** | **57** |
| 80 + 691 + 305 | **22/30** | **73** |
| 80 + 338 + 305 | **18/30** | **60** |
| 80 + 338 + 361 | **25/40** | **62** |
| 80 + 305 +361 | **21/30** | **70** |
| 80 + 184 + 691 | **35/40** | **87** |
| α-PBS | **4/20** | **20** |

### EXAMPLE 4: Active Maternal Immunization Assay using combinations of GBS 80 with GBS 690, GBS 691, GBS 338, GBS 305, and GBS 361

In this example additional combinations of GBS antigens were used in the Active Maternal Immunization Assay, this time with a GBS Type V, CJB111 isolate challenge. In these experiments, the challenge dose for the GBS Type V, CJB111 isolate sufficient to kill 70 - 90% of unimmunized pups is approximately equal to 60 x LD 50% (where LD 50% is the statistically derived Median Lethal Dose). The maternal mice were immunized according to the Active Maternal Immunization Assay schedule described above with the combinations of GBS antigens set forth in Table 8 below. As shown in Table 8, in this particular challenge study with this specific Type V strain isolate, the survival rates for all of the combinations achieved at least 70%.

**Table 8: Active Maternal Immunization Assay using combinations of GBS 80 with GBS 690, GBS 691, GBS 338, GBS 305 and GBS 361**

| | **I Challenge t=56 days** | |
|---|---|---|
| | **Type V CJB111 60x LD 50%** | |
| α-**GBS** | **Alive/treated** | **Survival %** |
| 80 + 690 + 691 | 24/30 | 80 |
| 80 + 690 + 338 | 11/17 | 70 |
| 80 + 691 + 338 | 7/10 | 70 |
| 80 + 691 + 305 | 21/30 | 70 |
| 80 + 338 + 305 | 26/30 | 87 |
| 80 + 338 + 361 | 26/30 | 87 |
| 80 + 305 +361 | 28/30 | 93 |
| GBS 80 | 21/30 | 70 |
| α-PBS | 5/18 | 28 |

The maternal mice in this model were also mated a second time and the resulting pups challenged with the same GBS isolate at a dramatically higher dose (600x LD 50%) at t=84 days. As in the example above, the parameters of this second, much stronger challenge were outside those of the standard Active Maternal Immunization Assay and were meant to probe the limits of the immunological memory generated from the original maternal immunization in the mouse model. As shown in Table 9, even under these extreme conditions, some of the survival rates remained above 70%. Surprisingly, the percent survival for two of the antigen groups actually increased (GBS 80, GBS 690 and GBs 338) and (GBS 80, GBS 691 and GBS 338).

**Table 9: Second generation pups challenged with higher dose**

| | **II Challenge t=84 days** | |
|---|---|---|
| | **Type V CJB111 600x LD 50%** | |
| α**-GBS** | **Alive/treated** | **Survival %** |
| 80 + 690 + 691 | 27/37 | 73 |
| 80 + 690 + 338 | 15/20 | 75 |
| 80 + 691 + 338 | 27/30 | 90 |
| 80 + 691 + 305 | 23/40 | 57 |
| 80 + 338 + 305 | 12/20 | 60 |
| 80 + 338 + 361 | 24/30 | 80 |
| 80 + 305 +361 | 24/30 | 80 |
| GBS 80 | 24/30 | 80 |
| α-PBS | ND | ND |

### EXAMPLE 5: Active Maternal Immunization Assay using combinations of GBS 80 with GBS 104, GBS 276, and GBS 322

In this example an additional combination of GBS antigens was used in the Active Maternal Immunization Assay, this time with an isolate challenge of different GBS strains. In these experiments, the challenge dose for the different GBS strains was sufficient to kill 60 - 90% of unimmunized pups and is equal to 10 x LD 50% (where LD 50% is the statistically derived Median Lethal Dose). The maternal mice were immunized according to the Active Maternal Immunization Assay schedule described above with the combination of GBS 80 antigen with GBS 104, GBS 276, and GBS 322 antigens in the GBS strains set forth in Table 10 below. Survival % was observed with the GBS combination with two different adjuvants, Alum and Freunds. As shown in Tables 10 and 11, in this particular challenge study, the survival rates for the combination in all of the GBS strains achieved up to 96%.

**Table 10: Active Maternal Immunization Assay using combinations of GBS 80 with GBS 104, GBS 276, and GBS 322 - Alum adjuvant**

| | | **ALUM** | | | |
|---|---|---|---|---|---|
| | | **Mix=322+80+104+276** | | **PBS** | |
| **GBS strains** | **Type** | **Alive/treated** | **Survival %** | **Alive/treated** | **Survival %** |
| JM9130013 | **VIII** | **32/36** | **89** | **18/46** | **40** |
| CJB111 | **V** | **118/145** | **81** | **21/110** | **19** |
| COH1 | **III** | **96/115** | **83** | **22/104** | **21** |
| M781 | **III** | **42/52** | **81** | **18/48** | **38** |
| 2603 | **V** | **79/145** | **54** | **28/128** | **22** |
| 18RS21 | **II** | **86/186** | **46** | **24/131** | **18** |
| DK21 | **II** | **31/140** | **22** | **28/118** | **24** |
| 7357b - | **Ib** | **25/88** | **28** | **25/106** | **23** |
| A909 | **Ia** | **4/40** | **10** | **9/60** | **15** |
| 090 | **Ia** | **2/31** | **6** | **4/53** | **7** |
| SMO53 | **VII** | **17/54** | **31** | **4/39** | **10** |

**Table 11: Active Maternal Immunization Assay using combinations of GBS 80 with GBS 104, GBS 276, and GBS 322 -Freund adjuvant**

| | | **Freund** | | | |
|---|---|---|---|---|---|
| | | **Mix=322+80+104+276** | | **PBS** | |
| **GBS strains** | **Type** | **Alive/treated** | **Survival %** | **Alive/treated** | **Survival %** |
| JM9130013 | **VIII** | **nd** | **nd** | **nd** | **nd** |
| CJB111 | **V** | **47/49** | **96** | **12/46** | **26** |
| COH1 | **III** | **47/50** | **94** | **12/50** | **24** |
| M781 | **III** | **33/50** | **66** | **6/50** | **12** |
| 2603 | **V** | **28/30** | **93** | **8/48** | **17** |
| 18RS21 | **II** | **31/78** | **40** | **10/46** | **22** |
| DK21 | **II** | **37/68** | **54** | **15/60** | **25** |
| H36B | **Ib** | **8/38** | **21** | **5/60** | **8** |
| 7357b - | **Ib** | **29/50** | **58** | **5/50** | **10** |
| A909 | **Ia** | **18/49** | **37** | **6/49** | **12** |

Accordingly, the invention therefore includes compositions comprising combinations of two or more GBS antigens, wherein the combination includes GBS 80 or a fragment thereof or a polypeptide sequence having sequence identity thereto.

In one embodiment, the combination may consist of two to thirteen GBS antigens, including GBS 80. As an example, the combination may contain GBS 80 and other GBS antigens selected from the group consisting of GBS 80, GBS 91, GBS 104, GBS 184, GBS 276, GBS 305, GBS 322, GBS 330, GBS 338, GBS 361, GBS 404, GBS 690, and GBS 691. Preferably, the combination includes GBS 80 in combination with one or more of GBS 104 and GBS 322. For example, the combination may include GBS 80, GBS 104, GBS 322 and GBS 67.

Instead of the full length antigen, the combination may comprise an immunogenic fragment of the selected GBS antigen and/or a polypeptide sequence having sequence identity to the selected antigen.

Preferably, the combination of GBS antigens consists of three, four, five, six, seven, eight, nine, or ten GBS antigens. Still more preferably, the combination of GBS antigens consists of three, four, or five GBS antigens.

Details of examples of GBS antigens for use in combination with GBS 80 are set forth below.

### GBS 91

GBS 91 refers to a GBS C3 binding polypeptide. Nucleotide and amino acid sequences of GBS 91 sequenced from serotype V isolated strain 2603 V/R are set forth in Ref. 2 as SEQ ID 8937 and SEQ ID 8938. These sequences are set forth below as SEQ ID NOS 10 and 11:
**SEQ ID NO. 10** 
ATGAAAAAAGGACAAGTAAATGATACTAAGCAATCTTACTCTCTACGTAAATATAAATTTGGTTTAGC
ATCAGTAATTTTAGGGTCATTCATAATGGTCACAAGTCCTGTTTTTGCGGATCAAACTACATCGGTTC
AAGTTAATAATCAGACAGGCACTAGTGTGGATGCTAATAATTCTTCCAATGAGACAAGTGCGTCAAGT
GTGATTACTTCCAATAATGATAGTGTTCAAGCGTCTGATAAAGTTGTAAATAGTCAAAATACGGCAAC
AAAGGACATTACTACTCCTTTAGTAGAGACAAAGCCAATGGTGGAAAAAACATTACCTGAACAAGGGA
ATTATGTTTATAGCAAAGAAACCGAGGTGAAAAATACACCTTCAAAATCAGCCCCAGTAGCTTTCTAT
GCAAAGAAAGGTGATAAAGTTTTCTATGACCAAGTATTTAATAAAGATAATGTGAAATGGATTTCATA
TAAGTCTTTTTGTGGCGTACGTCGATACGCAGCTATTGAGTCACTAGATCCATCAGGAGGTTCAGAGA
CTAAAGCACCTACTCCTGTAACAAATTCAGGAAGCAATAATCAAGAGAAAATAGCAACGCAAGGAAAT
TATACATTTTCACATAAAGTAGAAGTAAAAAATGAAGCTAAGGTAGCGAGTCCAACTCAATTTACATT
GGACAAAGGAGACAGAATTTTTTACGACCAAATACTAACTATTGAAGGAAATCAGTGGTTATCTTATA
AATCATTCAATGGTGTTCGTCGTTTTGTTTTGCTAGGTAAAGCATCTTCAGTAGAAAAAACTGAAGAT
AAAGAAAAAGTGTCTCCTCAACCACAAGCCCGTATTACTAAAACTGGTAGACTGACTATTTCTAACGA
AACAACTACAGGTTTTGATATTTTAATTACGAATATTAAAGATGATAACGGTATCGCTGCTGTTAAGG
TACCGGTTTGGACTGAACAAGGAGGGCAAGATGATATTAAATGGTATACAGCTGTAACTACTGGGGAT
GGCAACTACAAAGTAGCTGTATCATTTGCTGACCATAAGAATGAGAAGGGTCTTTATAATATTCATTT
ATACTACCAAGAAGCTAGTGGGACACTTGTAGGTGTAACAGGAACTAAAGTGACAGTAGCTGGAACTA
ATTCTTCTCAAGAACCTATTGAAAATGGTTTAGCAAAGACTGGTGTTTATAATATTATCGGAAGTACT
GAAGTAAAAAATGAAGCTAAAATATCAAGTCAGACCCAATTTACTTTAGAAAAAGGTGACAAAATAAA
TTATGATCAAGTATTGACAGCAGATGGTTACCAGTGGATTTCTTACAAATCTTATAGTGGTGTTCGTC
GCTATATTCCTGTGAAAAAGCTAACTACAAGTAGTGAAAAAGCGAAAGATGAGGCGACTAAACCGACT
AGTTATCCCAACTTACCTAAAACAGGTACCTATACATTTACTAAAACTGTAGATGTGAAAAGTCAACC
TAAAGTATCAAGTCCAGTGGAATTTAATTTTCAAAAGGGTGAAAAAATACATTATGATCAAGTGTTAG
TAGTAGATGGTCATCAGTGGATTTCATACAAGAGTTATTCCGGTATTCGTCGCTATATTGAAATT
**SEQ ID NO. 11**

MKKGQVNDTKQSYSLRKYKFGLASVILGSFIMVTSPVFADQTTSVQVNNQTGTSVDANNSSNETSASS
VITSNNDSVQASDKVVNSQNTATKDITTPLVETKPMVEKTLPEQGNYVYSKETEVKNTPSKSAPVAFY
AKKGDKVFYDQVFNKDNVKWISYKSFCGVRRYAAIESLDPSGGSETKAPTPVTNSGSNNQEKIATQGN
YTFSHKVEVKNEAKVASPTQFTLDKGDRIFYDQILTIEGNQWLSYKSFNGVRRFVLLGKASSVEKTED
KEKVSPQPQARITKTGRLTISNETTTGFDILITNIKDDNGIAAVKVPVWTEQGGQDDIKWYTAVTTGD

GNYKVAVSFADHKNEKGLYNIHLYYQEASGTLVGVTGTKVTVAGTNSSQEPIENGLAKTGVYNIIGST
EVKNEAKISSQTQFTLEKGDKINYDQVLTADGYQWISYKSYSGVRRYIPVKKLTTSSEKAKDEATKPT
SYPNLPKTGTYTFTKTVDVKSQPKVSSPVEFNFQKGEKIHYDQVLVVDGHQWISYKSYSGIRRYIEI

GBS 91 contains an N-terminal leader or signal sequence region which is indicated by the underlined sequence at the beginning of SEQ ID NO: 11 above. In one embodiment, one or more amino acids from this leader or signal sequence region of GBS 91 are removed. An example of such a GBS 91 fragment is set forth below as SEQ ID NO: 12.
**SEQ ID NO: 12**

DQTTSVQVNNQTGTSVDANNSSNETSASSVITSNNDSVQASDKVVNSQNTATKDITTPLVETKPMVEK
TLPEQGNYVYSKETEVKNTPSKSAPVAFYAKKGDKVFYDQVFNKDNVKWISYKSFCGVRRYAAIESLD
PSGGSETKAPTPVTNSGSNNQEKIATQGNYTFSHKVEVKNEAKVASPTQFTLDKGDRIFYDQILTIEG
NQWLSYKSFNGVRRFVLLGKASSVEKTEDKEKVSPQPQARITKTGRLTISNETTTGFDILITNIKDDN
GIAAVKVPVWTEQGGQDDIKWYTAVTTGDGNYKVAVSFADHKNEKGLYNIHLYYQEASGTLVGVTGTK
VTVAGTNSSQEPIENGLAKTGVYNIIGSTEVKNEAKISSQTQFTLEKGDKINYDQVLTADGYQWISYK
SYSGVRRYIPVKKLTTSSEKAKDEATKPTSYPNLPKTGTYTFTKTVDVKSQPKVSSPVEFNFQKGEKI
HYDQVLVVDGHQWISYKSYSGIRRYIEI

GBS 91 contains a C-terminal transmembrane region which may be located within the underlined region near the end of SEQ ID NO: 11 above. In one embodiment, one or more amino acids from the transmembrane and cytoplasmic regions are removed. An example of such a GBS 91 fragment is set forth below as SEQ ID NO: 13.
**SEQ ID NO: 13**

MKKGQVNDTKQSYSLRKYKFGLASVILGSFIMVTSPVFADQTTSVQVNNQTGTSVDANNSSNETSASS
VITSNNDSVQASDKVVNSQNTATKDITTPLVETKPMVEKTLPEQGNYVYSKETEVKNTPSKSAPVAFY
AKKGDKVFYDQVFNKDNVKWISYKSFCGVRRYAAIESLDPSGGSETKAPTPVTNSGSNNQEKIATQGN
YTFSHKVEVKNEAKVASPTQFTLDKGDRIFYDQILTIEGNQWLSYKSFNGVRRFVLLGKASSVEKTED
KEKVSPQPQARITKTGRLTISNETTTGFDILITNIKDDNGIAAVKVPVWTEQGGQDDIKWYTAVTTGD
GNYKVAVSFADHKNEKGLYNIHLYYQEASGTLVGVTGTKVTVAGTNSSQEPIENGLAKTGVYNIIGST
EVKNEAKISSQTQFTLEKGDKINYDQVLTADGYQWISYKSYSGVRRYIPVKKLTTSSEKAKDEATKPT
SYPNLPKTG

GBS 91 contains an amino acid motif indicative of a cell wall anchor: SEQ ID NO: 14 LTKTG (shown in italics in SEQ ID NO: 11 above). In one embodiment, both the transmembrane domain and the cell wall anchor motif are removed from GBS 91. An example of such a GBS 91 fragment is set forth below as SEQ ID NO: 15.
**SEQ ID NO: 15**

MKKGQVNDTKQSYSLRKYKFGLASVILGSFIMVTSPVFADQTTSVQVNNQTGTSVDANNSSNETSASS
VITSNNDSVQASDKVVNSQNTATKDITTPLVETKPMVEKTLPEQGNYVYSKETEVKNTPSKSAPVAFY
AKKGDKVFYDQVFNKDNVKWISYKSFCGVRRYAAIESLDPSGGSETKAPTPVTNSGSNNQEKIATQGN
YTFSHKVEVKNEAKVASPTQFTLDKGDRIFYDQILTIEGNQWLSYKSFNGVRRFVLLGKASSVEKTED
KEKVSPQPQARITKTGRLTISNETTTGFDILITNIKDDNGIAAVKVPVWTEQGGQDDIKWYTAVTTGD
GNYKVAVSFADHKNEKGLYNIHLYYQEASGTLVGVTGTKVTVAGTNSSQEPIENGLAKTGVYNIIGST

EVKNEAKISSQTQFTLEKGDKINYDQVLTADGYQWISYKSYSGVRRYIPVKKLTTSSEKAKDEATKPT
SYPN

In one embodiment, one or more amino acids from the leader or signal sequence region and one or more amino acids from the transmembrane and cytoplasmic regions are removed from the GBS 91 sequence. An example of such a GBS 91 fragment is set forth below as SEQ ID NO: 16.
**SEQ ID NO: 16**

DQTTSVQVNNQTGTSVDANNSSNETSASSVITSNNDSVQASDKVVNSQNTATKDITTPLVETKPMVEK
TLPEQGNYVYSKETEVKNTPSKSAPVAFYAKKGDKVFYDQVFNKDNVKWISYKSFCGVRRYAAIESLD
PSGGSETKAPTPVTNSGSNNQEKIATQGNYTFSHKVEVKNEAKVASPTQFTLDKGDRIFYDQILTIEG
NQWLSYKSFNGVRRFVLLGKASSVEKTEDKEKVSPQPQARITKTGRLTISNETTTGFDILITNIKDDN
GIAAVKVPVWTEQGGQDDIKWYTAVTTGDGNYKVAVSFADHKNEKGLYNIHLYYQEASGTLVGVTGTK
VTVAGTNSSQEPIENGLAKTGVYNIIGSTEVKNEAKISSQTQFTLEKGDKINYDQVLTADGYQWISYK
SYSGVRRYIPVKKLTTSSEKAKDEATKPTSYPNLPKTG

In another embodiment, the leader or signal sequence region, the transmembrane and cytoplasmic regions, and the cell wall anchor motif are all removed from the GBS 91 sequence. An example of such a GBS 91 fragment is set forth below as SEQ ID NO: 17.
**SEQ ID NO: 17**

DQTTSVQVNNQTGTSVDANNSSNETSASSVITSNNDSVQASDKVVNSQNTATKDITTPLVETKPMVEK
TLPEQGNYVYSKETEVKNTPSKSAPVAFYAKKGDKVFYDQVFNKDNVKWISYKSFCGVRRYAAIESLD
PSGGSETKAPTPVTNSGSNNQEKIATQGNYTFSHKVEVKNEAKVASPTQFTLDKGDRIFYDQILTIEG
NQWLSYKSFNGVRRFVLLGKASSVEKTEDKEKVSPQPQARITKTGRLTISNETTTGFDILITNIKDDN
GIAAVKVPVWTEQGGQDDIKWYTAVTTGDGNYKVAVSFADHKNEKGLYNIHLYYQEASGTLVGVTGTK
VTVAGTNSSQEPIENGLAKTGVYNIIGSTEVKNEAKISSQTQFTLEKGDKINYDQVLTADGYQWISYK
SYSGVRRYIPVKKLTTSSEKAKDEATKPTSYPN

Further information regarding GBS 91 can be found in WO 01/25440 (C3 binding polypeptide), WO 01/32882 (ID-65), WO 02/31156 (BVH) and Reinscheid et al., Microbiology (2002) 148: 3245-3254 (*bsp* gene), each of which are incorporated herein by reference in their entirety.

### GB S 104

GBS 104 refers to a putative cell wall surface anchor family protein. It has been referred to as emaA protein. Nucleotide and amino acid sequences of GBS 104 sequenced from serotype V isolated strain 2603 V/R are set forth in Ref. 2 as SEQ ID 8777 and SEQ ID 8778. These sequences are set forth below as SEQ ID NOS 18 and 19:
**SEQ ID NO. 18** 
ATGAAAAGAGACAAAAATATGGAGAGGTTATCAGTTACTTTACTAATCCTGTCCCAAATTCCATT
TGGTATATTGGTACAAGGTGAAACCCAAGATACCAATCAAGCACTTGGAAAAGTAATTGTTAAAAAAA
CGGGAGACAATGCTACACCATTAGGCAAAGCGACTTTTGTGTTAAAAAATGACAATGATAAGTCAGAA
ACAAGTCACGAAACGGTAGAGGGTTCTGGAGAAGCAACCTTTGAAAACATAAAACCTGGAGACTACAC
ATTAAGAGAAGAAACAGCACCAATTGGTTATAAAAAAACTGATAAAACCTGGAAAGTTAAAGTTGCAG
ATAACGGAGCAACAATAATCGAGGGTATGGATGCAGATAAAGCAGAGAAACGAAAAGAAGTTTTGAAT
GCCCAATATCCAAAATCAGCTATTTATGAGGATACAAAAGAAAATTACCCATTAGTTAATGTAGAGGG
TTCCAAAGTTGGTGAACAATACAAAGCATTGAATCCAATAAATGGAAAAGATGGTCGAAGAGAGATTG
CTGAAGGTTGGTTATCAAAAAAAATTACAGGGGTCAATGATCTCGATAAGAATAAATATAAAATTGAA
TTAACTGTTGAGGGTAAAACCACTGTTGAAACGAAAGAACTTAATCAACCACTAGATGTCGTTGTGCT
ATTAGATAATTCAAATAGTATGAATAATGAAAGAGCCAATAATTCTCAAAGAGCATTAAAAGCTGGGG
AAGCAGTTGAAAAGCTGATTGATAAAATTACATCAAATAAAGACAATAGAGTAGCTCTTGTGACATAT
GCCTCAACCATTTTTGATGGTACTGAAGCGACCGTATCAAAGGGAGTTGCCGATCAAAATGGTAAAGC
GCTGAATGATAGTGTATCATGGGATTATCATAAAACTACTTTTACAGCAACTACACATAATTACAGTT
ATTTAAATTTAACAAATGATGCTAACGAAGTTAATATTCTAAAGTCAAGAATTCCAAAGGAAGCGGAG
CATATAAATGGGGATCGCACGCTCTATCAATTTGGTGCGACATTTACTCAAAAAGCTCTAATGAAAGC
AAATGAAATTTTAGAGACACAAAGTTCTAATGCTAGAAAAAAACTTATTTTTCACGTAACTGATGGTG
TCCCTACGATGTCTTATGCCATAAATTTTAATCCTTATATATCAACATCTTACCAAAACCAGTTTAAT
TCTTTTTTAAATAAAATACCAGATAGAAGTGGTATTCTCCAAGAGGATTTTATAATCAATGGTGATGA
TTATCAAATAGTAAAAGGAGATGGAGAGAGTTTAAACTGTTTCGGATAGAAAAGTTCCTGTTACTG
GAGGAACGACACAAGCAGCTTATCGAGTACCGCAAAATCAACTCTCTGTAATGAGTAATGAGGGATAT
GCAATTAATAGTGGATATATTTATCTCTATTGGAGAGATTACAACTGGGTCTATCCATTTGATCCTAA
GACAAAGAAAGTTTCTGCAACGAAACAAATCAAAACTCATGGTGAGCCAACAACATTATACTTTAATG
GAAATATAAGACCTAAAGGTTATGACATTTTTACTGTTGGGATTGGTGTAAACGGAGATCCTGGTGCA
ACTCCTCTTGAAGCTGAGAAATTTATGCAATCAATATCAAGTAAAACAGAAAATTATACTAATGTTGA
TGATACAAATAAAATTTATGATGAGCTAAATAAATACTTTAAAACAATTGTTGAGGAAAAACATTCTA
TTGTTGATGGAAATGTGACTGATCCTATGGGAGAGATGATTGAATTCCAATTAAAAAATGGTCAAAGT
TTTACACATGATGATTACGTTTTGGTTGGAAATGATGGCAGTCAATTAAAAAATGGTGTGGCTCTTGG
TGGACCAAACAGTGATGGGGGAATTTTAAAAGATGTTACAGTGACTTATGATAAGACATCTCAAACCA
TCAAAATCAATCATTTGAACTTAGGAAGTGGACAAAAAGTAGTTCTTACCTATGATGTACGTTTAAAA
GATAACTATATAAGTAACAAATTTTACAATACAAATAATCGTACAACGCTAAGTCCGAAGAGTGAAAA
AGAACCAAATACTATTCGTGATTTCCCAATTCCCAAAATTCGTGATGTTCGTGAGTTTCCGGTACTAA
CCATCAGTAATCAGAAGAAAATGGGTGAGGTTGAATTTATTAAAGTTAATAAAGACAAACATTCAGAA
TCGCTTTTGGGAGCTAAGTTTCAACTTCAGATAGAAAAAGATTTTTCTGGGTATAAGCAATTTGTTCC
AGAGGGAAGTGATGTTACAACAAAGAATGATGGTAAAATTTATTTTAAAGCACTTCAAGATGGTAACT
ATAAATTATATGAAATTTCAAGTCCAGATGGCTATATAGAGGTTAAAACGAAACCTGTTGTGACATTT
ACAATTCAAAATGGAGAAGTTACGAACCTGAAAGCAGATCCAAATGCTAATAAAAATCAAATCGGGTA
TCTTGAAGGAAATGGTAAACATCTTATTACCAACACTCCCAAACGCCCACCAGGTGTTTTTCCTAAAA
CAGGGGGAATTGGTACAATTGTCTATATATTAGTTGGTTCTACTTTTATGATACTTACCATTTGTTCT
TTCCGTCGTAAACAATTG
**SEQ ID NO. 19**

MKKRQKIWRGLSVTLLILSQIPFGILVQGETQDTNQALGKVIVKKTGDNATPLGKATFVLKNDNDKSE
TSHETVEGSGEATFENIKPGDYTLREETAPIGYKKTDKTWKVKVADNGATIIEGMDADKAEKRKEVLN
AQYPKSAIYEDTKENYPLVNVEGSKVGEQYKALNPINGKDGRREIAEGWLSKKITGVNDLDKNKYKIE
LTVEGKTTVETKELNQPLDVVVLLDNSNSMNNERANNSQRALKAGEAVEKLIDKITSNKDNRVALVTY
ASTIFDGTEATVSKGVADQNGKALNDSVSWDYHKTTFTATTHNYSYLNLTNDANEVNILKSRIPKEAE
HINGDRTLYQFGATFTQKALMKANEILETQSSNARKKLIFHVTDGVPTMSYAINFNPYISTSYQNQFN
SFLNKIPDRSGILQEDFIINGDDYQIVKGDGESFKLFSDRKVPVTGGTTQAAYRVPQNQLSVMSNEGY
AINSGYIYLYWRDYNWVYPFDPKTKKVSATKQIKTHGEPTTLYFNGNIRPKGYDIFTVGIGVNGDPGA
TPLEAEKFMQSISSKTENYTNVDDTNKIYDELNKYFKTIVEEKHSIVDGNVTDPMGEMIEFQLKNGQS

FTHDDYVLVGNDGSQLKNGVALGGPNSDGGILKDVTVTYDKTSQTIKINHLNLGSGQKVVLTYDVRLK
DNYISNKFYNTNNRTTLSPKSEKEPNTIRDFPIPKIRDVREFPVLTISNQKKMGEVEFIKVNKDKHSE
SLLGAKFQLQIEKDFSGYKQFVPEGSDVTTKNDGKIYFKALQDGNYKLYEISSPDGYIEVKTKPVVTF
TIQNGEVTNLKADPNANKNQIGYLEGNGKHLITNTPKRPPGVFPKTGGIGTIVYILVGSTFMILTICS
FRRKQL

GBS 104 contains an N-terminal leader or signal sequence region which is indicated by the underlined sequence at the beginning of SEQ ID NO 19 above. In one embodiment, one or more amino acid sequences from the leader or signal sequence region of GBS 104 are removed. An example of such a GBS 104 fragment is set forth below as SEQ ID NO 20.
**SEQ ID NO 20**

GETQDTNQALGKVIVKKTGDNATPLGKATFVLKNDNDKSETSHETVEGSGEATFENIKPGDYTLREET
APIGYKKTDKTWKVKVADNGATIIEGMDADKAEKRKEVLNAQYPKSAIYEDTKENYPLVNVEGSKVGE
QYKALNPINGKDGRREIAEGWLSKKITGVNDLDKNKYKIELTVEGKTTVETKELNQPLDVVVLLDNSN
SMNNERANNSQRALKAGEAVEKLIDKITSNKDNRVALVTYASTIFDGTEATVSKGVADQNGKALNDSV
SWDYHKTTFTATTHNYSYLNLTNDANEVNILKSRIPKEAEHINGDRTLYQFGATFTQKALMKANEILE
TQSSNARKKLIFHVTDGVPTMSYAINFNPYISTSYQNQFNSFLNKIPDRSGILQEDFIINGDDYQIVK
GDGESFKLFSDRKVPVTGGTTQAAYRVPQNQLSVMSNEGYAINSGYIYLYWRDYNWVYPFDPKTKKVS
ATKQIKTHGEPTTLYFNGNIRPKGYDIFTVGIGVNGDPGATPLEAEKFMQSISSKTENYTNVDDTNKI
YDELNKYFKTIVEEKHSIVDGNVTDPMGEMIEFQLKNGQSFTHDDYVLVGNDGSQLKNGVALGGPNSD
GGILKDVTVTYDKTSQTIKINHLNLGSGQKVVLTYDVRLKDNYISNKFYNTNNRTTLSPKSEKEPNTI
RDFPIPKIRDVREFPVLTISNQKKMGEVEFIKVNKDKHSESLLGAKFQLQIEKDFSGYKQFVPEGSDV
TTKNDGKIYFKALQDGNYKLYEISSPDGYIEVKTKPVVTFTIQNGEVTNLKADPNANKNQIGYLEGNG
KHLITNTPKRPPGVFPKTGGIGTIVYILVGSTFMILTICSFRRKQL

GBS 104 contains a C-terminal transmembrane and/or cytoplasmic region which is indicated by the underlined region near the end of SEQ ID NO 19 above. In one embodiment, one or more amino acids from the transmembrane or cytomplasmic regions are removed. An example of such a GBS 104 fragment is set forth below as SEQ ID NO 21.
**SEQ ID NO: 21**

MKKRQKIWRGLSVTLLILSQIPFGILVQGETQDTNQALGKVIVKKTGDNATPLGKATFVLKNDNDKSE
TSHETVEGSGEATFENIKPGDYTLREETAPIGYKKTDKTWKVKVADNGATIIEGMDADKAEKRKEVLN
AQYPKSAIYEDTKENYPLVNVEGSKVGEQYKALNPINGKDGRREIAEGWLSKKITGVNDLDKNKYKIE
LTVEGKTTVETKELNQPLDVVVLLDNSNSMNNERANNSQRALKAGEAVEKLIDKITSNKDNRVALVTY
ASTIFDGTEATVSKGVADQNGKALNDSVSWDYHKTTFTATTHNYSYLNLTNDANEVNILKSRIPKEAE
HINGDRTLYQFGATFTQKALMKANEILETQSSNARKKLIFHVTDGVPTMSYAINFNPYISTSYQNQFN
SFLNKIPDRSGILQEDFIINGDDYQIVKGDGESFKLFSDRKVPVTGGTTQAAYRVPQNQLSVMSNEGY
AINSGYIYLYWRDYNWVYPFDPKTKKVSATKQIKTHGEPTTLYFNGNIRPKGYDIFTVGIGVNGDPGA
TPLEAEKFMQSISSKTENYTNVDDTNKIYDELNKYFKTIVEEKHSIVDGNVTDPMGEMIEFQLKNGQS
FTHDDYVLVGNDGSQLKNGVALGGPNSDGGILKDVTVTYDKTSQTIKINHLNLGSGQKVVLTYDVRLK
DNYISNKFYNTNNRTTLSPKSEKEPNTIRDFPIPKIRDVREFPVLTISNQKKMGEVEFIKVNKDKHSE
SLLGAKFQLQIEKDFSGYKQFVPEGSDVTTKNDGKIYFKALQDGNYKLYEISSPDGYIEVKTKPVVTF
TIQNGEVTNLKADPNANKNQIGYLEGNGKHLITNT

In one embodiment, one or more amino acids from the leader or signal sequence region and one or more amino acids from the transmembrane or cytoplasmic regions are removed. An example of such a GBS 104 fragment is set forth below as SEQ ID NO 22.
**SEQ ID NO: 22**

GETQDTNQALGKVIVKKTGDNATPLGKATFVLKNDNDKSETSHETVEGSGEATFENIKPGDYTLREET
APIGYKKTDKTWKVKVADNGATIIEGMDADKAEKRKEVLNAQYPKSAIYEDTKENYPLVNVEGSKVGE
QYKALNPINGKDGRREIAEGWLSKKITGVNDLDKNKYKIELTVEGKTTVETKELNQPLDVVVLLDNSN
SMNNERANNSQRALKAGEAVEKLIDKITSNKDNRVALVTYASTIFDGTEATVSKGVADQNGKALNDSV
SWDYHKTTFTATTHNYSYLNLTNDANEVNILKSRIPKEAEHINGDRTLYQFGATFTQKALMKANEILE
TQSSNARKKLIFHVTDGVPTMSYAINFNPYISTSYQNQFNSFLNKIPDRSGILQEDFIINGDDYQIVK
GDGESFKLFSDRKVPVTGGTTQAAYRVPQNQLSVMSNEGYAINSGYIYLYWRDYNWVYPFDPKTKKVS
ATKQIKTHGEPTTLYFNGNIRPKGYDIFTVGIGVNGDPGATPLEAEKFMQSISSKTENYTNVDDTNKI
YDELNKYFKTIVEEKHSIVDGNVTDPMGEMIEFQLKNGQSFTHDDYVLVGNDGSQLKNGVALGGPNSD
GGILKDVTVTYDKTSQTIKINHLNLGSGQKVVLTYDVRLKDNYISNKFYNTNNRTTLSPKSEKEPNTI
RDFPIPKIRDVREFPVLTISNQKKMGEVEFIKVNKDKHSESLLGAKFQLQIEKDFSGYKQFVPEGSDV
TTKNDGKIYFKALQDGNYKLYEISSPDGYIEVKTKPVVTFTIQNGEVTNLKADPNANKNQIGYLEGNG
KHLITNT

In other embodiments, additional fragments of GBS 104 are provided including an 830 amino acid fragment of GBS 104 of amino acids 28-858, a 359 amino acid fragment of GBS 104 of amino acids 28-387, a 581 amino acid fragment of GBS 104 of amino acids 28-609, or a 740 amino acid fragment of GBS 104 of amino acids 28-768.

### GBS 184

GBS 184 refers to a putative lipoprotein. Nucleotide and amino acid sequences of GBS 184 sequenced from serotype V isolated strain 2603 V/R are set forth in Ref. 2 as SEQ ID 1977 and SEQ ID 1978. These sequences are also set forth below as SEQ ID NOS 23 and 24.
**SEQ ID NO: 23** 
ATGAAAAAACAAAAACTATTACTGCTTATTGGAGGCTTATTAATAATGATAATGATGACAGCATGTAA
GGATTCAAAAATCCCAGAAAACCGCACAAAGGAAGAGTACCAGCTGAACAAAATTTTAAACCGTTTT
TTGAGTTTTTAGCACAAAAAGATAAAGATTTGAGCAAAATACAAAAATACTTACTATTAGTATCGGAT
TCAGGTGATGCATTAGATTTAGAATATTTCTATAGTATTCAAGATTTAAAAAAAAATAAGGATTTAGG
GAAGTTTGAAACAAGAAAAAGTCAAATAGAAAAGCCGGGTGGCTATAATGAGTTAGAAAATAAAGAGG
TCCCATTTGAATATTTTAAAAATAATATAGTTTATCCAAAAGGAAAACCGAATATTACATTTGATGAC
TTTATTATCGGAGCAATGGATACTAAAGAATTAAAAGAATTAAAAAAATTAAAAGTAAAAAGTTATTT
ATTAAAACATCCGGAAACTGAGTTGAAAGATATAACATATGAATTGCCGACACAGTCGAAGCTTATTA
AAAAA
**SEQ ID NO: 24**

MKKQKLLLLIGGLLIMIMMTACKDSKIPENRTKEEYQAEQNFKPFFEFLAQKDKDLSKIQKYLLLVSD
SGDALDLEYFYSIQDLKKNKDLGKFETRKSQIEKPGGYNELENKEVPFEYFKNNIVYPKGKPNITFDD
FIIGAMDTKELKELKKLKVKSYLLKHPETELKDITYELPTQSKLIKK

GBS 184 contains a N-terminal leader or signal sequence region which is indicated by the underlined sequence at the beginning of SEQ ID NO 24, above. In one embodiment, one or more amino acids from the leader or signal sequence are removed from GBS 184. An example of such a GBS 184 fragment is set forth below as SEQ ID NO: 25.
**SEQ ID NO: 25**

KDSKIPENRTKEEYQAEQNFKPFFEFLAQKDKDLSKIQKYLLLVSDSGDALDLEYFYSIQDLKKNKDL
GKFETRKSQIEKPGGYNELENKEVPFEYFKNNIVYPKGKPNITFDDFIIGAMDTKELKELKKLKVKSY
LLKHPETELKDITYELPTQSKLIKK

### GBS 276

GBS 276 refers to a C5a peptidase. Nucleotide and amino acid sequences of GBS 276 sequenced from serotype V isolated strain 2603 V/R are set forth in Ref. 2 as SEQ ID 8941 and SEQ ID 8942. These sequences are set forth below as SEQ ID NOS 26 and 27:
**SEQ ID NO. 26** 
TTGCGTAAAAACAAAAACTACCATTTGATAAACTTGCCATTGCGCTTATATCTACGAGCATCTTGCT
CAATGCACAATCAGACATTAAAGCAAATACTGTGACAGAAGACACTCCTGCTACCGAACAAGCCGTAG
AACCCCACAACCAATAGCAGTTTCTGAGGAATCACGATCATCAAAGGAAACTAAAACCTCACAAACT
CCTAGTGATGTAGGAGAAACAGTAGCAGATGACGCTAATGATCTAGCCCCTCAAGCTCCTGCTAAAAC
TGCTGATACACCAGCAACCTCAAAAGCGACTATTAGGGATTTGAACGACCCTTCTCATGTCAAAACCC
TGCAGGAAAAAGCAGGCAAGGGAGCTGGGACCGTTGTTGCAGTGATTGATGCTGGTTTTGATAAAAAT
CATGAAGCGTGGCGCTTAACAGACAAAACTAAAGCACGTTACCAATCAAAAGAAAATCTTGAAAAAGC
TAAAAAAGAGCACGGTATTACCTATGGCGAGTGGGTCAATGATAAGGTTGCTTATTACCACGACTATA
GTAAAGATGGTAAAAACGCTGTTGATCAAGAACACGGCACACACGTGTCAGGGATCTTGTCAGGAAAT
GCTCCATCTGAAATGAAAGAACCTTACCGCCTAGAAGGTGCGATGCCTGAGGCTCAATTGCTTTTGAT
GCGTGTCGAAATTGTAAATGGACTAGCAGACTATGCTCGTAACTACGCTCAAGCTATCAGAGATGCTG
TCAACTTGGGAGCTAAGGTGATTAATATGAGCTTTGGTAATGCTGCACTAGCTTACGCCAACCTTCCA
GACGAAACCAAAAAAGCCTTTGACTATGCCAAATCAAAAGGTGTTAGCATTGTGACCTCAGCTGGTAA
TGATAGTAGCTTTGGGGGCAAGCCCCGTCTACCTCTAGCAGATCATCCTGATTATGGGGTGGTTGGGA
CACCTGCAGCGGCAGATTCAACATTGACAGTTGCTTCTTACAGCCCAGATAAACAGCTCACTGAAACT
GCTACGGTCAAAACAGACGATCATCAAGATAAAGAAATGCCTGTTATTTCAACAAACCGTTTTGAGCC
AAACAAGGCTTACGACTATGCTTATGCTAATCGTGGTACGAAAGAGGATGATTTTAAGGATGTCGAAG
GTAAGATTGCCCTTATTGAACGTGGCGATATTGATTTCAAAGATAAGATTGCAAACGCTAAAAAAGCT
GGTGCTGTAGGGGTCTTGATCTATGACAATCAAGACAAGGGCTTCCCGATTGAATTGCCAAATGTTGA
CCAGATGCCTGCGGCCTTTATCAGTCGAAGAGACGGTCTCTTATTAAAAGACAATCCCCCAAAAACCA
TTACCTTCAATGCGACACCTAAGGTATTGCCAACAGCAAGTGGCACCAAACTAAGCCGCTTCTCAAGC
TGGGGTCTGACAGCTGACGGCAATATTAAACCGGATATTGCAGCACCCGGCCAAGATATTTTGTCATC
AGTGGCTAACAACAAGTATGCCAAACTTTCTGGAACTAGTATGTCTGCACCATTGGTAGCGGGTATCA
TGGGACTGTTGCAAAAGCAATATGAGACACAGTATCCTGATATGACACCATCAGAGCGTCTTGATTTA
GCTAAGAAAGTATTGATGAGCTCAGCAACTGCCCTATATGATGAAGATGAAAAAGCTTATTTTTCTCC
TCGCCAACAGGGAGCAGGAGCAGTCGATGCTAAAAAAGCTTCAGCAGCAACGATGTATGTAACAGATA
AGGACAATACCTCAAGCAAGGTTCACCTGAACAATGTTTCTGATAAATTTGAAGTAACAGTAACAGTT
CACAACAAATCTGATAAACCTCAAGAGTTGTATTACCAAGTAACTGTTCAAACAGATAAAGTAGATGG
AAAACACTTTGCCTTGGCTCCTAAAGCATTGTATGAGACATCATGGCAAAAAATCACAATTCCAGCCA
ATAGCAGCAAACAAGTCACCGTTCCAATCGATGCTAGTCGATTTAGCAAGGACTTGCTTGCCCAAATG
AAAAATGGCTATTTCTTAGAAGGTTTTGTTCGTTTCAAACAAGATCCTACAAAAGAAGAGCTTATGAG
CATTCCATATATTGGTTTCCGAGGTGATTTTGGCAATCTGTCAGCCTTAGAAAAACCAATCTATGATA
 
GCAAAGACGGTAGCAGCTACTATCATGAAGCAAATAGTGATGCCAAAGACCAATTAGATGGTGATGGA
TTACAGTTTTACGCTCTGAAAAATAACTTTACAGCACTTACCACAGAGTCTAACCCATGGACGATTAT
TAAAGCTGTCAAAGAAGGGGTTGAAAACATAGAGGATATCGAATCTTCAGAGATCACAGAAACCATTT
TTGCAGGTACTTTTGCAAAACAAGACGATGATAGCCACTACTATATCCACCGTCACGCTAATGGCAAA
CCATATGCTGCGATCTCTCCAAATGGGGACGGTAACAGAGATTATGTCCAATTCCAAGGTACTTTCTT
GCGTAATGCTAAAAACCTTGTGGCTGAAGTCTTGGACAAAGAAGGAAATGTTGTTTGGACAAGTGAGG
TAACCGAGCAAGTTGTTAAAAACTACAACAATGACTTGGCAAGCACACTTGGTTCAACCCGTTTTGAA
AAAACGCGTTGGGACGGTAAAGATAAAGACGGCAAAGTTGTTGCTAACGGAACCTACACCTATCGTGT
TCGCTACACGCCGATTAGCTCAGGTGCAAAAGAACAACACACTGATTTTGATGTGATTGTAGACAATA
CGACACCTGAAGTCGCAACATCGGCAACATTCTCAACAGAAGATAGTCGTTTGACACTTGCATCTAAA
CCAAAAACCAGCCAACCGGTTTACCGTGAGCGTATTGCTTACACTTATATGGATGAGGATCTGCCAAC
AACAGAGTATATTTCTCCAAATGAAGATGGTACCTTTACTCTTCCTGAAGAGGCTGAAACAATGGAAG
GCGCTACTGTTCCATTGAAAATGTCAGACTTTACTTATGTTGTTGAAGATATGGCTGGTAACATCACT
TATACACCAGTGACTAAGCTATTGGAGGGCCACTCTAATAAGCCAGAACAAGACGGTTCAGATCAAGC
ACCAGACAAGAAACCAGAAGCTAAACCAGAACAAGACGGTTCAGGTCAAACACCAGATAAAAAAAAAG
AAACTAAACCAGAAAAAGATAGTTCAGGTCAAACACCAGGTAAAACTCCTCAAAAAGGTCAATCTTCT
CGTACTCTAGAGAAACGATCTTCTAAGCGTGCTTTAGCTACAAAAGCATCAACAAGAGATCAGTTACC
AACGACTAATGACAAGGATACAAATCGTTTACATCTCCTTAAGTTAGTTATGACCACTTTCTTCTTGG
GA
**SEQ ID NO. 27**

MRKKQKLPFDKLAIALISTSILLNAQSDIKANTVTEDTPATEQAVEPPQPIAVSEESRSSKETKTSQT
PSDVGETVADDANDLAPQAPAKTADTPATSKATIRDLNDPSHVKTLQEKAGKGAGTVVAVIDAGFDKN
HEAWRLTDKTKARYQSKENLEKAKKEHGITYGEWVNDKVAYYHDYSKDGKNAVDQEHGTHVSGILSGN
APSEMKEPYRLEGAMPEAQLLLMRVEIVNGLADYARNYAQAIRDAVNLGAKVINMSFGNAALAYANLP
DETKKAFDYAKSKGVSIVTSAGNDSSFGGKPRLPLADHPDYGVVGTPAAADSTLTVASYSPDKQLTET
ATVKTDDHQDKEMPVISTNRFEPNKAYDYAYANRGTKEDDFKDVEGKIALIERGDIDFKDKIANAKKA
GAVGVLIYDNQDKGFPIELPNVDQMPAAFISRRDGLLLKDNPPKTITFNATPKVLPTASGTKLSRFSS
WGLTADGNIKPDIAAPGQDILSSVANNKYAKLSGTSMSAPLVAGIMGLLQKQYETQYPDMTPSERLDL
AKKVLMSSATALYDEDEKAYFSPRQQGAGAVDAKKASAATMYVTDKDNTSSKVHLNNVSDKFEVTVTV
HNKSDKPQELYYQVTVQTDKVDGKHFALAPKALYETSWQKITIPANSSKQVTVPIDASRFSKDLLAQM
KNGYFLEGFVRFKQDPTKEELMSIPYIGFRGDFGNLSALEKPIYDSKDGSSYYHEANSDAKDQLDGDG
LQFYALKNNFTALTTESNPWTIIKAVKEGVENIEDIESSEITETIFAGTFAKQDDDSHYYIHRHANGK
PYAAISPNGDGNRDYVQFQGTFLRNAKNLVAEVLDKEGNVVWTSEVTEQVVKNYNNDLASTLGSTRFE
KTRWDGKDKDGKVVANGTYTYRVRYTPISSGAKEQHTDFDVIVDNTTPEVATSATFSTEDSRLTLASK
PKTSQPVYRERIAYTYMDEDLPTTEYISPNEDGTFTLPEEAETMEGATVPLKMSDFTYVVEDMAGNIT
YTPVTKLLEGHSNKPEQDGSDQAPDKKPEAKPEQDGSGQTPDKKKETKPEKDSSGQTPGKTPQKGQSS
RTLEKRSSKRALATKASTRDQLPTTNDKDTNRLHLLKLVMTTFFLG

GBS 276 contains an N-terminal leader or signal sequence region which is indicated by the underlined sequence at the beginning of SEQ ID NO: 27 above. In one embodiment, one or more amino acids from the leader or signal sequence region of GBS 276 are removed. An example of such a GBS 276 fragment is set forth below as SEQ ID NO: 28.
**SEQ ID NO: 28**

QSDIKANTVTEDTPATEQAVEPPQPIAVSEESRSSKETKTSQTPSDVGETVADDANDLAPQAPAKTAD
TPATSKATIRDLNDPSHVKTLQEKAGKGAGTVVAVIDAGFDKNHEAWRLTDKTKARYQSKENLEKAKK
EHGITYGEWVNDKVAYYHDYSKDGKNAVDQEHGTHVSGILSGNAPSEMKEPYRLEGAMPEAQLLLMRV
EIVNGLADYARNYAQAIRDAVNLGAKVINMSFGNAALAYANLPDETKKAFDYAKSKGVSIVTSAGNDS
SFGGKPRLPLADHPDYGVVGTPAAADSTLTVASYSPDKQLTETATVKTDDHQDKEMPVISTNRFEPNK
AYDYAYANRGTKEDDFKDVEGKIALIERGDIDFKDKIANAKKAGAVGVLIYDNQDKGFPIELPNVDQM
PAAFISRRDGLLLKDNPPKTITFNATPKVLPTASGTKLSRFSSWGLTADGNIKPDIAAPGQDILSSVA
NNKYAKLSGTSMSAPLVAGIMGLLQKQYETQYPDMTPSERLDLAKKVLMSSATALYDEDEKAYFSPRQ
QGAGAVDAKKASAATMYVTDKDNTSSKVHLNNVSDKFEVTVTVHNKSDKPQELYYQVTVQTDKVDGKH
FALAPKALYETSWQKITIPANSSKQVTVPIDASRFSKDLLAQMKNGYFLEGFVRFKQDPTKEELMSIP
YIGFRGDFGNLSALEKPIYDSKDGSSYYHEANSDAKDQLDGDGLQFYALKNNFTALTTESNPWTIIKA
VKEGVENIEDIESSEITETIFAGTFAKQDDDSHYYIHRHANGKPYAAISPNGDGNRDYVQFQGTFLRN
AKNLVAEVLDKEGNVVWTSEVTEQVVKNYNNDLASTLGSTRFEKTRWDGKDKDGKVVANGTYTYRVRY
TPISSGAKEQHTDFDVIVDNTTPEVATSATFSTEDSRLTLASKPKTSQPVYRERIAYTYMDEDLPTTE
YISPNEDGTFTLPEEAETMEGATVPLKMSDFTYVVEDMAGNITYTPVTKLLEGHSNKPEQDGSDQAPD
KKPEAKPEQDGSGQTPDKKKETKPEKDSSGQTPGKTPQKGQSSRTLEKRSSKRALATKASTRDQLPTT
NDKDTNRLHLLKLVMTTFFLG

GBS 276 contains a C-terminal transmembrane and/or cytoplasmic region which is indicated by the underlined sequence near the end of SEQ ID NO: 27 above. In one embodiment, one or more amino acids from the transmembrane or cytoplasmic regions of GBS 276 are removed. An example of such a GBS 276 fragment is set forth below as SEQ ID NO: 29.
**SEQ ID NO: 29**

MRKKQKLPFDKLAIALISTSILLNAQSDIKANTVTEDTPATEQAVEPPQPIAVSEESRSSKETKTSQT
PSDVGETVADDANDLAPQAPAKTADTPATSKATIRDLNDPSHVKTLQEKAGKGAGTVVAVIDAGFDKN
HEAWRLTDKTKARYQSKENLEKAKKEHGITYGEWVNDKVAYYHDYSKDGKNAVDQEHGTHVSGILSGN
APSEMKEPYRLEGAMPEAQLLLMRVEIVNGLADYARNYAQAIRDAVNLGAKVINMSFGNAALAYANLP
DETKKAFDYAKSKGVSIVTSAGNDSSFGGKPRLPLADHPDYGVVGTPAAADSTLTVASYSPDKQLTET
ATVKTDDHQDKEMPVISTNRFEPNKAYDYAYANRGTKEDDFKDVEGKIALIERGDIDFKDKIANAKKA
GAVGVLIYDNQDKGFPIELPNVDQMPAAFISRRDGLLLKDNPPKTITFNATPKVLPTASGTKLSRFSS
WGLTADGNIKPDIAAPGQDILSSVANNKYAKLSGTSMSAPLVAGIMGLLQKQYETQYPDMTPSERLDL
AKKVLMSSATALYDEDEKAYFSPRQQGAGAVDAKKASAATMYVTDKDNTSSKVHLNNVSDKFEVTVTV
HNKSDKPQELYYQVTVQTDKVDGKHFALAPKALYETSWQKITIPANSSKQVTVPIDASRFSKDLLAQM
KNGYFLEGFVRFKQDPTKEELMSIPYIGFRGDFGNLSALEKPIYDSKDGSSYYHEANSDAKDQLDGDG
LQFYALKNNFTALTTESNPWTIIKAVKEGVENIEDIESSEITETIFAGTFAKQDDDSHYYIHRHANGK
PYAAISPNGDGNRDYVQFQGTFLRNAKNLVAEVLDKEGNVVWTSEVTEQVVKNYNNDLASTLGSTRFE
KTRWDGKDKDGKVVANGTYTYRVRYTPISSGAKEQHTDFDVIVDNTTPEVATSATFSTEDSRLTLASK
PKTSQPVYRERIAYTYMDEDLPTTEYISPNEDGTFTLPEEAETMEGATVPLKMSDFTYVVEDMAGNIT
YTPVTKLLEGHSNKPEQDGSDQAPDKKPEAKPEQDGSGQTPDKKKETKPEKDSSGQTPGKTPQKGQSS
RTLEKRSSKRALATK

In one embodiment, one or more amino acids from the leader or signal sequence region and one or more amino acids from the transmembrane or cytoplasmic regions of GBS 276 are removed. An example of such a GBS 276 fragment is set forth below as SEQ ID NO: 30.
**SEQ ID NO: 30**

QSDIKANTVTEDTPATEQAVEPPQPIAVSEESRSSKETKTSQTPSDVGETVADDANDLAPQAPAKTAD
TPATSKATIRDLNDPSHVKTLQEKAGKGAGTVVAVIDAGFDKNHEAWRLTDKTKARYQSKENLEKAKK
EHGITYGEWVNDKVAYYHDYSKDGKNAVDQEHGTHVSGILSGNAPSEMKEPYRLEGAMPEAQLLLMRV
EIVNGLADYARNYAQAIRDAVNLGAKVINMSFGNAALAYANLPDETKKAFDYAKSKGVSIVTSAGNDS
SFGGKPRLPLADHPDYGVVGTPAAADSTLTVASYSPDKQLTETATVKTDDHQDKEMPVISTNRFEPNK
AYDYAYANRGTKEDDFKDVEGKIALIERGDIDFKDKIANAKKAGAVGVLIYDNQDKGFPIELPNVDQM
PAAFISRRDGLLLKDNPPKTITFNATPKVLPTASGTKLSRFSSWGLTADGNIKPDIAAPGQDILSSVA
NNKYAKLSGTSMSAPLVAGIMGLLQKQYETQYPDMTPSERLDLAKKVLMSSATALYDEDEKAYFSPRQ
QGAGAVDAKKASAATMYVTDKDNTSSKVHLNNVSDKFEVTVTVHNKSDKPQELYYQVTVQTDKVDGKH
FALAPKALYETSWQKITIPANSSKQVTVPIDASRFSKDLLAQMKNGYFLEGFVRFKQDPTKEELMSIP
YIGFRGDFGNLSALEKPIYDSKDGSSYYHEANSDAKDQLDGDGLQFYALKNNFTALTTESNPWTIIKA
VKEGVENIEDIESSEITETIFAGTFAKQDDDSHYYIHRHANGKPYAAISPNGDGNRDYVQFQGTFLRN
AKNLVAEVLDKEGNVVWTSEVTEQVVKNYNNDLASTLGSTRFEKTRWDGKDKDGKVVANGTYTYRVRY
TPISSGAKEQHTDFDVIVDNTTPEVATSATFSTEDSRLTLASKPKTSQPVYRERIAYTYMDEDLPTTE
YISPNEDGTFTLPEEAETMEGATVPLKMSDFTYVVEDMAGNITYTPVTKLLEGHSNKPEQDGSDQAPD
KKPEAKPEQDGSGQTPDKKKETKPEKDSSGQTPGKTPQKGQSSRTLEKRSSKRALATK

Further description of GBS 276 can be found in the following references: Qi Chen et al., "Immunization with C5a Peptidase or Peptidase-Type III Polysaccharide conjugate Vaccines Enhances Clearance of Group B Streptococci from Lungs of Infected Mice", Infection and Immunity (2002) 70 (11):6409 - 6415; Beckmann et al., "Identification of Novel Adhesions from Group B Streptococci by Use of Phage Display Reveals that C5a Peptidase Mediates Fibronectin Binding" Infection and Immunity (2002) 70(6):2869 - 2876; Cheng et al., "The Group B Streptococcal C5a Peptidase Is Both a Specific Protease and an Invasin" Infection and Immunity (2002) 70(5) 2408 - 2413; and Cheng et al., "Antibody against Surface-Bound C5a Peptidase Is Opsonic and Initiates Macrophage Killing of Group B Streptococci" Infection and Immunity (2001) 69(4):2302 - 2308.

### GBS 305

GBS 305 refers to a UDP-N-acetylmuramoylalanine-D-glutamate ligase, also referred to as Mur D. Nucleotide and amino acid sequences of GBS 305 sequenced from serotype V isolated strain 2603 V/R are set forth in Ref. 2 as SEQ ID 207 and SEQ ID 208. These sequences are set forth below as SEQ ID NOS 31 and 32:
**SEQ ID NO. 31** 
ATGGGACGAGTAATGAAAACAATAACAACATTTGAAAATAAAAAGTTTTAGTCCTTGGTTTAGCACG
ATCTGGAGAAGCTGCTGCACGTTTGTTAGCTAAGTTAGGAGCAATAGTGACAGTTAATGATGGCAAAC
CATTTGATGAAAATCCAACAGCACAGTCTTTGTTGGAAGAGGGTATTAAAGTGGTTTGTGGTAGTCAT
CCTTTAGAATTGTTAGATGAGGATTTTTGTTACATGATTAAAAATCCAGGAATACCTTATAACAATCC
TATGGTCAAAAAAGCATTAGAAAAACAAATCCCTGTTTTGACTGAAGTGGAATTAGCATACTTAGTTT
CAGAATCTCAGCTAATAGGTATTACAGGCTCTAACGGGAAAACGACAACGACAACGATGATTGCAGAA
GTCTTAAATGCTGGAGGTCAGAGAGGTTTGTTAGCTGGGAATATCGGCTTTCCTGCTAGTGAAGTTGT
TCAGGCTGCGAATGATAAAGATACTCTAGTTATGGAATTATCAAGTTTTCAGCTAATGGGAGTTAAGG
AATTTCGTCCTCATATTGCAGTAATTACTAATTTAATGCCAACTCATTTAGATTATCATGGGTCTTTT
GAAGATTATGTTGCTGCAAAATGGAATATCCAAAATCAAATGTCTTCATCTGATTTTTTGGTACTTAA
TTTTAATCAAGGTATTTCTAAAGAGTTAGCTAAAACTACTAAAGCAACAATCGTTCCTTTCTCTACTA
CGGAAAAAGTTGATGGTGCTTACGTACAAGACAAGCAACTTTTCTATAAAGGGGAGAATATTATGTCA
 
GTAGATGACATTGGTGTCCCAGGAAGCCATAACGTAGAGAATGCTCTAGCAACTATTGCGGTTGCTAA
ACTGGCTGGTATCAGTAATCAAGTTATTAGAGAAACTTTAAGCAATTTTGGAGGTGTTAAACACCGCT
TGCAATCACTCGGTAAGGTTCATGGTATTAGTTTCTATAACGACAGCAAGTCAACTAATATATTGGCA
ACTCAAAAAGCATTATCTGGCTTTGATAATACTAAAGTTATCCTAATTGCAGGAGGTCTTGATCGCGG
TAATGAGTTTGATGAATTGATACCAGATATCACTGGACTTAAACATATGGTTGTTTTAGGGGAATCGG
CATCTCGAGTAAAACGTGCTGCACAAAAAGCAGGAGTAACTTATAGCGATGCTTTAGATGTTAGAGAT
GCGGTACATAAAGCTTATGAGGTGGCACAACAGGGCGATGTTATCTTGCTAAGTCCTGCAAATGCATC
ATGGGACATGTATAAGAATTTCGAAGTCCGTGGTGATGAATTCATTGATACTTTCGAAAGTCTTAGAG
GAGAG
**SEQ ID NO.32**

MGRVMKTITTFENKKVLVLGLARSGEAAARLLAKLGAIVTVNDGKPFDENPTAQSLLEEGIKVVCGSH
PLELLDEDFCYMIKNPGIPYNNPMVKKALEKQIPVLTEVELAYLVSESQLIGITGSNGKTTTTTMIAE
VLNAGGQRGLLAGNIGFPASEVVQAANDKDTLVMELSSFQLMGVKEFRPHIAVITNLMPTHLDYHGSF
EDYVAAKWNIQNQMSSSDFLVLNFNQGISKELAKTTKATIVPFSTTEKVDGAYVQDKQLFYKGENIMS
VDDIGVPGSHNVENALATIAVAKLAGISNQVIRETLSNFGGVKHRLQSLGKVHGISFYNDSKSTNILA
TQKALSGFDNTKVILIAGGLDRGNEFDELIPDITGLKHMVVLGESASRVKRAAQKAGVTYSDALDVRD
AVHKAYEVAQQGDVILLSPANASWDMYKNFEVRGDEFIDTFESLRGE

GBS 305 contains an N-terminal leader or signal sequence region which is indicated by the underlined sequence at the beginning of SEQ ID NO: 32 above. In one embodiment, one or more amino acids from the leader or signal sequence region are removed from GBS 305. An example of such a GBS 305 fragment is set forth below as SEQ ID NO: 33.
**SEQ ID NO: 33**

ITTFENKKVLVLGLARSGEAAARLLAKLGAIVTVNDGKPFDENPTAQSLLEEGIKVVCGSHPLELLDE
DFCYMIKNPGIPYNNPMVKKALEKQIPVLTEVELAYLVSESQLIGITGSNGKTTTTTMIAEVLNAGGQ
RGLLAGNIGFPASEVVQAANDKDTLVMELSSFQLMGVKEFRPHIAVITNLMPTHLDYHGSFEDYVAAK
WNIQNQMSSSDFLVLNFNQGISKELAKTTKATIVPFSTTEKVDGAYVQDKQLFYKGENIMSVDDIGVP
GSHNVENALATIAVAKLAGISNQVIRETLSNFGGVKHRLQSLGKVHGISFYNDSKSTNILATQKALSG
FDNTKVILIAGGLDRGNEFDELIPDITGLKHMVVLGESASRVKRAAQKAGVTYSDALDVRDAVHKAYE
VAQQGDVILLSPANASWDMYKNFEVRGDEFIDTFESLRGE

GBS 305 contains a C-terminal transmembrane or cytoplasmic region indicated by the underlined sequence near the end of SEQ ID NO: 32 above. In one embodiment, one or more amino acids from the transmembrane or cytomplasmic regions are removed from GBS 305. An example of such a GBS 305 fragment is set forth below as SEQ ID NO: 34.
**SEQ ID NO: 34**

MGRVMKTITT FENKKVLVLGLARSGEAAARLLAKLGAIVTVNDGKPFDENPTAQSLLEEGIKVVCGSH
PLELLDEDFC YMIKNPGIPYNNPMVKKALEKQIPVLTEVELAYLVSESQLIGITGSNGKTTTTTMIAE
VLNAGGQRGLLAGNIGFPASEVVQAANDKDTLVMELSSFQLMGVKEFRPHIAVITNLMPTHLDYHGSF
EDYVAAKWNI QNQMSSSDFLVLNFNQGISKELAKTTKATIVPFSTTEKVDGAYVQDKQLFYKGENIMS
VDDIGVPGSHNVENALATIAVAKLAGISNQVIRETLSNFGGVKHRLQSLGKVHGISFYNDSK

In one embodiment one or more amino acids from the leader or signal sequence region and one or more amino acids from the transmembrane or cytoplasmic regions are removed from GBS 305. An example of such a GBS 305 fragment is set forth below as SEQ ID NO: 35.
**SEQ ID NO: 35**

ITTFENKKVLVLGLARSGEAAARLLAKLGAIVTVNDGKPFDENPTAQSLLEEGIKVVCGSHPLELLDE
DFCYMIKNPGIPYNNPMVKKALEKQIPVLTEVELAYLVSESQLIGITGSNGKTTTTTMIAEVLNAGGQ
RGLLAGNIGFPASEVVQAANDKDTLVMELSSFQLMGVKEFRPHIAVITNLMPTHLDYHGSFEDYVAAK
WNIQNQMSSSDFLVLNFNQGISKELAKTTKATIVPFSTTEKVDGAYVQDKQLFYKGENIMSVDDIGVP
GSHNVENALATIAVAKLAGISNQVIRETLSNFGGVKHRLQSLGKVHGISFYNDSK

### GBS 322

GBS 322 refers to a surface immunogenic protein, also referred to as "sip". Nucleotide and amino acid sequences of GBS 322 sequenced from serotype V isolated strain 2603 V/R are set forth in Ref. 2 as SEQ ID 8539 and SEQ ID 8540. These sequences are set forth below as SEQ ID NOS 36 and 37:
**SEQ ID NO. 36** 
ATGAATAAAAAGGTACTATTGACATCGACAATGGCAGCTTCGCTATTATCAGTCGCAAGTGTTCAAGC
ACAAGAAACAGATACGACGTGGACAGCACGTACTGTTTCAGAGGTAAAGGCTGATTTGGTAAAGCAAG
ACAATAAATCATCATATACTGTGAAATATGGTGATACACTAAGCGTTATTTCAGAAGCAATGTCAATT
GATATGAATGTCTTAGCAAAAATAAATAACATTGCAGATATCAATCTTATTTATCCTGAGACAACACT
GACAGTAACTTACGATCAGAAGAGTCATACTGCCACTTCAATGAAAATAGAAACACCAGCAACAAATG
CTGCTGGTCAAACAACAGCTACTGTGGATTTGAAAACCAATCAAGTTTCTGTTGCAGACCAAAAAGTT
TCTCTCAATACAATTTCGGAAGGTATGACACCAGAAGCAGCAACAACGATTGTTTCGCCAATGAAGAC
ATATTCTTCTGCGCCAGCTTTGAAATCAAAAGAAGTATTAGCACAAGAGCAAGCTGTTAGTCAAGCAG
CAGCTAATGAACAGGTATCACCAGCTCCTGTGAAGTCGATTACTTCAGAAGTTCCAGCAGCTAAAGAG
GAAGTTAAACCAACTCAGACGTCAGTCAGTCAGTCAACAACAGTATCACCAGCTTCTGTTGCCGCTGA
AACACCAGCTCCAGTAGCTAAAGTAGCACCGGTAAGAACTGTAGCAGCCCCTAGAGTGGCAAGTGTTA
AAGTAGTCACTCCTAAAGTAGAAACTGGTGCATCACCAGAGCATGTATCAGCTCCAGCAGTTCCTGTG
ACTACGACTTCACCAGCTACAGACAGTAAGTTACAAGCGACTGAAGTTAAGAGCGTTCCGGTAGCACA
AAAAGCTCCAACAGCAACACCGGTAGCACAACCAGCTTCAACAACAAATGCAGTAGCTGCACATCCTG
AAAATGCAGGGCTCCAACCTCATGTTGCAGCTTATAAAGAAAAAGTAGCGTCAACTTATGGAGTTAAT
GAATTCAGTACATACCGTGCGGGAGATCCAGGTGATCATGGTAAAGGTTTAGCAGTTGACTTTATTGT
AGGTACTAATCAAGCACTTGGTAATAAAGTTGCACAGTACTCTACACAAAATATGGCAGCAAATAACA
TTTCATATGTTATCTGGCAACAAAAGTTTTACTCAAATACAAACAGTATTTATGGACCTGCTAATACT
TGGAATGCAATGCCAGATCGTGGTGGCGTTACTGCCAACCACTATGACCACGTTCACGTATCATTTAA
CAAATAATATAAAAAGGAAGCTATTTGGCTTCTTTTTATATGCCTTGAATAGACTTTCAAGGTTCT
TATATAATTTTTATTA
**SEQ ID NO. 37**

MNKKVLLTSTMAASLLSVASVQAQETDTTWTARTVSEVKADLVKQDNKSSYTVKYGDTLSVISEAMSI
DMNVLAKINNIADINLIYPETTLTVTYDQKSHTATSMKIETPATNAAGQTTATVDLKTNQVSVADQKV
SLNTISEGMTPEAATTIVSPMKTYSSAPALKSKEVLAQEQAVSQAAANEQVSPAPVKSITSEVPAAKE
EVKPTQTSVSQSTTVSPASVAAETPAPVAKVAPVRTVAAPRVASVKVVTPKVETGASPEHVSAPAVPV
TTTSPATDSKLQATEVKSVPVAQKAPTATPVAQPASTTNAVAAHPENAGLQPHVAAYKEKVASTYGVN
EFSTYRAGDPGDHGKGLAVDFIVGTNQALGNKVAQYSTQNMAANNISYVIWQQKFYSNTNSIYGPANT
WNAMPDRGGVTANHYDHVHVSFNK

GBS 322 contains an N-terminal leader or signal sequence region which is indicated by the underlined sequence near the beginning of SEQ ID NO: 37. In one embodiment, one or more amino acids from the leader or signal sequence region of GBS 322 are removed. An example of such a GBS 322 fragment is set forth below as SEQ ID NO: 38.
**SEQ ID NO: 38**

DLVKQDNKSSYTVKYGDTLSVISEAMSIDMNVLAKINNIADINLIYPETTLTVTYDQKSHTATSMKIE
TPATNAAGQTTATVDLKTNQVSVADQKVSLNTISEGMTPEAATTIVSPMKTYSSAPALKSKEVLAQEQ
AVSQAAANEQVSPAPVKSITSEVPAAKEEVKPTQTSVSQSTTVSPASVAAETPAPVAKVAPVRTVAAP
RVASVKVVTPKVETGASPEHVSAPAVPVTTTSPATDSKLQATEVKSVPVAQKAPTATPVAQPASTTNA
VAAHPENAGLQPHVAAYKEKVASTYGVNEFSTYRAGDPGDHGKGLAVDFIVGTNQALGNKVAQYSTQN
MAANNISYVIWQQKFYSNTNSIYGPANTWNAMPDRGGVTANHYDHVHVSFNK

### GBS 330

GBS 330 refers to a pyruvate kinase, also referred to as "pyk". Nucleotide and amino acid sequences of GBS 330 sequenced from serotype V isolated strain 2603 V/R are set forth in Ref. 2 as SEQ ID 8791 and SEQ ID 8792. These sequences are set forth below as SEQ ID NOS 39 and 40:
**SEQ ID NO. 39** 
ATGAATAAACGCGTAAAAATCGTTGCAACACTTGGTCCTGCGGTTGAATTCCGTGGTGGTAAGAAGTT
TGGTGAGTCTGGATACTGGGGTGAAAGCCTTGACGTAGAAGCTTCAGCAGAAAAAATTGCTCAATTGA
TTAAAGAAGGTGCTAACGTTTTCCGTTTCAACTTCTCACATGGAGATCATGCTGAGCAAGGAGCTCGT
ATGGCTACTGTTCGTAAAGCAGAAGAGATTGCAGGACAAAAAGTTGGCTTCCTCCTTGATACTAAAGG
ACCTGAAATTCGTACAGAACTTTTTGAAGATGGTGCAGATTTCCATTCATATACAACAGGTACAAAAT
TACGTGTTGCTACTAAGCAAGGTATCAAATCAACTCCAGAAGTGATTGCATTGAATGTTGCTGGTGGA
CTTGACATCTTTGATGACGTTGAAGTTGGTAAGCAAATCCTTGTTGATGATGGTAAACTAGGTCTTAC
TGTGTTTGCAAAAGATAAAGACACTCGTGAATTTGAAGTAGTTGTTGAGAATGATGGCCTTATTGGTA
AACAAAAAGGTGTAAACATCCCTTATACTAAAATTCCTTTCCCAGCACTTGCAGAACGCGATAATGCT
GATATCCGTTTTGGACTTGAGCAAGGACTTAACTTTATTGCTATCTCATTTGTACGTACTGCTAAAGA
TGTTAATGAAGTTCGTGCTATTTGTGAAGAAACTGGSMATGGACACGTTAAGTTGTTTGCTAAAATTG
AAAATCAACAAGGTATCGATAATATTGATGAGATTATCGAAGCAGCAGATGGTATTATGATTGCTCGT
GGTGATATGGGTATCGAAGTTCCATTTGAAATGGTTCCAGTTTACCAAAAAATGATCATTACTAAAGT
TAATGCAGCTGGTAAAGCAGTTATTACAGCAACAAATATGCTTGAAACAATGACTGATAAACCACGTG
CGACTCGTTCAGAAGTATCTGATGTCTTCAATGCTGTTATTGATGGTACTGATGCTACAATGCTTTCA
GGTGAGTCAGCTAATGGTAAATACCCAGTTGAGTCAGTTCGTACAATGGCTACTATTGATAAAAATGC
TCAAACATTACTCAATGAGTATGGTCGCTTAGACTCATCTGCATTCCCACGTAATAACAAAACTGATG
TTATTGCATCTGCGGTTAAAGATGCAACACACTCAATGGATATCAAACTTGTTGTAACAATTACTGAA
ACAGGTAATACAGCTCGTGCCATTTCTAAATTCCGTCCAGATGCAGACATTTTGGCTGTTACATTTGA
TGAAAAAGTACAACGTTCATTGATGATTAACTGGGGTGTTATCCCTGTCCTTGCAGACAAACCAGCAT
CTACAGATGATATGTTTGAGGTTGCAGAACGTGTAGCACTTGAAGCAGGATTTGTTGAATCAGGCGAT
AATATCGTTATCGTTGCAGGTGTTCCTGTAGGTACAGGTGGAACTAACACAATGCGTGTTCGTACTGT
TAAA
**SEQ ID NO. 40**

MNKRVKIVATLGPAVEFRGGKKFGESGYWGESLDVEASAEKIAQLIKEGANVFRFNFSHGDHAEQGAR
MATVRKAEEIAGQKVGFLLDTKGPEIRTELFEDGADFHSYTTGTKLRVATKQGIKSTPEVIALNVAGG
LDIFDDVEVGKQILVDDGKLGLTVFAKDKDTREFEVVVENDGLIGKQKGVNIPYTKIPFPALAERDNA
DIRFGLEQGLNFIAISFVRTAKDVNEVRAICEETGXGHVKLFAKIENQQGIDNIDEIIEAADGIMIAR
GDMGIEVPFEMVPVYQKMIITKVNAAGKAVITATNMLETMTDKPRATRSEVSDVFNAVIDGTDATMLS
GESANGKYPVESVRTMATIDKNAQTLLNEYGRLDSSAFPRNNKTDVIASAVKDATHSMDIKLVVTITE
TGNTARAISKFRPDADILAVTFDEKVQRSLMINWGVIPVLADKPASTDDMFEVAERVALEAGFVESGD
NIVIVAGVPVGTGGTNTMRVRTVK

### GBS 338

GBS 338 refers to a Sat D protein. Nucleotide and amino acid sequences of GBS 338 sequenced from serotype V isolated strain 2603 V/R are set forth in Ref. 2 as SEQ ID 8637 and SEQ ID 8638. These sequences are set forth below as SEQ ID NOS 41 and 42:
**SEQ ID NO. 41** 
TTGTCTGCTATAATAGACAAAAAGGTGGTGATATTTATGTATTTAGCATTAATCGGTGATATCATTAA
TTCAAAACAGATACTTGAACGTGAAACTTTCCAACAGTCTTTTCAGCAACTAATGACCGAACTATCTG
ATGTATATGGTGAAGAGCTGATTTCTCCATTCACTATTACAGCTGGTGATGAATTTCAAGCTTTATTG
AAACCATCAAAAAAGGTATTTCAAATTATTGACCATATTCAACTAGCTCTAAAACCTGTTAATGTAAG
GTTCGGCCTCGGTACAGGAAACATTATAACATCCATCAATTCAAATGAAAGTATCGGTGCTGATGGTC
CTGCCTACTGGCATGCTCGCTCAGCTATTAATCATATACATGATAAAAATGATTATGGAACAGTTCAA
GTAGCTATTTGCCTTGATGATGAAGACCAAAACCTTGAATTAACACTAAATAGTCTCATTTCAGCTGG
TGATTTTATCAAGTCAAAATGGACTACAAACCATTTTCAAATGCTTGAGCACTTAATACTTCAAGATA
ATTATCAAGAACAATTTCAACATCAAAAGTTAGCCCAACTGGAAAATATTGAACCTAGTGCGCTGACT
AAACGCCTTAAAGCAAGCGGTCTGAAGATTTACTTAAGAACGAGAACACAGGCAGCCGATCTATTAGT
TAAAAGTTGCACTCAAACTAAAGGGGGAAGCTATGATTTC
**SEQ ID NO. 42**

MSAIIDKKVVIFMYLALIGDIINSKQILERETFQQSFQQLMTELSDVYGEELISPFTITAGDEFQALL
KPSKKVFQIIDHIQLALKPVNVRFGLGTGNIITSINSNESIGADGPAYWHARSAINHIHDKNDYGTVQ
VAICLDDEDQNLELTLNSLISAGDFIKSKWTTNHFQMLEHLILQDNYQEQFQHQKLAQLENIEPSALT
KRLKASGLKIYLRTRTQAADLLVKSCTQTKGGSYDF

GBS 338 may contain an N-terminal leader or signal sequence region which is indicated by the underlined sequence at the beginning of SEQ ID NO: 42 above. In one embodiment, one or more amino acids from the leader or signal sequence region are removed from GBS 338. An example of such a GBS 338 fragment is set forth below as SEQ ID NO: 43.
**SEQ ID NO: 43**

MYLALIGDIINSKQILERETFQQSFQQLMTELSDVYGEELISPFTITAGDEFQALLKPSKKVFQIIDH
IQLALKPVNVRFGLGTGNIITSINSNESIGADGPAYWHARSAINHIHDKNDYGTVQVAICLDDEDQNL
ELTLNSLISAGDFIKSKWTTNHFQMLEHLILQDNYQEQFQHQKLAQLENIEPSALTKRLKASGLKIYL
RTRTQAADLLVKSCTQTKGGSYDF

### GBS 361

GBS 361 refers to a cylI protein. Nucleotide and amino acid sequences of GBS 361 sequenced from serotype V isolated strain 2603 V/R are set forth in Ref. 2 as SEQ ID 8769 and SEQ ID 8770. These sequences are set forth below as SEQ ID NOS 44 and 45:
**SEQ ID NO. 44** 
ATGAGCGTATATGTTAGTGGAATAGGAATTATTTCTTCTTTGGGAAAGAATTATAGCGAGCATAAACA
GCATCTCTTCGACTTAAAAGAAGGAATTTCTAAACATTTATATAAAAATCACGACTCTATTTAGAAT
CTTATACAGGAAGCATAACTAGTGACCCAGAGGTTCCTGAGCAATACAAAGATGAGACACGTAATTTT
AAATTTGCTTTTACCGCTTTTGAAGAGGCTCTTGCTTCTTCAGGTGTTAATTTAAAAGCTTATCATAA
TATTGCTGTGTGTTTAGGGACCTCACTTGGGGGAAAGAGTGCTGGTCAAAATGCCTTGTATCAATTTG
AAGAAGGAGAGCGTCAAGTAGATGCTAGTTTATTAGAAAAAGCATCTGTTTACCATATTGCTGATGAA
TTGATGGCTTATCATGATATTGTGGAGCTTCGTATGTTATTTCAACCGCCTGTTCTGCAAGTAATAA
TGCCGTAATATTAGGAACACAATTACTTCAAGATGGCGATTGTGATTTAGCTATTTGTGGTGGCTGTG
ATGAGTTAAGTGATATTTCTTTAGCAGGCTTCACATCACTAGGAGCTATTAATACAGAAATGGCATGT
CAGCCCTATTCTTCTGGAAAAGGAATCAATTTGGGTGAGGGCGCTGGTTTTGTTGTTCTTGTCAAAGA
TCAGTCCTTAGCTAAATATGGAAAAATTATCGGTGGTCTTATTACTTCAGATGGTTATCATATAACAG
CACCTAAGCCAACAGGTGAAGGGGCGGCACAGATTGCAAAGCAGCTAGTGACTCAAGCAGGTATTGAC
TACAGTGAGATTGACTATATTAACGGTCACGGTACAGGTACTCAAGCTAATGATAAAATGGAAAAAAA
TATGTATGGTAAGTTTTTCCCGACAACGACATTGATCAGCAGTACCAAGGGGCAAACGGGTCATACTC
TAGGGGCTGCAGGTATTATCGAATTGATTAATTGTTTAGCGGCAATAGAGGAACAGACTGTACCAGCA
ACTAAAAATGAGATTGGGATAGAAGGTTTTCCAGAAAATTTTGTCTATCATCAAAAGAGAGAATACCC
AATAAGAAATGCTTTAAATTTTTCGTTTGCTTTTGGTGGAAATAATAGTGGTGTCTTATTGTCATCTT
TAGATTCACCTCTAGAAACATTACCTGCTAGAGAAAATCTTAAAATGGCTATCTTATCATCTGTTGCT
TCCATTTCTAAGAATGAATCACTTTCTATAACCTATGAAAAAGTTGCTAGTAATTTCAACGACTTTGA
AGCATTACGCTTTAAAGGGGCTAGACCACCCAAAACTGTCAACCCAGCACAATTTAGGAAAATGGATG
ATTTTTCCAAAATGGTTGCCGTAACAACAGCTCAAGCACTAATAGAAAGCAATATTAATCTAAAAAAA
CAAGATACTTCAAAAGTAGGAATTGTATTTACAACACTTTCTGGACCAGTTGAGGTTGTTGAAGGTAT
TGAAAAGCAAATCACAACAGAAGGATATGCACATGTTTCTGCTTCACGATTCCCGTTTACAGTAATGA
ATGCAGCAGCTGGTATGCTTTCTATCATTTTTAAAATAACAGGTCCTTTATCTGTCATTTCGACAAAT
AGTGGAGCGCTTGATGGTATACAATATGCCAAGGAAATGATGCGTAACGATAATCTAGACTATGTGAT
TCTTGTTTCTGCTAATCAGTGGACAGACATGAGTTTTATGTGGTGGCAACAATTAAACTATGATAGTC
AAATGTTTGTCGGTTCTGATTATTGTTCAGCACAAGTCCTCTCTCGTCAAGCATTGGATAATTCTCCT
ATAATATTAGGTAGTAAACAATTAAAATATAGCCATAAAACATTCACAGATGTGATGACTATTTTTGA
TGCTGCGCTTCAAAATTTATTATCAGACTTAGGACTAACCATAAAAGATATCAAAGGTTTCGTTTGGA
ATGAGCGGAAGAAGGCAGTTAGTTCAGATTATGATTTCTTAGCGAACTTGTCTGAGTATTATAATATG
CCAAACCTTGCTTCTGGTCAGTTTGGATTTTCATCTAATGGTGCTGGTGAAGAACTGGACTATACTGT
TAATGAAAGTATAGAAAAGGGCTATTATTTAGTCCTATCTTATTCGATCTTCGGTGGTATCTCTTTTG
CTATTATTGAAAAAAGG
**SEQ ID NO. 45**

MSVYVSGIGIISSLGKNYSEHKQHLFDLKEGISKHLYKNHDSILESYTGSITSDPEVPEQYKDETRNF
KFAFTAFEEALASSGVNLKAYHNIAVCLGTSLGGKSAGQNALYQFEEGERQVDASLLEKASVYHIADE
LMAYHDIVGASYVISTACSASNNAVILGTQLLQDGDCDLAICGGCDELSDISLAGFTSLGAINTEMAC
QPYSSGKGINLGEGAGFVVLVKDQSLAKYGKIIGGLITSDGYHITAPKPTGEGAAQIAKQLVTQAGID
YSEIDYINGHGTGTQANDKMEKNMYGKFFPTTTLISSTKGQTGHTLGAAGIIELINCLAAIEEQTVPA
TKNEIGIEGFPENFVYHQKREYPIRNALNFSFAFGGNNSGVLLSSLDSPLETLPARENLKMAILSSVA
SISKNESLSITYEKVASNFNDFEALRFKGARPPKTVNPAQFRKMDDFSKMVAVTTAQALIESNINLKK
QDTSKVGIVFTTLSGPVEVVEGIEKQITTEGYAHVSASRFPFTVMNAAAGMLSIIFKITGPLSVISTN
SGALDGIQYAKEMMRNDNLDYVILVSANQWTDMSFMWWQQLNYDSQMFVGSDYCSAQVLSRQALDNSP

IILGSKQLKYSHKTFTDVMTIFDAALQNLLSDLGLTIKDIKGFVWNERKKAVSSDYDFLANLSEYYNM
PNLASGQFGFSSNGAGEELDYTVNESIEKGYYLVLSYSIFGGISFAIIEKR

GBS 361 may contain an N-terminal leader or signal sequence region which is indicated by the underlined sequence at the beginning of SEQ ID NO: 45 above. In one embodiment, one or more amino acids from the leader or signal sequence region are removed from GBS 361. An example of such a GBS 361 fragment is set forth below as SEQ ID NO: 46.
**SEQ ID NO: 46**

VSGIGIISSLGKNYSEHKQHLFDLKEGISKHLYKNHDSILESYTGSITSDPEVPEQYKDETRNFKFAF
TAFEEALASSGVNLKAYHNIAVCLGTSLGGKSAGQNALYQFEEGERQVDASLLEKASVYHIADELMAY
HDIVGASYVISTACSASNNAVILGTQLLQDGDCDLAICGGCDELSDISLAGFTSLGAINTEMACQPYS
SGKGINLGEGAGFVVLVKDQSLAKYGKIIGGLITSDGYHITAPKPTGEGAAQIAKQLVTQAGIDYSEI
DYINGHGTGTQANDKMEKNMYGKFFPTTTLISSTKGQTGHTLGAAGIIELINCLAAIEEQTVPATKNE
IGIEGFPENFVYHQKREYPIRNALNFSFAFGGNNSGVLLSSLDSPLETLPARENLKMAILSSVASISK
NESLSITYEKVASNFNDFEALRFKGARPPKTVNPAQFRKMDDFSKMVAVTTAQALIESNINLKKQDTS
KVGIVFTTLSGPVEVVEGIEKQITTEGYAHVSASRFPFTVMNAAAGMLSIIFKITGPLSVISTNSGAL
DGIQYAKEMMRNDNLDYVILVSANQWTDMSFMWWQQLNYDSQMFVGSDYCSAQVLSRQALDNSPIILG
SKQLKYSHKTFTDVMTIFDAALQNLLSDLGLTIKDIKGFVWNERKKAVSSDYDFLANLSEYYNMPNLA
SGQFGFSSNGAGEELDYTVNESIEKGYYLVLSYSIFGGISFAIIEKR

### GB S 404

Nucleotide and amino acid sequences of GBS 404 sequenced from serotype V isolated strain 2603 V/R are set forth in Ref. 2 as SEQ ID 8799 and SEQ ID 8800. These sequences are set forth below as SEQ ID NOS 47 and 48:
**SEQ ID NO. 47** 
ATGAAAATAGATGACCTAAGAAAAAGCGACAATGTTGAAGATCGTCGCTCCAGTAGCGGAGGTTCATT
CTCTAGCGGAGGAAGTGGATTACCGATTCTTCAACTTTTATTGCTGCGAGGAGTTGGAAAACCAAGC
TTGTGGTTTAATCATCTTACTGCTACTTGGCGGAGGGGACTAACCAGCATTTTTAATGACTCATCC
TCACCTTCTAGTTACCAATCTCAGAATGTCTCACGTTCTGTTGATAATAGCGCAACGAGAGAACAAAT
CGATTTCGTTAATAAAGTCCTTGGCTCAACTGAGGATTTCTGGTCACAAGAATTCCAAACCCAAGGTT
TTGGAAATTATAAGGAACCAAAACTTGTTCTTTACACCAATTCAATTCAAACAGGTTGTGGTATAGGT
GAATCTGCTTCAGGACCATTTTATTGTTCAGCAGATAAAAAAATCTATCTTGATATTTCTTTTTACAA
TGAATTATCACATAAATATGGTGCTACTGGTGATTTTGCTATGGCCTACGTCATCGCCCACGAAGTTG
GTCACCACATTCAAACAGAGTTAGGCATTATGGATAAGTATAATAGAATGCGACACGGACTTACTAAG
AAAGAAGCAAATGCTTTAAATGTTCGGCTAGAACTTCAAGCAGATTATTATGCAGGGGTATGGGCTCA
CTACATCAGGGGAAAAAATCTCTTAGAACAAGGAGACTTTGAAGAGGCCATGAATGCTGCCCACGCCG
TCGGAGACGATACCCTTCAGAAAGAAACCTACGGAAAATTAGTGCCTGATAGCTTTACCCATGGAACA
GCTGAACAACGCCAACGTTGGTTTAACAAAGGCTTTCAATATGGTGACATCCAACACGGTGATACTTT
CTCCGTAGAACATCTA
**SEQ ID NO. 48**

MKIDDLRKSDNVEDRRSSSGGSFSSGGSGLPILQLLLLRGSWKTKLVVLIILLLLGGGGLTSIFNDSS
SPSSYQSQNVSRSVDNSATREQIDFVNKVLGSTEDFWSQEFQTQGFGNYKEPKLVLYTNSIQTGCGIG
ESASGPFYCSADKKIYLDISFYNELSHKYGATGDFAMAYVIAHEVGHHIQTELGIMDKYNRMRHGLTK
KEANALNVRLELQADYYAGVWAHYIRGKNLLEQGDFEEAMNAAHAVGDDTLQKETYGKLVPDSFTHGT
AEQRQRWFNKGFQYGDIQHGDTFSVEHL

### GBS 690

Nucleotide and amino acid sequences of GBS 690 sequenced from serotype V isolated strain 2603 V/R are set forth in Ref. 2 as SEQ ID 9965 and SEQ ID 9966. These sequences are set forth as SEQ ID NOS 49 and 50 below:
**SEQ ID NO. 49** 
ATGAGTAAACGACAAAATTTAGGAATTAGTAAAAAAGGAGCAATTATATCAGGGCTCTCAGTGGCACT
AATTGTAGTAATAGGTGGCTTTTTATGGGTACAATCTCAACCTAATAAGAGTGCAGTAAAAACTAACT
ACAAAGTTTTTAATGTTAGAGAAGGAAGTGTTTCGTCCTCAACTCTTTTGACAGGAAAAGCTAAGGCT
AATCAAGAACAGTATGTGTATTTTGATGCTAATAAAGGTAATCGAGCAACTGTCACAGTTAAAGTGGG
TGATAAAATCACAGCTGGTCAGCAGTTAGTTCAATATGATACAACAACTGCACAAGCAGCCTACGACA
CTGCTAATCGTCAATTAAATAAAGTAGCGCGTCAGATTAATAATCTAAAGACAACAGGAAGTCTTCCA
GCTATGGAATCAAGTGATCAATCTTCTTCATCATCACAAGGACAAGGGACTCAATCGACTAGTGGTGC
GACGAATCGTCTACAGCAAAATTATCAAAGTCAAGCTAATGCTTCATACAACCAACAACTTCAAGATT
TGAATGATGCTTATGCAGATGCACAGGCAGAAGTAAATAAAGCACAAAAAGCATTGAATGATACTGTT
ATTACAAGTGACGTATCAGGGACAGTTGTTGAAGTTAATAGTGATATTGATCCAGCTTCAAAAACTAG
TCAAGTACTTGTCCATGTAGCAACTGAAGGTAAACTCCAAGTACAAGGAACGATGAGTGAGTATGATT
TGGCTAATGTTAAAAAAGACCAGGCTGTTAAAATAAAATCTAAGGTCTATCCTGACAAGGAATGGGAA
GGTAAAATTTCATATATCTCAAATTATCCAGAAGCAGAAGCAAACAACAATGACTCTAATAACGGCTC
TAGTGCTGTAAATTATAAATATAAAGTAGATATTACTAGCCCTCTCGATGCATTAAAACAAGGTTTTA
CCGTATCAGTTGAAGTAGTTAATGGAGATAAGCACCTTATTGTCCCTACAAGTTCTGTGATAAACAAA
GATAATAAACACTTTGTTTGGGTATACAATGATTCTAATCGTAAAATTTCCAAAGTTGAAGTCAAAAT
TGGTAAAGCTGATGCTAAGACACAAGAAATTTTATCAGGTTTGAAAGCAGGACAAATCGTGGTTACTA
ATCCAAGTAAAACCTTCAAGGATGGGCAAAAAATTGATAATATTGAATCAATCGATCTTAACTCTAAT
AAGAAATCAGAGGTGAAA
**SEQ ID NO. 50**

MSKRQNLGISKKGAIISGLSVALIVVIGGFLWVQSQPNKSAVKTNYKVFNVREGSVSSSTLLTGKAKA
NQEQYVYFDANKGNRATVTVKVGDKITAGQQLVQYDTTTAQAAYDTANRQLNKVARQINNLKTTGSLP
AMESSDQSSSSSQGQGTQSTSGATNRLQQNYQSQANASYNQQLQDLNDAYADAQAEVNKAQKALNDTV
ITSDVSGTVVEVNSDIDPASKTSQVLVHVATEGKLQVQGTMSEYDLANVKKDQAVKIKSKVYPDKEWE
GKISYISNYPEAEANNNDSNNGSSAVNYKYKVDITSPLDALKQGFTVSEVVNGDKHLIVPTSSVINK
DNKHFVWVYNDSNRKISKVEVKIGKADAKTQEILSGLKAGQIVVTNPSKTFKDGQKIDNIESIDLNSN
KKSEVK

GBS 690 contains an N-terminal leader or signal sequence region which is indicated by the underlined sequence at the beginning of SEQ ID NO: 50 above. In one embodiment, one or more amino acids from the leader or signal sequence region of GBS 690 are removed. An example of such a GBS 690 fragment is set forth below as SEQ ID NO: 51.
**SEQ ID NO: 51**

FLWVQSQPNKSAVKTNYKVFNVREGSVSSSTLLTGKAKANQEQYVYFDANKGNRATVTVKVGDKITAG
QQLVQYDTTTAQAAYDTANRQLNKVARQINNLKTTGSLPAMESSDQSSSSSQGQGTQSTSGATNRLQQ
NYQSQANASYNQQLQDLNDAYADAQAEVNKAQKALNDTVITSDVSGTVVEVNSDIDPASKTSQVLVHV
ATEGKLQVQGTMSEYDLANVKKDQAVKIKSKVYPDKEWEGKISYISNYPEAEANNNDSNNGSSAVNYK
YKVDITSPLDALKQGFTVSVEVVNGDKHLIVPTSSVINKDNKHFVWVYNDSNRKISKVEVKIGKADAK
TQEILSGLKAGQIVVTNPSKTFKDGQKIDNIESIDLNSNKKSEVK

### GBS 691

GBS 691 refers to an iron compound ABC transporter, or a substrate binding protein. Nucleotide and amino acid sequences of GBS 691 sequenced from serotype V isolated strain 2603 V/R are set forth in Ref. 2 as SEQ ID 3691 and SEQ ID 3692. These sequences are set forth as SEQ ID NOS 52 and 53 below:
**SEQ ID NO. 52** 
ATGAAAAAATTGGAATTATTGTCCTCACACTACTGACCTTCTTTTTGGTATCTTGCGGACAACAAAC
TAAACAAGAAAGCACTAAAACAACTATTTCTAAAATGCCTAAAATTGAAGGCTTCACCTATTATGGAA
AAATTCCTGAAAATCCGAAAAAAGTAATTAATTTTACATATTCTTACACTGGGTATTTATTAAAACTA
GGTGTTAATGTTTCAAGTTACAGTTTAGACTTAGAAAAAGATAGCCCCGTTTTTGGTAAACAACTGAA
AGAAGCTAAAAAATTAACTGCTGATGATACAGAAGCTATTGCCGCACAAAAACCTGATTTAATCATGG
TTTTCGATCAAGATCCAAACATCAATACTCTGAAAAAAATTGCACCAACTTTAGTTATTAAATATGGT
GCACAAAATTATTTAGATATGATGCCAGCCTTGGGGAAAGTATTCGGTAAAGAAAAAGAAGCTAATCA
GTGGGTTAGCCAATGGAAAACTAAAACTCTCGCTGTCAAAAAAGATTTACACCATATCTTAAAGCCTA
ACACTACTTTTACTATTATGGATTTTTATGATAAAAATATCTATTTATATGGTAATAATTTTGGACGC
GGTGGAGAACTAATCTATGATTCACTAGGTTATGCTGCCCCAGAAAAAGTCAAAAAAGATGTCTTTAA
AAAAGGGTGGTTTACCGTTTCGCAAGAAGCAATCGGTGATTACGTTGGAGATTATGCCCTTGTTAATA
TAAACAAAACGACTAAAAAAGCAGCTTCATCACTTAAAGAAAGTGATGTCTGGAAGAATTTACCAGCT
GTCAAAAAAGGGCACATCATAGAAAGTAACTACGACGTGTTTTATTTCTCTGACCCTCTATCTTTAGA
AGCTCAATTAAAATCATTTACAAAGGCTATCAAAGAAAATACAAAT
**SEQ ID NO. 53**

MKKIGIIVLTLLTFFLVSCGQQTKQESTKTTISKMPKIEGFTYYGKIPENPKKVINFTYSYTGYLLKL
GVNVSSYSLDLEKDSPVFGKQLKEAKKLTADDTEAIAAQKPDLIMVFDQDPNINTLKKIAPTLVIKYG
AQNYLDMMPALGKVFGKEKEANQWVSQWKTKTLAVKKDLHHILKPNTTFTIMDFYDKNIYLYGNNFGR
GGELIYDSLGYAAPEKVKKDVFKKGWFTVSQEAIGDYVGDYALVNINKTTKKAASSLKESDVWKNLPA
VKKGHIIESNYDVFYFSDPLSLEAQLKSFTKAIKENTN

GBS 691 contains an N-terminal leader or signal sequence region which is indicated by the underlined sequence at the beginning of SEQ ID NO: 53 above. In one embodiment, one or more amino acids are removed from the leader or signal sequence region of GBS 691. An example of such a GBS 691 fragment is set forth below as SEQ ID NO: 54.
**SEQ ID NO: 54**

EGFTYYGKIPENPKKVINFTYSYTGYLLKLGVNVSSYSLDLEKDSPVFGKQLKEAKKLTADDTEAIAA
QKPDLIMVFDQDPNINTLKKIAPTLVIKYGAQNYLDMMPALGKVFGKEKEANQWVSQWKTKTLAVKKD
LHHILKPNTTFTIMDFYDKNIYLYGNNFGRGGELIYDSLGYAAPEKVKKDVFKKGWFTVSQEAIGDYV
GDYALVNINKTTKKAASSLKESDVWKNLPAVKKGHIIESNYDVFYFSDPLSLEAQLKSFTKAIKENTN

GBS 691 contains a C-terminal transmembrane or cytosplasmic region which is indicated by the underlined sequence at the end of SEQ ID NO: 53 above. In one embodiment, one or more amino acids are removed from the transmembrane or cytoplasmic region of GBS 691. An example of such a GBS 691 fragment is set forth below as SEQ ID NO: 55.
**SEQ ID NO: 55**

MKKIGIIVLTLLTFFLVSCGQQTKQESTKTTISKMPKIEGFTYYGKIPENPKKVINFTYSYTGYLLKL
GVNVSSYSLDLEKDSPVFGKQLKEAKKLTADDTEAIAAQKPDLIMVFDQDPNINTLKKIAPTLVIKYG
AQNYLDMMPALGKVFGKEKEANQWVSQWKTKTLAVKKDLHHILKPNTTFTIMDFYDKNIYLYGNNFGR
GGELIYDSLGYAAPEKVKKDVFKKGWFTVSQEAIGDYVGDYALVNINKTTKKAASSLKESDVWKNLPA
VKKGHIIESNYDVFYFSDPLSLEAQLKSFT

In one embodiment, one or more amino acids from the leader or signal sequence region and one or more amino acids from the transmembrane or cytosplasmic region are removed from GBS 691. One example of such a GBS 691 fragment is set forth below as SEQ ID NO: 56
**SEQ ID NO: 56**

EGFTYYGKIPENPKKVINFTYSYTGYLLKLGVNVSSYSLDLEKDSPVFGKQLKEAKKLTADDTEAIAA
QKPDLIMVFDQDPNINTLKKIAPTLVIKYGAQNYLDMMPALGKVFGKEKEANQWVSQWKTKTLAVKKD
LHHILKPNTTFTIMDFYDKNIYLYGNNFGRGGELIYDSLGYAAPEKVKKDVFKKGWFTVSQEAIGDYV
GDYALVNINKTTKKAASSLKESDVWKNLPAVKKGHIIESNYDVFYFSDPLSLEAQLKSFT

Additional examples of GBS antigens which may be used in combination with GBS 80 are set forth below.

### GBS 4

GBS 4 refers to another putative cell wall surface anchor family protein. Nucleotide and amino acid sequences of GBS 4 sequenced from serotype V isolated strain 2603 V/R are set forth in Ref. 2 as SEQ ID 1 and SEQ ID 2. These sequences are also set forth below as SEQ ID NOS 57 and 58:
**SEQ ID NO. 57** 
ATGAAAGTGAAAAATAAGATTTTAACGATGGTAGCACTTACTGTCTTAACATGTGCTACTTATTCATC
AATCGGTTATGCTGATACAAGTGATAAGAATACTGACACGAGTGTCGTGACTACGACCTTATCTGAGG
AGAAAAGATCAGATGAACTAGACCAGTCTAGTACTGGTTCTTCTTCTGAAAATGAATCGAGTTCATCA
AGTGAACCAGAAACAAATCCGTCAACTAATCCACCTACAACAGAACCATCGCAACCCTCACCTAGTGA
AGAGAACAAGCCTGATGGTAGAACGAAGACAGAAATTGGCAATAATAAGGATATTTCTAGTGGAACAA
AAGTATTAATTTCAGAAGATAGTATTAAGAATTTTAGTAAAGCAAGTAGTGATCAAGAAGAAGTGGAT
CGCGATGAATCATCATCTTCAAAAGCAAATGATGGAAAAAAGGCCACAGTAAGCCTAAAAAGGAACT
TCCTAAAACAGGAGATAGCCACTCAGATACTGTAATAGCATCTACGGGAGGGATTATTCTGTTATCAT
TAAGTTTTTACAATAAGAAAATGAAACTTTAT
**SEQ ID NO. 58**

MKVKNKILTMVALTVLTCATYSSIGYADTSDKNTDTSVVTTTLSEEKRSDELDQSSTGSSSENESSSS
SEPETNPSTNPPTTEPSQPSPSEENKPDGRTKTEIGNNKDISSGTKVLISEDSIKNFSKASSDQEEVD
RDESSSSKANDGKKGHSKPKKELPKTGDSHSDTVIASTGGIILLSLSFYNKKMKLY

GBS 4 contains an N-terminal leader or signal sequence which is underlined at the beginning of SEQ ID NO: 58 above. In one embodiment, one or more amino acids from the N-terminal leader or signal peptide domain of GBS 4 are removed. An example of such a GBS 4 fragment is set forth below as SEQ ID NO 59.
**SEQ ID NO 59**

DTSDKNTDTSVVTTTLSEEKRSDELDQSSTGSSSENESSSSSEPETNPSTNPPTTEPSQPSPSEENKP
DGRTKTEIGNNKDISSGTKVLISEDSIKNFSKASSDQEEVDRDESSSSKANDGKKGHSKPKKELPKTG
DSHSDTVIASTGGIILLSLSFYNKKMKLY

A further N-terminal section of GBS 4 may be removed to facilitate recombinant expression. An example of such a GBS 4 fragment is set forth below as SEQ ID NO: 60.
**SEQ ID NO: 60**

DQSSTGSSSENESSSSSEPETNPSTNPPTTEPSQPSPSEENKPDGRTKTEIGNNKDISSGTKVLISED
S IKNFSKASSDQEEVDRDESSSSKANDGKKGHSKPKKELPKTGDSHSDTVIASTGGIILLSLSFYNKK
MKLY

GBS 4 contains an C-terminal transmembrane region which is underlined at the end of SEQ ID NO: 58 above. In one embodiment, one or more amino acids from the C-terminal transmembrane region is removed. An example of such a GBS 4 fragment is set forth below as SEQ ID NO: 61.
**SEQ ID NO: 61**

MKVKNKILTMVALTVLTCATYSSIGYADTSDKNTDTSVVTTTLSEEKRSDELDQSSTGSSSENESSSS
SEPETNPSTNPPTTEPSQPSPSEENKPDGRTKTEIGNNKDISSGTKVLISEDSIKNFSKASSDQEEVD
RDESSSSKANDGKKGHSKPKKE

In one embodiment, both the N-terminal leader or signal domain and the C-terminal transmembrane domain are removed from the GBS 4 sequence. An example of such a GBS 4 fragment is set forth below as SEQ ID NO: 62.
**SEQ ID NO: 62**

DTSDKNTDTSVVTTTLSEEKRSDELDQSSTGSSSENESSSSSEPETNPSTNPPTTEPSQPSPSEENKP
DGRTKTEIGNNKDISSGTKVLISEDSIKNFSKASSDQEEVDRDESSSSKANDGKKGHSKPKKE

In yet another embodiment, the N-terminal leader or signal domain, a further N-terminal region and the C-terminal transmembrane domain are removed from the GBS 4 sequence. An example of such a GBS 4 fragment is set forth below as SEQ ID NO: 63.
**SEQ ID NO: 63**

DQSSTGSSSENESSSSSEPETNPSTNPPTTEPSQPSPSEENKPDGRTKTEIGNNKDISSGTKVLISED
SIKNFSKASSDQEEVDRDESSSSKANDGKKGHSKPKKE

### GBS 22

GBS 22 refers to a putative adhesion lipoprotein. Nucleotide and amino acid sequences of GBS 22 sequenced from serotype V isolated strain 2603 V/R are set forth in Ref. 2 as SEQ 8583 and SEQ ID 8584. These sequences are set forth below as SEQ ID NOS 64 and 65:
**SEQ ID NO. 64** 
ATGAAAAGGATACGGAAAAGCCTTATTTTTGTTCTCGGAGTAGTTACCCTAATTTGCTTATGTGCTTG
TACTAAACAAAGCCAGCAAAAAAATGGCTTGTCAGTAGTGACTAGCTTTATCCAGTATATTCCATTA
CAAAAGCAGTTTCTGGTGATTTGAATGATATTAAAATGATTCGATCACAGTCAGGTATTCATGGTTTT
GAACCCTCATCAAGTGATGTTGCTGCCATTTATGATGCTGATCTATTTCTTTATCATTCGCACACACT
AGAAGCTTGGGCGAGACGTTTGGAACCTAGTTTGCATCACTCTAAAGTATCTGTAATTGAAGCTTCAA
AAGGTATGACTTTGGATAAAGTTCATGGCTTAGAAGATGTAGAGGCAGAAAAAGGAGTAGATGAGTCA
ACCTTGTATGACCCTCACACTTGGAATGACCCTGTAAAAGTATCTGAGGAAGCACAACTCATCGCTAC
ACAATTAGCTAAAAAGGATCCTAAAAACGCTAAGGTTTATCAAAAAAATGCTGATCAATTTAGTGACA
AGGCAATGGCTATTGCAGAGAAGTATAAGCCAAAATTTAAAGCTGCAAAGTCTAAATACTTTGTGACT
TCACATACAGCATTCTCATACTTAGCTAAGCGATACGGATTGACTCAGTTAGGTATTGCAGGTGTCTC
AACCGAGCAAGAACCTAGTGCTAAAAAATTAGCCGAAATTCAGGAGTTTGTGAAAACATATAAGGTTA
AGACTATTTTTGTTGAAGAAGGAGTCTCACCTAAATTAGCTCAAGCAGTAGCTTCAGCTACTCGAGTT
AAAATTGCAAGTTTAAGTCCTTTARAAGCAGTTCCCAAAAACAATAAAGATTACTTAGAAAATTTGGA
AACTAATCTTAAGGTACTTGTCAAATCGTTAAATCAATAG
**SEQ ID NO. 65**

MKRI RKSLIFVLGVVTLICLCACTKQSQQKNGLSVVTSFYPVYSITKAVSGDLNDIKMIRSQSGIHGF
EPSS SDVAAIYDADLFLYHSHTLEAWARRLEPSLHHSKVSVIEASKGMTLDKVHGLEDVEAEKGVDES
TLYD PHTWNDPVKVSEEAQLIATQLAKKDPKNAKVYQKNADQFSDKAMAIAEKYKPKFKAAKSKYFVT
SHTA FSYLAKRYGLTQLGIAGVSTEQEPSAKKLAEIQEFVKTYKVKTI FVEEGVS PKLAQAVASATRV
KIAS LSPLXAVPKNNKDYLENLETNLKVLVKSLNQ

### GBS 85

GBS 85 refers to a putative cell division protein (DivIB). Nucleotide and amino acid sequences of GBS 85 sequenced from serotype V isolated strain 2603 V/R are set forth in Ref. 2 as SEQ ID 215 and SEQ ID 216. These sequences are set forth below as SEQ ID NOS 66 and 67:
**SEQ ID NO. 66** 
ATGCCTAAGAAGAAATCAGATACCCCAGAAAAAGAAGAAGTTGTCTTAACGGAATGGCAAAAGCGTAA
CCTTGAATTTTTAAAAAAACGCAAAGAAGATGAAGAACAAAAACGTATTAACGAAAAATTACGCT
TAGATAAAAGAAGTAAATTAAATATTTCTTCTCCTGAAGAACCTCAAAATACTACTAAAATTAAGAAG
CTTCATTTTCCAAAGATTTCAAGACCTAAGATTGAAAAGAAACAGAAAAAAGAAAAAATAGTCAACAG
CTTAGCCAAAACTAATCGCATTAGAACTGCACCTATATTTGTAGTAGCATTCCTAGTCATTTTAGTTT
CCGTTTTCCTACTAACTCCTTTTAGTAAGCAAAAAACAATAACAGTTAGTGGAAATCAGCATACACCT
GATGATATTTTGATAGAGAAAACGAATATTCAAAAAAACGATTATTTCTTTTCTTTAATTTTTAAACA
TAAAGCTATTGAACAACGTTTAGCTGCAGAAGATGTATGGGTAAAAACAGCTCAGATGACTTATCAAT
TTCCCAATAAGTTTCATATTCAAGTTCAAGAAAATAAGATTATTGCATATGCACATACAAAGCAAGGA
TATCAACCTGTCTTGGAAACTGGAAAAAAGGCTGATCCTGTAAATAGTTCAGAGCTACCAAAGCACTT
CTTAACAATTAACCTTGATAAGGAAGATAGTATTAAGCTATTAATTAAAGATTTAAAGGCTTTAGACC
CTGATTTAATAAGTGAGATTCAGGTGATAAGTTTAGCTGATTCTAAAACGACACCTGACCTCCTGCTG
TTAGATATGCACGATGGAAATAGTATTAGAATACCATTATCTAAATTTAAAGAAAGACTTCCTTTTTA
CAAACAAATTAAGAAGAACCTTAAGGAACCTTCTATTGTTGATATGGAAGTGGGAGTTTACACAACAA
CAAATACCATTGAATCAACCCCTGTTAAAGCAGAAGATACAAAAAATAAATCAACTGATAAAACACAA
ACACAAAATGGTCAGGTTGCGGAAAATAGTCAAGGACAAACAAATAACTCAAATACTAATCAACAAGG
ACAACAGATAGCAACAGAGCAGGCACCTAACCCTCAAAATGTTAAT
**SEQ ID NO. 67**

MPKKKSDTPEKEEVVLTEWQKRNLEFLKKRKEDEEEQKRINEKLRLDKRSKLNISSPEEPQNTTKIKK
LHFPKISRPKIEKKQKKEKIVNSLAKTNRIRTAPIFVVAFLVILVSVFLLTPFSKQKTITVSGNQHTP
DDILIEKTNIQKNDYFFSLIFKHKAIEQRLAAEDVWVKTAQMTYQFPNKFHIQVQENKIIAYAHTKQG

YQPVLETGKKADPVNSSELPKHFLTINLDKEDSIKLLIKDLKALDPDLISEIQVISLADSKTTPDLLL
LDMHDGNSIRIPLSKFKERLPFYKQIKKNLKEPSIVDMEVGVYTTTNTIESTPVKAEDTKNKSTDKTQ
TQNGQVAENSQGQTNNSNTNQQGQQIATEQAPNPQNVN

### GB S 147

GBS 147 refers to a putative protease. Nucleotide and amino acid sequences of GBS 147 sequenced from serotype V isolated strain 2603 V/R are set forth in Ref. 2 as SEQ ID 8525 and SEQ ID 8526. These sequences are set forth below as SEQ ID NOS 68 and 69.
**SEQ ID NO. 68** 
GTGGATAAACATCACTCAAAAAGGCTATTTTAAAGTTAACACTTATAACAACTAGTATTTTATTAAT
GCATAGCAATCAAGTGAATGCAGAGGAGCAAGAATTAAAAAACCAAGAGCAATCACCTGTAATTGCTA
ATGTTGCTCAACAGCCATCGCCATCGGTAACTACTAATACTGTTGAAAAAACATCTGTAACAGCTGCT
TCTGCTAGTAATACAGCGAAAGAAATGGGTGATACATCTGTAAAAAATGACAAAACAGAAGATGAATT
ATTAGAAGAGTTATCTAAAAACCTTGATACGTCTAATTTGGGGGCTGATCTTGAAGAAGAATATCCCT
CTAAACCAGAGACAACCAACAATAAAGAAAGCAATGTAGTAACAAATGCTTCAACTGCAATAGCACAG
AAAGTTCCCTCAGCATATGAAGAGGTGAAGCCAGAAAGCAAGTCATCGCTTGCTGTTCTTGATACATC
TAAAATAACAAAATTACAAGCCATAACCCAAAGAGGAAAGGGAAATGTAGTAGCTATTATTGATACTG
GCTTTGATATTAACCATGATATTTTTCGTTTAGATAGCCCAAAAGATGATAAGCACAGCTTTAAAACT
AAGACAGAATTTGAGGAATTAAAAGCAAAACATAATATCACTTATGGAAATGGGTTAACGATAAGAT
TGTTTTTGCACATAACTACGCCAACAATACAGAAACGGTGGCTGATATTGCAGCAGCTATGAAAGATG
GTTATGGTTCAGAAGCAAAGAATATTTCGCATGGTACACACGTTGCTGGTATTTTTGTAGGTAATAGT
AAACGTCCAGCAATCAATGGTCTTCTTTTAGAAGGTGCAGCGCCAAATGCTCAAGTCTTATTAATGCG
TATTCCAGATAAAATTGATTCGGACAAATTTGGTGAAGCATATGCTAAAGCAATCACAGACGCTGTTA
ATCTAGGAGCAAAAACGATTAATATGAGTATTGGAAAAACAGCTGATTCTTTAATTGCTCTCAATGAT
AAAGTTAAATTAGCACTTAAATTAGCTTCTGAGAAGGGCGTTGCAGTTGTTGTGGCTGCCGGAAATGA
AGGCGCATTTGGTATGGATTATAGCAAACCATTATCAACTAATCCTGACTACGGTACGGTTAATAGTC
CAGCTATTTCTGAAGATACTTTGAGTGTTGCTAGCTATGAATCACTTAAAACTATCAGTGAGGTCGTT
GAAACAACTATTGAAGGTAAGTTAGTTAAGTTGCCGATTGTGACTTCTAAACCTTTTGACAAAGGTAA
GGCCTACGATGTGGTTTATGCCAATTATGGTGCAAAAAAAGACTTTGAAGGTAAGGACTTTAAAGGTA
AGATTGCATTAATTGAGCGTGGTGGTGGACTTGATTTTATGACTAAAATCACTCATGCTACAAATGCA
GGTGTTGTTGGTATCGTTATTTTTAACGATCAAGAAAAACGTGGAAATTTTCTAATTCCTTACCGTGA
ATTACCTGTGGGGATTATTAGTAAAGTAGATGGCGAGCGTATAAAAAATACTTCAAGTCAGTTAACAT
TTAACCAGAGTTTTGAAGTAGTTGATAGCCAAGGTGGTAATCGTATGCTGGAACAATCAAGTTGGGGC
GTGACAGCTGAAGGAGCAATCAAGCCTGATGTAACAGCTTCTGGCTTTGAAATTTATTCTTCAACCTA
TAATAATCAATACCAAACAATGTCTGGTACAAGTATGGCTTCACCACATGTTGCAGGATTAATGACAA
TGCTTCAAAGTCATTTGGCTGAGAAATATAAAGGGATGAATTTAGATTCTAAAAAATTGCTAGAATTG
TCTAAAAACATCCTCATGAGCTCAGCAACAGCATTATATAGTGAAGAGGATAAGGCGTTTTATTCACC
ACGTCAGCAAGGTGCAGGTGTAGTTGATGCTGAAAAAGCTATCCAAGCTCAATATTATATTACTGGAA
ACGATGGCAAAGCTAAAATTAATCTCAAACGAATGGGAGATAAATTTGATATCACAGTTACAATTCAT
AAACTTGTAGAAGGTGTCAAAGAATTGTATTATCAAGCTAATGTAGCAACAGAACAAGTAAATAAAGG
TAAATTTGCCCTTAAACCACAAGCCTTGCTAGATACTAATTGGCAGAAAGTAATTCTTCGTGATAAAG
AAACACAAGTTCGATTTACTATTGATGCTAGTCAATTTAGTCAGAAATTAAAAGAACAGATGGCAAAT
GGTTATTTCTTAGAAGGTTTTGTACGTTTAAAGAAGCCAAGGATAGTAATCAGGAGTTAATGAGTAT
TCCTTTTGTAGGATTTAATGGTGATTTTGCGAACTTACAAGCACTTGAAACACCGATTTATAAGACGC
TTTCTAAAGGTAGTTTCTACTATAAACCAAATGATACAACTCATAAAGACCAATTGGAGTACAATGAA
TCAGCTCCTTTTGAAAGCAACAACTATACTGCCTTGTTAACACAATCAGCGTCTTGGGGCTATGTTGA
TTATGTCAAAAATGGTGGGGAGTTAGAATTAGCACCGGAGAGTCCAAAAAGAATTATTTAGGAACTT
TTGAGAATAAGGTTGAGGATAAAACAATTCATCTTTTGGAAAGAGATGCAGCGAATAATCCATATTTT
GCCATTTCTCCAAATAAAGATGGAAATAGGGACGAAATCACTCCCCAGGCAACTTTCTTAAGAAATGT
TAAGGATATTTCTGCTCAAGTTCTAGATCAAAATGGAAATGTTATTTGGCAAAGTAAGGTTTTACCAT
 
CTTATCGTAAAAATTTCCATAATAATCCAAAGCAAAGTGATGGTCATTATCGTATGGATGCTCTTCAG
TGGAGTGGTTTAGATAAGGATGGCAAAGTTGTAGCAGATGGTTTTTATACTTATCGCTTACGTTACAC
ACCAGTAGCAGAAGGAGCAAATAGTCAGGAGTCAGACTTTAAAGTACAAGTAAGTACTAAGTCACCAA
ATCTTCCTTCACGAGCTCAGTTTGATGAAACTAATCGAACATTAAGCTTAGCCATGCCTAAGGAAAGT
AGTTATGTTCCTACATATCGTTTACAATTAGTTTTATCTCATGTTGTAAAAGATGAAGAATATGGGGA
TGAGACTTCTTACCATTATTTCCATATAGATCAAGAAGGTAAAGTGACACTTCCTAAAACGGTTAAGA
TAGGAGAGAGTGAGGTTGCGGTAGACCCTAAGGCCTTGACACTTGTTGTGGAAGATAAAGCTGGTAAT
TTCGCAACGGTAAAATTGTCTGATCTCTTGAATAAGGCAGTAGTATCAGAGAAAGAAAACGCTATAGT
AATTTCTAACAGTTTCAAATATTTTGATAACTTGAAAAAAGAACCTATGTTTATTTCTAAAAAAGAAA
AAGTAGTAAACAAGAATCTAGAAGAAATAATATTAGTTAAGCCGCAAACTACAGTTACTACTCAATCA
TTGTCTAAAGAAATAACTAAATCAGGAAATGAGAAAGTCCTCACTTCTACAAACAATAATAGTAGCAG
AGTAGCTAAGATCATATCACCTAAACATAACGGGGATTCTGTTAACCATACCTTACCTAGTACATCAG
ATAGAGCAACGAATGGTCTATTTGTTGGTACTTTGGCATTGTTATCTAGTTTACTTCTTTATTTGAAA
CCCAAAAAGACTAAAAATAATAGTAAA
**SEQ ID NO. 69**

VDKHHSKKAILKLTLITTSILLMHSNQVNAEEQELKNQEQSPVIANVAQQPSPSVTTNTVEKTSVTAA
SASNTAKEMGDTSVKNDKTEDELLEELSKNLDTSNLGADLEEEYPSKPETTNNKESNVVTNASTAIAQ
KVPSAYEEVKPESKSSLAVLDTSKITKLQAITQRGKGNVVAIIDTGFDINHDIFRLDSPKDDKHSFKT
KTEFEELKAKHNITYGKWVNDKIVFAHNYANNTETVADIAAAMKDGYGSEAKNISHGTHVAGIFVGNS
KRPAINGLLLEGAAPNAQVLLMRIPDKIDSDKFGEAYAKAITDAVNLGAKTINMSIGKTADSLIALND
KVKLALKLASEKGVAVVVAAGNEGAFGMDYSKPLSTNPDYGTVNSPAISEDTLSVASYESLKTISEVV
ETTIEGKLVKLPIVTSKPFDKGKAYDVVYANYGAKKDFEGKDFKGKIALIERGGGLDFMTKITHATNA
GVVGIVIFNDQEKRGNFLIPYRELPVGIISKVDGERIKNTSSQLTFNQSFEVVDSQGGNRMLEQSSWG
VTAEGAIKPDVTASGFEIYSSTYNNQYQTMSGTSMASPHVAGLMTMLQSHLAEKYKGMNLDSKKLLEL
SKNILMSSATALYSEEDKAFYSPRQQGAGVVDAEKAIQAQYYITGNDGKAKINLKRMGDKFDITVTIH
KLVEGVKELYYQANVATEQVNKGKFALKPQALLDTNWQKVILRDKETQVRFTIDASQFSQKLKEQMAN
GYFLEGFVRFKEAKDSNQELMSIPFVGFNGDFANLQALETPIYKTLSKGSFYYKPNDTTHKDQLEYNE
SAPFESNNYTALLTQSASWGYVDYVKNGGELELAPESPKRIILGTFENKVEDKTIHLLERDAANNPYF
AISPNKDGNRDEITPQATFLRNVKDISAQVLDQNGNVIWQSKVLPSYRKNFHNNPKQSDGHYRMDALQ
WSGLDKDGKVVADGFYTYRLRYTPVAEGANSQESDFKVQVSTKSPNLPSRAQFDETNRTLSLAMPKES
SYVPTYRLQLVLSHVVKDEEYGDETSYHYFHIDQEGKVTLPKTVKIGESEVAVDPKALTLVVEDKAGN
FATVKLSDLLNKAVVSEKENAIVISNSFKYFDNLKKEPMFISKKEKVVNKNLEEIILVKPQTTVTTQS
LSKEITKSGNEKVLTSTNNNSSRVAKIISPKHNGDSVNHTLPSTSDRATNGLFVGTLALLSSLLLYLK
PKKTKNNSK

GBS 147 contains an N-terminal leader or signal sequence region which is indicated by the underlined sequence at the beginning of SEQ ID NO 69 above. In one embodiment, one or more amino acids from the leader or signal sequence region of GBS 147 are removed. An example of such a GBS 147 fragment is set forth below as SEQ ID NO: 70.
**SEQ ID NO: 70**

EEQELKNQE QSPVIANVAQQPSPSVTTNTVEKTSVTAASASNTAKEMGDTSVKNDKTEDELLEELSKN
LDTSNLGADLEEEYPSKPETTNNKESNVVTNASTAIAQKVPSAYEEVKPESKSSLAVLDTSKITKLQA
ITQRGKGNVVAIIDTGFDINHDIFRLDSPKDDKHSFKTKTEFEELKAKHNITYGKWVNDKIVFAHNYA
NNTETVADI AAAMKDGYGSEAKNISHGTHVAGIFVGNSKRPAINGLLLEGAAPNAQVLLMRIPDKIDS
DKFGEAYAKAITDAVNLGAKTINMSIGKTADSLIALNDKVKLALKLASEKGVAVVVAAGNEGAFGMDY
SKPLSTNPDYGTVNSPAISEDTLSVASYESLKTISEVVETTIEGKLVKLPIVTSKPFDKGKAYDVVYA
NYGAKKDFE GKDFKGKIALIERGGGLDFMTKITHATNAGVVGIVIFNDQEKRGNFLIPYRELPVGIIS
KVDGERIKNTSSQLTFNQSFEVVDSQGGNRMLEQSSWGVTAEGAIKPDVTASGFEIYSSTYNNQYQTM
SGTSMASPHVAGLMTMLQSHLAEKYKGMNLDSKKLLELSKNILMSSATALYSEEDKAFYSPRQQGAGV

VDAEKAIQAQYYITGNDGKAKINLKRMGDKFDITVTIHKLVEGVKELYYQANVATEQVNKGKFALKPQ
ALLDTNWQKVILRDKETQVRFTIDASQFSQKLKEQMANGYFLEGFVRFKEAKDSNQELMSIPFVGFNG
DFANLQALETPIYKTLSKGSFYYKPNDTTHKDQLEYNESAPFESNNYTALLTQSASWGYVDYVKNGGE
LELAPESPKRIILGTFENKVEDKTIHLLERDAANNPYFAISPNKDGNRDEITPQATFLRNVKDISAQV
LDQNGNVIWQSKVLPSYRKNFHNNPKQSDGHYRMDALQWSGLDKDGKVVADGFYTYRLRYTPVAEGAN
SQESDFKVQVSTKSPNLPSRAQFDETNRTLSLAMPKESSYVPTYRLQLVLSHVVKDEEYGDETSYHYF
HIDQEGKVTLPKTVKIGESEVAVDPKALTLVVEDKAGNFATVKLSDLLNKAVVSEKENAIVISNSFKY
FDNLKKEPMFISKKEKVVNKNLEEIILVKPQTTVTTQSLSKEITKSGNEKVLTSTNNNSSRVAKIISP
KHNGDSVNHTLPSTSDRATNGLFVGTLALLSSLLLYLKPKKTKNNSK

GBS 147 also contains a C-terminal transmembrane and/or cytoplasmic region which may be located within the underlined sequence near the end of SEQ ID NO: 69 above. In one embodiment, one or more amino acids from the transmembrane and/or cytoplasmic region are removed. An example of such a GBS 147 fragment is set forth below as SEQ ID NO: 71.
**SEQ ID NO: 71**

VDKHHSKKAILKLTLITTSILLMHSNQVNAEEQELKNQEQSPVIANVAQQPSPSVTTNTVEKTSVTAA
SASNTAKEMGDTSVKNDKTEDELLEELSKNLDTSNLGADLEEEYPSKPETTNNKESNVVTNASTAIAQ
KVPSAYEEVKPESKSSLAVLDTSKITKLQAITQRGKGNVVAIIDTGFDINHDIFRLDSPKDDKHSFKT
KTEFEELKAKHNITYGKWVNDKIVFAHNYANNTETVADIAAAMKDGYGSEAKNISHGTHVAGIFVGNS
KRPAINGLLLEGAAPNAQVLLMRIPDKIDSDKFGEAYAKAITDAVNLGAKTINMSIGKTADSLIALND
KVKLALKLASEKGVAVVVAAGNEGAFGMDYSKPLSTNPDYGTVNSPAISEDTLSVASYESLKTISEVV
ETTIEGKLVKLPIVTSKPFDKGKAYDVVYANYGAKKDFEGKDFKGKIALIERGGGLDFMTKITHATNA
GVVGIVIFNDQEKRGNFLIPYRELPVGIISKVDGERIKNTSSQLTFNQSFEVVDSQGGNRMLEQSSWG
VTAEGAIKPDVTASGFEIYSSTYNNQYQTMSGTSMASPHVAGLMTMLQSHLAEKYKGMNLDSKKLLEL
SKNILMSSATALYSEEDKAFYSPRQQGAGVVDAEKAIQAQYYITGNDGKAKINLKRMGDKFDITVTIH
KLVEGVKELYYQANVATEQVNKGKFALKPQALLDTNWQKVILRDKETQVRFTIDASQFSQKLKEQMAN
GYFLEGFVRFKEAKDSNQELMSIPFVGFNGDFANLQALETPIYKTLSKGSFYYKPNDTTHKDQLEYNE
SAPFESNNYTALLTQSASWGYVDYVKNGGELELAPESPKRIILGTFENKVEDKTIHLLERDAANNPYF
AISPNKDGNRDEITPQATFLRNVKDISAQVLDQNGNVIWQSKVLPSYRKNFHNNPKQSDGHYRMDALQ
WSGLDKDGKVVADGFYTYRLRYTPVAEGANSQESDFKVQVSTKSPNLPSRAQFDETNRTLSLAMPKES
SYVPTYRLQLVLSHVVKDEEYGDETSYHYFHIDQEGKVTLPKTVKIGESEVAVDPKALTLVVEDKAGN
FATVKLSDLLNKAVVSEKENAIVISNSFKYFDNLKKEPMFISKKEKVVNKNLEEIILVKPQTTVTTQS
LSKEITKSGNEKVLTSTNNNSSRVAKIISPKHNGDSVNHT

In one embodiment, one or more amino acids from the leader or signal sequence region and one or more amino acids from the transmembrane or cytoplasmic region are removed from the GBS 147 sequence. An example of such a GBS 147 fragment is set forth below as SEQ ID NO 72.
**SEQ ID NO: 72**

EEQELKNQEQSPVIANVAQQPSPSVTTNTVEKTSVTAASASNTAKEMGDTSVKNDKTEDELLEELSKN
LDTSNLGADLEEEYPSKPETTNNKESNVVTNASTAIAQKVPSAYEEVKPESKSSLAVLDTSKITKLQA
ITQRGKGNVVAIIDTGFDINHDIFRLDSPKDDKHSFKTKTEFEELKAKHNITYGKWVNDKIVFAHNYA
NNTETVADIAAAMKDGYGSEAKNISHGTHVAGIFVGNSKRPAINGLLLEGAAPNAQVLLMRIPDKIDS
DKFGEAYAKAITDAVNLGAKTINMSIGKTADSLIALNDKVKLALKLASEKGVAVVVAAGNEGAFGMDY
SKPLSTNPDYGTVNSPAISEDTLSVASYESLKTISEVVETTIEGKLVKLPIVTSKPFDKGKAYDVVYA
NYGAKKDFEGKDFKGKIALIERGGGLDFMTKITHATNAGVVGIVIFNDQEKRGNFLIPYRELPVGIIS
KVDGERIKNTSSQLTFNQSFEVVDSQGGNRMLEQSSWGVTAEGAIKPDVTASGFEIYSSTYNNQYQTM
SGTSMASPHVAGLMTMLQSHLAEKYKGMNLDSKKLLELSKNILMSSATALYSEEDKAFYSPRQQGAGV

VDAEKAI QAQYYITGNDGKAKINLKRMGDKFDITVTIHKLVEGVKELYYQANVATEQVNKGKFALKPQ
ALLDTNWQKVILRDKETQVRFTIDASQFSQKLKEQMANGYFLEGFVRFKEAKDSNQELMSIPFVGFNG
DFANLQALETPIYKTLSKGSFYYKPNDTTHKDQLEYNESAPFESNNYTALLTQSASWGYVDYVKNGGE
LELAPES PKRIILGTFENKVEDKTIHLLERDAANNPYFAISPNKDGNRDEITPQATFLRNVKDISAQV
LDQNGNVIWQSKVLPSYRKNFHNNPKQSDGHYRMDALQWSGLDKDGKVVADGFYTYRLRYTPVAEGAN
SQESDFKVQVSTKSPNLPSRAQFDETNRTLSLAMPKESSYVPTYRLQLVLSHVVKDEEYGDETSYHYF
HIDQEGKVTLPKTVKIGESEVAVDPKALTLVVEDKAGNFATVKLSDLLNKAVVSEKENAIVISNSFKY
FDNLKKE PMFISKKEKVVNKNLEEIILVKPQTTVTTQSLSKEITKSGNEKVLTSTNNNSSRVAKIISP
KHNGDSVNHT

### GBS 173

GBS 173 refers to an amidase family protein. Nucleotide and amino acid sequences of GBS 173 sequenced from serotype V isolated strain 2603 V/R are set forth in Ref. 2 as SEQ ID 8787 and SEQ ID 8788. These sequences are set forth below as SEQ ID NOS 73 and 74:
**SEQ ID NO. 73** 
ATGAAAC GTAAATACTTTATTCTTAATACGGTGACGGTTTTAACGTTAGCTGCTGCAATGAATACTAG
CAGTATC TATGCTAATAGTACTGAGACAAGTGCTTCAGTAGTTCCTACTACAAATACTATCGTTCAAA
CTAATGACAGTAATCCTACCGCAAAATTTGTATCAGAATCAGGACAATCTGTAATAGGTCAAGTAAAA
CCAGATAATTCTGCGGCGCTTACAACAGTTGACACGCCTCATCATATTTCAGCTCCAGATGCTTTAAA
AACAACT CAATCAAGTCCTGTCGTTGAGAGTACTTCTACTAAGTTAACTGAAGAGACTTACAAACAAA
AAGATGG TCAAGATTTAGCCAACATGGTGAGAAGTGGTCAAGTTACTAGTGAGGAACTCGTTAATATG
GCATACGATATTATTGCTAAAGAAAACCCATCTTTAAATGCAGTCATTACTACTAGACGCCAAGAAGC
TATTGAAGAGGCTAGAAAACTTAAAGATACCAATCAGCCGTTTTTAGGTGTTCCCTTGTTAGTCAAGG
GGTTAGGGCACAGTATTAAAGGTGGTGAAACCAATAATGGCTTGATCTATGCAGATGGAAAAATTAGC
ACATTTGACAGTAGCTATGTCAAAAAATATAAAGATTTAGGATTTATTATTTTAGGACAAACGAACTT
TCCAGAGTATGGGTGGCGTAATATAACAGATTCTAAATTATACGGTCTAACGCATAATCCTTGGGATC
TTGCTCATAATGCTGGTGGCTCTTCTGGTGGAAGTGCAGCAGCCATTGCTAGCGGAATGACGCCAATT
GCTAGCGGTAGTGATGCTGGTGGTTCTATCCGTATTCCATCTTCTTGGACGGGCTTGGTAGGTTTAAA
ACCAACAAGAGGATTGGTGAGTAATGAAAAGCCAGATTCGTATAGTACAGCAGTTCATTTTCCATTAA
CTAAGTCATCTAGAGACGCAGAAACATTATTAACTTATCTAAAGAAAAGCGATCAAACGCTAGTATCA
GTTAATGATTTAAAATCTTTACCAATTGCTTATACTTTGAAATCACCAATGGGAACAGAAGTTAGTCA
AGATGCTAAAAACGCTATTATGGACAACGTCACATTCTTAAGAAAACAAGGATTCAAAGTAACAGAGA
TAGACTTACCAATTGATGGTAGAGCATTAATGCGTGATTATTCAACCTTGGCTATTGGCATGGGAGGA
GCTTTTTCAACAATTGAAAAAGACTTAAAAAAACATGGTTTTACTAAAGAAGACGTTGATCCTATTAC
TTGGGCAGTTCATGTTATTTATCAAAATTCAGATAAGGCTGAACTTAAGAAATCTATTATGGAAGCCC
AAAAACATATGGATGATTATCGTAAGGCAATGGAGAAGCTTCACAAGCAATTTCCTATTTTCTTATCG
CCAACGACCGCAAGTTTAGCCCCTCTAAATACAGATCCATATGTAACAGAGGAAGATAAAAGAGCGAT
TTATAATATGGAAAACTTGAGCCAAGAAGAAAGAATTGCTCTCTTTAATCGCCAGTGGGAGCCTATGT
TGCGTAGAACACCTTTTACACAAATTGCTAATATGACAGGACTCCCAGCTATCAGTATCCCGACTTAC
TTATCTGAGTCTGGTTTACCCATAGGGACGATGTTAATGGCAGGTGCAAACTATGATATGGTATTAAT
TAAATTGCAACTTTCTTTGAAAAACATCATGGTTTTAATGTTAAATGGCAAAGAATAATAGATAAAG
AAGTGAAACCATCTACTGGCCTAATACAGCCTACTAACTCCCTCTTTAAAGCTCATTCATCATTAGTA
AATTTAGAAGAAAATTCACAAGTTACTCAAGTATCTATCTCTAAAAAATGGATGAAATCGTCTGTTAA
AAATAAACCATCCGTAATGGCATATCAAAAAGCACTTCCTAAAACAGGTGATACAGAATCAAGCCTAT
CTCCAGTTTTAGTAGTAACCCTTTTATTAGCTTGTTTTAGCTTTGTAACAAAAAAGAATCAGAAAAGT
**SEQ ID NO. 74**

MKRKYFILNTVTVLTLAAAMNTSSIYANSTETSASVVPTTNTIVQTNDSNPTAKFVSESGQSVIGQVK
PDNSAALTTVDTPHHISAPDALKTTQSSPVVESTSTKLTEETYKQKDGQDLANMVRSGQVTSEELVNM
AYDIIAKENPSLNAVITTRRQEAIEEARKLKDTNQPFLGVPLLVKGLGHSIKGGETNNGLIYADGKIS
TFDSSYVKKYKDLGFIILGQTNFPEYGWRNITDSKLYGLTHNPWDLAHNAGGSSGGSAAAIASGMTPI
ASGSDAGGSIRIPSSWTGLVGLKPTRGLVSNEKPDSYSTAVHFPLTKSSRDAETLLTYLKKSDQTLVS
VNDLKSLPIAYTLKSPMGTEVSQDAKNAIMDNVTFLRKQGFKVTEIDLPIDGRALMRDYSTLAIGMGG
AFSTIEKDLKKHGFTKEDVDPITWAVHVIYQNSDKAELKKSIMEAQKHMDDYRKAMEKLHKQFPIFLS
PTTASLAPLNTDPYVTEEDKRAIYNMENLSQEERIALFNRQWEPMLRRTPFTQIANMTGLPAISIPTY
LSESGLPIGTMLMAGANYDMVLIKFATFFEKHHGFNVKWQRIIDKEVKPSTGLIQPTNSLFKAHSSLV
NLEENSQVTQVSISKKWMKSSVKNKPSVMAYQKALPKTGDTESSLSPVLVVTLLLACFSFVTKKNQKS

GBS 173 contains an N-terminal leader or signal sequence region which is indicated by the underlined sequences at the beginning of SEQ ID NO: 74 above. In one embodiment, one or more amino acids from the leader or signal sequence of GBS 173 are removed. An example of such a GBS 173 fragment is set forth below as SEQ ID NO: 75.
**SEQ ID NO: 75**

TTNTIVQTNDSNPTAKFVSESGQSVIGQVKPDNSAALTTVDTPHHISAPDALKTTQSSPVVESTSTKL
TEETYKQKDGQDLANMVRSGQVTSEELVNMAYDIIAKENPSLNAVITTRRQEAIEEARKLKDTNQPFL
GVPLLVKGLGHSIKGGETNNGLIYADGKISTFDSSYVKKYKDLGFIILGQTNFPEYGWRNITDSKLYG
LTHNPWDLAHNAGGSSGGSAAAIASGMTPIASGSDAGGSIRIPSSWTGLVGLKPTRGLVSNEKPDSYS
TAVHFPLTKSSRDAETLLTYLKKSDQTLVSVNDLKSLPIAYTLKSPMGTEVSQDAKNAIMDNVTFLRK
QGFKVTEIDLPIDGRALMRDYSTLAIGMGGAFSTIEKDLKKHGFTKEDVDPITWAVHVIYQNSDKAEL
KKSIMEAQKHMDDYRKAMEKLHKQFPIFLSPTTASLAPLNTDPYVTEEDKRAIYNMENLSQEERIALF
NRQWEPMLRRTPFTQIANMTGLPAISIPTYLSESGLPIGTMLMAGANYDMVLIKFATFFEKHHGFNVK
WQRIIDKEVKPSTGLIQPTNSLFKAHSSLVNLEENSQVTQVSISKKWMKSSVKNKPSVMAYQKALPKT
GDTESSLSPVLVVTLLLACFSFVTKKNQKS

GBS 173 may also contain a C-terminal transmembrane and/or cytoplasmic region which may be located within the underlined region near the end of SEQ ID NO: 74 above. In one embodiment, one or more amino acids from the transmembrane or cytoplasmic region of GBS 173 are removed. An example of such a GBS 173 fragment is set forth below as SEQ ID NO: 76.
**SEQ ID NO: 76**

MKRKYFILNTVTVLTLAAAMNTSSIYANSTETSASVVPTTNTIVQTNDSNPTAKFVSESGQSVIGQVK
PDNSAALTTVDTPHHISAPDALKTTQSSPVVESTSTKLTEETYKQKDGQDLANMVRSGQVTSEELVNM
AYDIIAKENPSLNAVITTRRQEAIEEARKLKDTNQPFLGVPLLVKGLGHSIKGGETNNGLIYADGKIS
TFDSSYVKKYKDLGFIILGQTNFPEYGWRNITDSKLYGLTHNPWDLAHNAGGSSGGSAAAIASGMTPI
ASGSDAGGSIRIPSSWTGLVGLKPTRGLVSNEKPDSYSTAVHFPLTKSSRDAETLLTYLKKSDQTLVS
VNDLKSLPIAYTLKSPMGTEVSQDAKNAIMDNVTFLRKQGFKVTEIDLPIDGRALMRDYSTLAIGMGG
AFSTIEKDLKKHGFTKEDVDPITWAVHVIYQNSDKAELKKSIMEAQKHMDDYRKAMEKLHKQFPIFLS
PTTASLAPLNTDPYVTEEDKRAIYNMENLSQEERIALFNRQWEPMLRRTPFTQIANMTGLPAISIPTY
LSESGLPIGTMLMAGANYDMVLIKFATFFEKHHGFNVKWQRIIDKEVKPSTGLIQPTNSLFKAHSSLV
NLEENSQVTQVSISKKWMKSSVKNK

In one embodiment, one or more amino acids from the leader or signal sequence region and one or more amino acids from the transmembrane or cytoplasmic region are removed. An example of such a GBS 173 fragment is set forth below as SEQ ID NO: 77.
**SEQ ID NO: 77**

TTNTIVQTNDSNPTAKFVSESGQSVIGQVKPDNSAALTTVDTPHHISAPDALKTTQSSPVVESTSTKL
TEETYKQKDGQDLANMVRSGQVTSEELVNMAYDIIAKENPSLNAVITTRRQEAIEEARKLKDTNQPFL
GVPLLVKGLGHSIKGGETNNGLIYADGKISTFDSSYVKKYKDLGFIILGQTNFPEYGWRNITDSKLYG
LTHNPWDLAHNAGGSSGGSAAAIASGMTPIASGSDAGGSIRIPSSWTGLVGLKPTRGLVSNEKPDSYS
TAVHFPLTKSSRDAETLLTYLKKSDQTLVSVNDLKSLPIAYTLKSPMGTEVSQDAKNAIMDNVTFLRK
QGFKVTEIDLPIDGRALMRDYSTLAIGMGGAFSTIEKDLKKHGFTKEDVDPITWAVHVIYQNSDKAEL
KKSIMEAQKHMDDYRKAMEKLHKQFPIFLSPTTASLAPLNTDPYVTEEDKRAIYNMENLSQEERIALF
NRQWEPMLRRTPFTQIANMTGLPAISIPTYLSESGLPIGTMLMAGANYDMVLIKFATFFEKHHGFNVK
WQRIIDKEVKPSTGLIQPTNSLFKAHSSLVNLEENSQVTQVSISKKWMKSSVKNK

### GBS 313

Nucleotide and amino acid sequences of GBS 313 sequenced from serotype V isolated strain 2603 V/R are set forth in Ref. 2 as SEQ ID 4089 and SEQ ID 4090. These sequences are set forth as SEQ ID NOS 78 and 79 below:
**SEQ ID NO. 78** 
ATGAAACGTATTGCTGTTTTAACTAGTGGTGGTGACGCCCCTGGTATGAACGCTGCTATCCGTGCAGT
TGTTCGTAAAGCAATTTCTGAAGGTATGGAAGTTTACGGCATCAACCAAGGTTACTATGGTATGGTGA
CAGGGGATATTTTCCCTTTGGATGCTAATTCTGTTGGGGATACTATCAACCGTGGAGGAACGTTTTTA
CGTTCAGCACGTTATCCTGAATTTGCTGAACTTGAAGGTCAGCTTAAAGGGATTGAACAGCTTAAAAA
ACACGGTATTGAAGGTGTAGTAGTTATCGGTGGTGATGGTTCTTATCATGGTGCTATGCGTCTAACTG
AGCACGGTTTCCCAGCTGTTGGTTTGCCGGGTACAATTGATAACGATATCGTTGGCACTGACTATACT
ATTGGTTTTGACACAGCAGTTGCGACAGCAGTTGAGAATCTTGACCGTCTTCGTGATACATCAGCAAG
TCATAACCGTACTTTTGTTGTTGAGGTTATGGGAAGAAATGCAGGAGATATCGCTCTTTGGTCAGGTA
TCGCTGCAGGTGCAGATCAAATTATTGTTCCTGAAGAAGAGTTCAATATTGATGAAGTTGTCTCAAAT
GTTAGAGCTGGCTATGCAGCTGGTAAACATCACCAAATCATCGTCCTTGCAGAAGGTGTTATGAGTGG
TGATGAGTTTGCAAAAACAATGAAAGCAGCAGGAGACGATAGCGATCTTCGTGTGACGAATTTAGGAC
ATCTGCTCCGTGGTGGTAGTCCGACGGCTCGTGATCGTGTCTTAGCATCTCGTATGGGAGCGTACGCT
GTTCAATTGTTGAAAGAAGGTCGTGGTGGTTTAGCCGTTGGTGTCCACAACGAAGAAATGGTTGAAAG
TCCAATTTTAGGTTTAGCAGAAGAAGGTGCTTTGTTCAGCTTGACTGATGAAGGAAAAATCGTTGTTA
ATAATCCGCATAAAGCGGACCTTCGCTTGGCAGCACTTAATCGTGACCTTGCCAACCAAAGTAGTAAA
**SEQ ID NO. 79**

MKRIAVLTSGGDAPGMNAAIRAVVRKAISEGMEVYGINQGYYGMVTGDIFPLDANSVGDTINRGGTFL
RSARYPE FAELEGQLKGIEQLKKHGIEGVVVIGGDGSYHGAMRLTEHGFPAVGLPGTIDNDIVGTDYT
IGFDTAVATAVENLDRLRDTSASHNRTFVVEVMGRNAGDIALWSGIAAGADQIIVPEEEFNIDEVVSN
VRAGYAAGKHHQIIVLAEGVMSGDEFAKTMKAAGDDSDLRVTNLGHLLRGGSPTARDRVLASRMGAYA
VQLLKEGRGGLAVGVHNEEMVESPILGLAEEGALFSLTDEGKIVVNNPHKADLRLAALNRDLANQSSK

### GBS 328

GBS 328 belongs to the 5'-nucleotidase family. Nucleotide and amino acid sequences of GBS 328 sequenced from serotype V isolated strain 2603 V/R are set forth in Ref. 2 as SEQ ID 6015 and SEQ ID 6016. These sequences are set forth below as SEQ ID NOS 80 and 81:
**SEQ ID NO. 80** 
ATGAAAAAGAAAATTATTTTGAAAAGTAGTGTTCTTGGTTTAGTCGCTGGGACTTCTATTATGTTCTC
AAGCGTGTTCGCGGACCAAGTCGGTGTCCAAGTTATAGGCGTCAATGACTTTCATGGTGCACTTGACA
ATACTGGAACAGCAAATATGCCTGATGGAAAAGTTGCTAATGCTGGTACTGCTGCTCAATTAGATGCT
TATATGGATGACGCTCAAAAAGATTTCAAACAAACTAACCCTAATGGTGAAAGCATTAGGGTTCAAGC
AGGCGATATGGTTGGAGCAAGTCCAGCCAACTCTGGGCTTCTTCAAGATGAACCAACTGTCAAAAATT
TTAATGCAATGAATGTTGAGTATGGCACATTGGGTAACCATGAATTTGATGAAGGGTTGGCAGAATAT
AATCGTATCGTTACTGGTAAAGCCCCTGCTCCAGATTCTAATATTAATAATATTACGAAATCATACCC
ACATGAAGCTGCAAAACAAGAAATTGTAGTGGCAAATGTTATTGATAAAGTTAACAAACAAATTCCTT
ACAATTGGAAGCCTTACGCTATTAAAAATATTCCTGTAAATAACAAAAGTGTGAACGTTGGCTTTATC
GGGATTGTCACCAAAGACATCCCAAACCTTGTCTTACGTAAAAATTATGAACAATATGAATTTTTAGA
TGAAGCTGAAACAATCGTTAAATACGCCAAAGAATTACAAGCTAAAAATGTCAAAGCTATTGTAGTTC
TCGCACATGTACCTGCAACAAGTAAAAATGATATTGCTGAAGGTGAAGCAGCAGAAATGATGAAAAAA
GTCAATCAACTCTTCCCTGAAAATAGCGTAGATATTGTCTTTGCTGGACACAATCATCAATATACAAA
TGGTCTTGTTGGTAAAACTCGTATTGTACAAGCGCTCTCTCAAGGAAAAGCCTATGCTGATGTACGTG
GTGTCTTAGATACTGATACACAAGATTTCATTGAGACCCCTTCAGCTAAAGTAATTGCAGTTGCTCCT
GGTAAAAAAACAGGTAGTGCCGATATTCAAGCCATTGTTGACCAAGCTAATACTATCGTTAAACAAGT
AACAGAAGCTAAAATTGGTACTGCCGAGGTAAGTGTCATGATTACGCGTTCTGTTGATCAAGATAATG
TTAGTCCGGTAGGCAGCCTCATCACAGAGGCTCAACTAGCAATTGCTCGAAAAAGCTGGCCAGATATC
GATTTTGCCATGACAAATAATGGTGGCATTCGTGCTGACTTACTCATCAAACCAGATGGAACAATCAC
CTGGGGAGCTGCACAAGCAGTTCAACCTTTTGGTAATATCTTACAAGTCGTCGAAATTACTGGTAGAG
ATCTTTATAAAGCACTCAACGAACAATACGACCAAAAACAAAATTTCTTCCTTCAAATAGCTGGTCTG
CGATACACTTACACAGATAATAAAGAGGGCGGGGAAGAAACACCATTTAAAGTTGTAAAAGCTTATAA
ATCAAATGGTGAGGAAATCAATCCTGATGCAAAATACAAATTAGTTATCAATGACTTTTTATTCGGTG
GTGGTGATGGCTTTGCAAGCTTCAGAAATGCCAAACTTCTAGGAGCCATTAACCCCGATACAGAGGTA
TTTATGGCCTATATCACTGATTTAGAAAAAGCTGGTAAAAAAGTGAGCGTTCCAAATAATAAACCTAA
AATCTATGTCACTATGAAGATGGTTAATGAAACTATTACACAAAATGATGGTACACATAGCATTATTA
AGAAACTTTATTTAGATCGACAAGGAAATATTGTAGCACAAGAGATTGTATCAGACACTTTAAACCAA
ACAAAATCAAAATCTACAAAAATCAACCCTGTAACTACAATTCACAAAAAACAATTACACCAATTTAC
AGCTATTAACCCTATGAGAAATTATGGCAAACCATCAAACTCCACTACTGTAAAATCAAAACAATTAC
CAAAAACAAACTCTGAATATGGACAATCATTCCTTATGTCTGTCTTTGGTGTTGGACTTATAGGAATT
GCTTTAAATACAAAGAAAAAACATATGAAA
**SEQ ID NO. 81**

MKKKIILKSSVLGLVAGTSIMFSSVFADQVGVQVIGVNDFHGALDNTGTANMPDGKVANAGTAAQLDA
YMDDAQKDFKQTNPNGESIRVQAGDMVGASPANSGLLQDEPTVKNFNAMNVEYGTLGNHEFDEGLAEY
NRIVTGKAPAPDSNINNITKSYPHEAAKQEIVVANVIDKVNKQIPYNWKPYAIKNIPVNNKSVNVGFI
GIVTKDIPNLVLRKNYEQYEFLDEAETIVKYAKELQAKNVKAIVVLAHVPATSKNDIAEGEAAEMMKK
VNQLFPENSVDIVFAGHNHQYTNGLVGKTRIVQALSQGKAYADVRGVLDTDTQDFIETPSAKVIAVAP
GKKTGSADIQAIVDQANTIVKQVTEAKIGTAEVSVMITRSVDQDNVSPVGSLITEAQLAIARKSWPDI
DFAMTNNGGIRADLLIKPDGTITWGAAQAVQPFGNILQVVEITGRDLYKALNEQYDQKQNFFLQIAGL
RYTYTDNKEGGEETPFKVVKAYKSNGEEINPDAKYKLVINDFLFGGGDGFASFRNAKLLGAINPDTEV
FMAYITDLEKAGKKVSVPNNKPKIYVTMKMVNETITQNDGTHSIIKKLYLDRQGNIVAQEIVSDTLNQ
TKSKSTKINPVTTIHKKQLHQFTAINPMRNYGKPSNSTTVKSKQLPKTNSEYGQSFLMSVFGVGLIGI
ALNTKKKHMK

GBS 328 may contain an N-terminal leader or signal sequence region which is indicated by the underlined sequence at the beginning of SEQ ID NO: 81 above. In one embodiment, one or more amino acids from the leader or signal sequence region of GBS 328 are removed. An example of such a GBS 328 fragment is set forth below as SEQ ID NO: 82.
**SEQ ID NO: 82**

HGALDNTGTANMPDGKVANAGTAAQLDAYMDDAQKDFKQTNPNGESIRVQAGDMVGASPANSGLLQDE
PTVKNFNAMNVEYGTLGNHEFDEGLAEYNRIVTGKAPAPDSNINNITKSYPHEAAKQEIVVANVIDKV
NKQIPYNWKPYAIKNIPVNNKSVNVGFIGIVTKDIPNLVLRKNYEQYEFLDEAETIVKYAKELQAKNV
KAIVVLAHVPATSKNDIAEGEAAEMMKKVNQLFPENSVDIVFAGHNHQYTNGLVGKTRIVQALSQGKA
YADVRGVLDTDTQDFIETPSAKVIAVAPGKKTGSADIQAIVDQANTIVKQVTEAKIGTAEVSVMITRS
VDQDNVSPVGSLITEAQLAIARKSWPDIDFAMTNNGGIRADLLIKPDGTITWGAAQAVQPFGNILQVV
EITGRDLYKALNEQYDQKQNFFLQIAGLRYTYTDNKEGGEETPFKVVKAYKSNGEEINPDAKYKLVIN
DFLFGGGDGFASFRNAKLLGAINPDTEVFMAYITDLEKAGKKVSVPNNKPKIYVTMKMVNETITQNDG
THSIIKKLYLDRQGNIVAQEIVSDTLNQTKSKSTKINPVTTIHKKQLHQFTAINPMRNYGKPSNSTTV
KSKQLPKTNSEYGQSFLMSVFGVGLIGIALNTKKKHMK

GBS 328 may also contain a transmembrane and/or cytoplasmic domain region. In one embodiment, one or more amino acids from the transmembrane and/or cytoplasmic domain region of GBS 328 are removed. An example of such a GBS 328 fragment is set forth below as SEQ ID NO: 83.
**SEQ ID NO: 83**

MKKKIILKSSVLGLVAGTSIMFSSVFADQVGVQVIGVNDFHGALDNTGTANMPDGKVANAGTAAQLDA
YMDDAQKDFKQTNPNGESIRVQAGDMVGASPANSGLLQDEPTVKNFNAMNVEYGTLGNHEFDEGLAEY
NRIVTGKAPAPDSNINNITKSYPHEAAKQEIVVANVIDKVNKQIPYNWKPYAIKNIPVNNKSVNVGFI
GIVTKDIPNLVLRKNYEQYEFLDEAETIVKYAKELQAKNVKAIVVLAHVPATSKNDIAEGEAAEMMKK
VNQLFPENSVDIVFAGHNHQYTNGLVGKTRIVQALSQGKAYADVRGVLDTDTQDFIETPSAKVIAVAP
GKKTGSADIQAIVDQANTIVKQVTEAKIGTAEVSVMITRSVDQDNVSPVGSLITEAQLAIARKSWPDI
DFAMTNNGGIRADLLIKPDGTITWGAAQAVQPFGNILQVVEITGRDLYKALNEQYDQKQNFFLQIAGL
RYTYTDNKEGGEETPFKVVKAYKSNGEEINPDAKYKLVINDFLFGGGDGFASFRNAKLLGAINPDTEV
FMAYITDLEKAGKKVSVPNNKPKIYVTMKMVNETITQNDGTHSIIKKLYLDRQGNIVAQEIVSDTLNQ
TKSKSTKINPVTTIHKKQLHQFTAINPMRNYGKPSNSTTVKS

In one embodiment, one or more amino acids from the leader or signal sequence region and one or more amino acids from the transmembrane or cytoplasmic region of GBS 328 are removed. An example of such a GBS 328 fragment is set forth below as SEQ ID NO: 84.
**SEQ ID NO: 84**

HGAL DNTGTANMPDGKVANAGTAAQLDAYMDDAQKDFKQTNPNGESIRVQAGDMVGASPANSGLLQDE
PTVKNFNAMNVEYGTLGNHEFDEGLAEYNRIVTGKAPAPDSNINNITKSYPHEAAKQEIVVANVIDKV
NKQI PYNWKPYAIKNIPVNNKSVNVGFIGIVTKDIPNLVLRKNYEQYEFLDEAETIVKYAKELQAKNV
KAIVVLAHVPATSKNDIAEGEAAEMMKKVNQLFPENSVDIVFAGHNHQYTNGLVGKTRIVQALSQGKA
YADVRGVLDTDTQDFIETPSAKVIAVAPGKKTGSADIQAIVDQANTIVKQVTEAKIGTAEVSVMITRS
VDQDNVSPVGSLITEAQLAIARKSWPDIDFAMTNNGGIRADLLIKPDGTITWGAAQAVQPFGNILQVV

EITGRDLYKALNEQYDQKQNFFLQIAGLRYTYTDNKEGGEETPFKVVKAYKSNGEEINPDAKYKLVIN
DFLFGGGDGFASFRNAKLLGAINPDTEVFMAYITDLEKAGKKVSVPNNKPKIYVTMKMVNETITQNDG
THSIIKKLYLDRQGNIVAQEIVSDTLNQTKSKSTKINPVTTIHKKQLHQFTAINPMRNYGKPSNSTTV
KS

### GB S 656

GBS 656 refers to a putative DNA-entry nuclease. Nucleotide and amino acid sequences of GBS 656 sequenced from serotype V isolated strain 2603 V/R are set forth in Ref. 2 as SEQ ID 9323 and SEQ ID 9324. These sequences are set forth below as SEQ ID NOS 85 and 86:
**SEQ ID NO. 85** 
ATGAAAAGATTACATAAACTGTTTATAACCGTAATTGCTACATTAGGTATGTTGGGGGTAATGACCTT
TGGTCTTCCAACGCAGCCGCAAAACGTAACGCCGATAGTACATGCTGATGTCAATTCATCTGTTGATA
CGAGCCAGGAATTTCAAAATAATTTAAAAAATGCTATTGGTAACCTACCATTTCAATATGTTAATGGT
ATTTATGAATTAAATAATAATCAGACAAATTTAAATGCTGATGTCAATGTTAAAGCGTATGTTCAAAA
TACAATTGACAATCAACAAAGACTATCAACTGCTAATGCAATGCTTGATAGAACCATTCGTCAATATC
AAAATCGCAGAGATACCACTCTTCCCGATGCAAATTGGAAACCATTAGGTTGGCATCAAGTAGCTACT
AATGACCATTATGGACATGCAGTCGACAAGGGGCATTTAATTGCCTATGCTTTAGCTGGAAATTTCAA
AGGTTGGGATGCTTCCGTGTCAAATCCTCAAAATGTTGTCACACAAACAGCTCATTCCAACCAATCAA
ATCAAAAAATCAATCGTGGACAAAATTATTATGAAAGCTTAGTTCGTAAGGCGGTTGACCAAAACAAA
CGTGTTCGTTACCGTGTAACTCCATTGTACCGTAATGATACTGATTTAGTTCCATTTGCAATGCACCT
AGAAGCTAAATCACAAGATGGCACATTAGAATTTAATGTTGCTATTCCAAACACACAAGCATCATACA
CTATGGATTATGCAACAGGAGAAATAACACTAAAT
**SEQ ID NO.86**

MKRLHKLFITVIATLGMLGVMTFGLPTQPQNVTPIVHADVNSSVDTSQEFQNNLKNAIGNLPFQYVNG
IYELNNNQTNLNADVNVKAYVQNTIDNQQRLSTANAMLDRTIRQYQNRRDTTLPDANWKPLGWHQVAT
NDHYGHAVDKGHLIAYALAGNFKGWDASVSNPQNVVTQTAHSNQSNQKINRGQNYYESLVRKAVDQNK
RVRYRVTPLYRNDTDLVPFAMHLEAKSQDGTLEFNVAIPNTQASYTMDYATGEITLN

### GBS 67

The following offers examples of preferred GBS 67 fragments. Nucleotide and amino acid sequence of GBS 67 sequences from serotypeV isolated strain 2603 are set forth below as SEQ ID NOS: 87 and 88.
**SEQ ID NO: 87** 
ATGAGAAAATACCAAAAATTTTCTAAAATATTGACGTTAAGTCTTTTTTGTTTGTCGCAAATACCGCT
TAATACCAATGTTTTAGGGGAAAGTACCGTACCGGAAAATGGTGCTAAAGGAAAGTTAGTTGTTAAAA
AGACAGATGACCAGAACAAACCACTTTCAAAAGCTACCTTTGTTTTAAAAACTACTGCTCATCCAGAA
AGTAAAATAGAAAAAGTAACTGCTGAGCTAACAGGTGAAGCTACTTTTGATAATCTCATACCTGGAGA
TTATACTTTATCAGAAGAAACAGCGCCCGAAGGTTATAAAAAGACTAACCAGACTTGGCAAGTTAAGG
TTGAGAGTAATGGAAAAACTACGATACAAAATAGTGGTGATAAAAATTCCACAATTGGACAAAATCAG
GAAGAACTAGATAAGCAGTATCCCCCACAGGAATTTATGAAGATACAAAGGAATCTTATAAACTTGA
GCATGTTAAAGGTTCAGTTCCAAATGGAAAGTCAGAGGCAAAAGCAGTTAACCCATATTCAAGTGAAG
GTGAGCATATAAGAGAAATTCCAGAGGGAACATTATCTAAACGTATTTCAGAAGTAGGTGATTTAGCT
CATAATAAATATAAAATTGAGTTAACTGTCAGTGGAAAAACCATAGTAAAACCAGTGGACAAACAAAA
GCCGTTAGATGTTGTCTTCGTACTCGATAATTCTAACTCAATGAATAACGATGGCCCAAATTTTCAAA
GGCATAATAAAGCCAAGAAAGCTGCCGAAGCTCTTGGGACCGCAGTAAAAGATATTTTAGGAGCAAAC
 
AGTGATAATAGGGTTGCATTAGTTACCTATGGTTCAGATATTTTTGATGGTAGGAGTGTAGATGTCGT
AAAAGGATTTAAAGAAGATGATAAATATTATGGCCTTCAAACTAAGTTCACAATTCAGACAGAGAATT
ATAGTCATAAACAATTAACAAATAATGCTGAAGAGATTATAAAAAGGATTCCGACAGAAGCTCCTAAA
GCTAAGTGGGGATCTACTACCAATGGATTAACTCCAGAGCAACAAAAGGAGTACTATCTTAGTAAAGT
AGGAGAAACATTTACTATGAAAGCCTTCATGGAGGCAGATGATATTTTGAGTCAAGTAAATCGAAATA
GTCAAAAAATTATTGTTCATGTAACTGATGGTGTTCCTACGAGATCATATGCTATTAATAATTTTAAA
CTGGGTGCATCATATGAAAGCCAATTTGAACAAATGAAAAAAAATGGATATCTAAATAAAAGTAATTT
TCTACTTACTGATAAGCCCGAGGATATAAAAGGAAATGGGGAGAGTTACTTTTTGTTTCCCTTAGATA
GTTATCAAACACAGATAATCTCTGGAAACTTACAAAAACTTCATTATTTAGATTTAAATCTTAATTAC
CCTAAAGGTACAATTTATCGAAATGGACCAGTGAAAGAACATGGAACACCAACCAAACTTTATATAAA
TAGTTTAAAACAGAAAAATTATGACATTTTTAATTTTGGTATCGATATATCTGGTTTTAGACAAGTTT
ATAATGAGGAGTATAAGAAAAATCAAGATGGTACTTTTCAAAAATTGAAAGAGGAAGCTTTTAAACTT
TCAGATGGAGAAATCACAGAACTAATGAGGTCGTTCTCTTCCAAACCTGAGTACTACACCCCTATCGT
AACTTCAGCCGATACATCTAACAATGAAATTTTATCTAAAATTCAGCAACAATTTGAAACGATTTTAA
CAAAAGAAAACTCAATTGTTAATGGAACTATCGAAGATCCTATGGGTGATAAAATCAATTTACAGCTT
GGTAATGGACAAACATTACAGCCAAGTGATTATACTTTACAGGGAAATGATGGAAGTGTAATGAAGGA
TGGTATTGCAACTGGTGGGCCTAATAATGATGGTGGAATACTTAAGGGGGTTAAATTAGAATACATCG
GAAATAAACTCTATGTTAGAGGTTTGAATTTAGGAGAAGGTCAAAAAGTAACACTCACATATGATGTG
AAACTAGATGACAGTTTTATAAGTAACAAATTCTATGACACTAATGGTAGAACAACATTGAATCCTAA
GTCAGAGGATCCTAATACACTTAGAGATTTTCCAATCCCTAAAATTCGTGATGTGAGAGAATATCCTA
CAATAACGATTAAAAACGAGAAGAAGTTAGGTGAAATTGAATTTATAAAAGTTGATAAAGATAATAAT
AAGTTGCTTCTCAAAGGAGCTACGTTTGAACTTCAAGAATTTAATGAAGATTATAAACTTTATTTACC
AATAAAAAATAATAATTCAAAAGTAGTGACGGGAGAAAACGGCAAAATTTCTTACAAAGATTTGAAAG
ATGGCAAATATCAGTTAATAGAAGCAGTTTCGCCGGAGGATTATCAAAAAATTACTAATAAACCAATT
TTAACTTTTGAAGTGGTTAAAGGATCGATAAAAAATATAATAGCTGTTAATAAACAGATTTCTGAATA
TCATGAGGAAGGTGACAAGCATTTAATTACCAACACGCATATTCCACCAAAAGGAATTATTCCTATGA
CAGGTGGGAAAGGAATTCTATCTTTCATTTTAATAGGTGGAGCTATGATGTCTATTGCAGGTGGAATT
TATATTTGGAAAAGGTATAAGAAATCTAGTGATATGTCCATCAAAAAAGAT
**SEQ ID NO: 88**

MRKYQKFSKILTLSLFCLSQIPLNTNVLGESTVPENGAKGKLVVKKTDDQNKPLSKATFVLKTTAHPE
SKIEKVTAELTGEATFDNLIPGDYTLSEETAPEGYKKTNQTWQVKVESNGKTTIQNSGDKNSTIGQNQ
EELDKQYPPTGIYEDTKESYKLEHVKGSVPNGKSEAKAVNPYSSEGEHIREIPEGTLSKRISEVGDLA
HNKYKIELTVSGKTIVKPVDKQKPLDVVFVLDNSNSMNNDGPNFQRHNKAKKAAEALGTAVKDILGAN
SDNRVALVTYGSDIFDGRSVDVVKGFKEDDKYYGLQTKFTIQTENYSHKQLTNNAEEIIKRIPTEAPK
AKWGSTTNGLTPEQQKEYYLSKVGETFTMKAFMEADDILSQVNRNSQKIIVHVTDGVPTRSYAINNFK
LGASYESQFEQMKKNGYLNKSNFLLTDKPEDIKGNGESYFLFPLDSYQTQIISGNLQKLHYLDLNLNY
PKGTIYRNGPVKEHGTPTKLYINSLKQKNYDIFNFGIDISGFRQVYNEEYKKNQDGTFQKLKEEAFKL
SDGEITELMRSFSSKPEYYTPIVTSADTSNNEILSKIQQQFETILTKENSIVNGTIEDPMGDKINLQL
GNGQTLQPSDYTLQGNDGSVMKDGIATGGPNNDGGILKGVKLEYIGNKLYVRGLNLGEGQKVTLTYDV
KLDDSFISNKFYDTNGRTTLNPKSEDPNTLRDFPIPKIRDVREYPTITIKNEKKLGEIEFIKVDKDNN
KLLLKGATFELQEFNEDYKLYLPIKNNNSKVVTGENGKISYKDLKDGKYQLIEAVSPEDYQKITNKPI
LTFEVVKGSIKNIIAVNKQISEYHEEGDKHLITNTHIPPKGIIPMTGGKGILSFILIGGAMMSIAGGI
YIWKRYKKSSDMSIKKD

GBS 67 contains a C-terminus transmembrane region which is indicated by the underlined region closest to the C-terminus of SEQ ID NO: 88 above. In one embodiment; one or more amino acids from the transmembrane region is removed and or the amino acid is truncated before the transmembrane region. An example of such a GBS 67 fragment is set forth below as SEQ ID NO: 89.
**SEQ ID NO: 89**

MRKYQKFSKILTLSLFCLSQIPLNTNVLGESTVPENGAKGKLVVKKTDDQNKPLSKATFVLKTTAHPE
SKIEKVTAELTGEATFDNLIPGDYTLSEETAPEGYKKTNQTWQVKVESNGKTTIQNSGDKNSTIGQNQ
EELDKQYPPTGIYEDTKESYKLEHVKGSVPNGKSEAKAVNPYSSEGEHIREIPEGTLSKRISEVGDLA
HNKYKIELTVSGKTIVKPVDKQKPLDVVFVLDNSNSMNNDGPNFQRHNKAKKAAEALGTAVKDILGAN
SDNRVALVTYGSDIFDGRSVDVVKGFKEDDKYYGLQTKFTIQTENYSHKQLTNNAEEIIKRIPTEAPK
AKWGSTTNGLTPEQQKEYYLSKVGETFTMKAFMEADDILSQVNRNSQKIIVHVTDGVPTRSYAINNFK
LGASYESQFEQMKKNGYLNKSNFLLTDKPEDIKGNGESYFLFPLDSYQTQIISGNLQKLHYLDLNLNY
PKGTIYRNGPVKEHGTPTKLYINSLKQKNYDIFNFGIDISGFRQVYNEEYKKNQDGTFQKLKEEAFKL
SDGEITELMRSFSSKPEYYTPIVTSADTSNNEILSKIQQQFETILTKENSIVNGTIEDPMGDKINLQL
GNGQTLQPSDYTLQGNDGSVMKDGIATGGPNNDGGILKGVKLEYIGNKLYVRGLNLGEGQKVTLTYDV
KLDDSFISNKFYDTNGRTTLNPKSEDPNTLRDFPIPKIRDVREYPTITIKNEKKLGEIEFIKVDKDNN
KLLLKGATFELQEFNEDYKLYLPIKNNNSKVVTGENGKISYKDLKDGKYQLIEAVSPEDYQKITNKPI
LTFEVVKGSIKNIIAVNKQISEYHEEGDKHLITNTHIPPKGIIPMTGGKGILS

GBS 67 contains an amino acid motif indicative of a cell wall anchor (an LPXTG motif):
**SEQ ID NO: 90** IPMTG. (shown in italics in SEQ ID NO: 88 above). In some recombinant host cell systems, it may be preferable to remove this motif to facilitate secretion of a recombinant GBS 67 protein from the host cell. Accordingly, in one preferred fragment of GBS 67 for use in the invention, the transmembrane and the cell wall anchor motif are removed from GBS 67. An example of such a GBS 67 fragment is set forth below as SEQ ID NO: 91.
**SEQ ID NO: 91**

MRKYQKFSKILTLSLFCLSQIPLNTNVLGESTVPENGAKGKLVVKKTDDQNKPLSKATFVLKTTAHPE
SKIEKVTAELTGEATFDNLIPGDYTLSEETAPEGYKKTNQTWQVKVESNGKTTIQNSGDKNSTIGQNQ
EELDKQYPPTGIYEDTKESYKLEHVKGSVPNGKSEAKAVNPYSSEGEHIREIPEGTLSKRISEVGDLA
HNKYKIELTVSGKTIVKPVDKQKPLDVVFVLDNSNSMNNDGPNFQRHNKAKKAAEALGTAVKDILGAN
SDNRVALVTYGSDIFDGRSVDVVKGFKEDDKYYGLQTKFTIQTENYSHKQLTNNAEEIIKRIPTEAPK
AKWGSTTNGLTPEQQKEYYLSKVGETFTMKAFMEADDILSQVNRNSQKIIVHVTDGVPTRSYAINNFK
LGASYESQFEQMKKNGYLNKSNFLLTDKPEDIKGNGESYFLFPLDSYQTQIISGNLQKLHYLDLNLNY
PKGTIYRNGPVKEHGTPTKLYINSLKQKNYDIFNFGIDISGFRQVYNEEYKKNQDGTFQKLKEEAFKL
SDGEITELMRSFSSKPEYYTPIVTSADTSNNEILSKIQQQFETILTKENSIVNGTIEDPMGDKINLQL
GNGQTLQPSDYTLQGNDGSVMKDGIATGGPNNDGGILKGVKLEYIGNKLYVRGLNLGEGQKVTLTYDV
KLDDSFISNKFYDTNGRTTLNPKSEDPNTLRDFPIPKIRDVREYPTITIKNEKKLGEIEFIKVDKDNN
KLLLKGATFELQEFNEDYKLYLPIKNNNSKVVTGENGKISYKDLKDGKYQLIEAVSPEDYQKITNKPI
LTFEVVKGSIKNIIAVNKQISEYHEEGDKHLITNTHIPPKGI

The compositions of the invention may also include combinations including one or more known GBS antigens in combination with GBS 80.

There is an upper limit to the number of GBS antigens which will be in the compositions of the invention. Preferably, the number of GBS antigens in a composition of the invention is less than 20, less than 19, less than 18, less than 17, less than 16, less than 15, less than 14, less than 13, less than 12, less than 11, less than 10, less than 9, less than 8, less than 7, less than 6, less than 5, less than 4, or less than 3. Still more preferably, the number of GBS antigens in a composition of the invention is less than 6, less than 5, or less than 4. Still more preferably, the number of GBS antigens in a composition of the invention is 3.

The GBS antigens used in the invention are preferably isolated, i.e., separate and discrete, from the whole organism with which the molecule is found in nature or, when the polynucleotide or polypeptide is not found in nature, is sufficiently free of other biological macromolecules so that the polynucleotide or polypeptide can be used for its intended purpose.

### Fusion Proteins

The GBS antigens used in the invention may be present in the composition as individual separate polypeptides, but it is preferred that at least two (*i.e.* 2, 3, 4, 5, 6, 7, 8, 9, 10, 11, 12, 13, 14, 15, 16, 17, or 18) of the antigens are expressed as a single polypeptide chain (a "hybrid" or "fusion" polypeptide). Such fusion polypeptides offer two principal advantages: first, a polypeptide that may be unstable or poorly expressed on its own can be assisted by adding a suitable fusion partner that overcomes the problem; second, commercial manufacture is simplified as only one expression and purification need be employed in order to produce two polypeptides which are both antigenically useful.

The fusion polypeptide may comprise two or more polypeptide sequences from the group consisting of GBS 80, GBS 91, GBS 104, GBS 184, GBS 276, GBS 305, GBS 322, GBS 330, GBS 338, GBS 361, GBS 404, GBS 690 and GBS 691. Preferably, the polypeptide sequences are selected from the group consisting of GBS 80, GBS 104 and GBS 322. Most preferably, the fusion peptide includes a polypeptide sequence from GBS 80. Accordingly, the invention includes a fusion peptide comprising a first amino acid sequence and a second amino acid sequence, wherein said first and second amino acid sequences are selected from a GBS antigen or a fragment thereof of the above antigen group. Preferably, the first and second amino acid sequences in the fusion polypeptide comprise different epitopes.

### EXAMPLE 7: Examples of fragments for fusion proteins from GBS 80 with GBS 104, and GBS 322

Examples of GBS fragments for fusion proteins are provided from GBS 322, GBS 104, and GBS 80. One example of a fragment of GBS 322 in a fusion protein is a 407 amino acid fragment with the signal peptide removed. Fragments of GBS 104 may also be incorporated in fusion proteins. An example of GBS 104 fragments includes an 830 amino acid fragment, a 359 amino acid fragment from near the N-terminus, a 581 amino acid fragment from near the N-terminus, and a 740 amino acid fragment from near the N-terminus. Examples of GBS 80 fragments include a 446 amino acid fragment and a 235 amino acid fragment. Table 13 below summarizes the examples of fragments for fusion proteins and their locations within the corresponding full length GBS protein.

**Table 13: Active Maternal Immunization Assay using combinations of GBS 80 with GBS 104 and GBS 322**

| **GBS** | **Size (AA)** | **SEQ ID NO** | **From ... to** |
|---|---|---|---|
| 322 | **407** | **92** | **25-432** |
| 104 | **830** | **96** | **28-858** |
| 104 N1 | **359** | **97** | **28-387** |
| 104 N2 | **581** | **98** | **28-609** |
| 104 N3 | **740** | **99** | **28-768** |
| 80 | **446** | **100** | **37-483** |
| 80N | **235** | **101** | **37-272** |

Hybrids (or fusions) consisting of amino acid sequences from two, three, four, five, six, seven, eight, nine, or ten GBS antigens are preferred. In particular, hybrids consisting of amino acid sequences from two, three, four, or five GBS antigens are preferred.

Different hybrid polypeptides may be mixed together in a single formulation. Within such combinations, a GBS antigen may be present in more than one hybrid polypeptide and/or as a non-hybrid polypeptide. It is preferred, however, that an antigen is present either as a hybrid or as a non-hybrid, but not as both.

Hybrid polypeptides can be represented by the formula **NH₂-A-{-X-L-}*ₙ*-B-COOH**, wherein: X is an amino acid sequence of a GBS antigen or a fragment thereof from the antigen group set forth above; L is an optional linker amino acid sequence; A is an optional N-terminal amino acid sequence; B is an optional C-terminal amino acid sequence; and *n* is 2, 3, 4, 5, 6, 7, 8, 9, 10, 11, 12, 13, 14 or 15.

If a -X- moiety has a leader peptide sequence in its wild-type form, this may be included or omitted in the hybrid protein. In some embodiments, the leader peptides will be deleted except for that of the -X- moiety located at the N-terminus of the hybrid protein *i.e.* the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker amino acid sequence -L- may be present or absent. For instance, when *n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc*. Linker amino acid sequence(s) -L- will typically be short (*e.g.* 20 or fewer amino acids *i.e*. 19, 18, 17, 16, 15, 14, 13, 12, 11, 10, 9, 8, 7, 6, 5, 4, 3, 2, 1). Examples comprise short peptide sequences which facilitate cloning, poly-glycine linkers (*i.e.* comprising Gly*ₙ* where *n* = 2, 3, 4, 5, 6, 7, 8, 9, 10 or more), and histidine tags (*i.e.* His*ₙ* where *n* = 3, 4, 5, 6, 7, 8, 9, 10 or more). Other suitable linker amino acid sequences will be apparent to those skilled in the art. A useful linker is GSGGGG, with the Gly-Ser dipeptide being formed from a *Bam*HI restriction site, thus aiding cloning and manipulation, and the (Gly)₄ tetrapeptide being a typical poly-glycine linker.

-A- is an optional N-terminal amino acid sequence. This will typically be short (*e.g*. 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g*. histidine tags *i.e.* His*ₙ* where *n* = 3, 4, 5, 6, 7, 8, 9, 10 or more). Other suitable N-terminal amino acid sequences will be apparent to those skilled in the art. If X₁ lacks its own N-terminus methionine, -A-is preferably an oligopeptide (*e.g*. with 1, 2, 3, 4, 5, 6, 7 or 8 amino acids) which provides a N-terminus methionine.

-B- is an optional C-terminal amino acid sequence. This will typically be short (*e.g*. 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g*. comprising histidine tags *i.e.* His*ₙ* where *n* = 3, 4, 5, 6, 7, 8, 9, 10 or more), or sequences which enhance protein stability. Other suitable C-terminal amino acid sequences will be apparent to those skilled in the art. Most preferably, *n* is 2 or 3.

### EXAMPLE 8: Active Maternal Immunization Assay using fusion proteins of Fragments of GBS 80, GBS 67, and GBS 322

In this example, fusion proteins of GBS antigens was used in the Active Maternal Immunization Assay with an isolate challenge of different GBS strains. In these experiments, the challenge dose for the different GBS strains was sufficient to kill approximately 70 - 90% of unimmunized pups and is equal to 10 x LD 50% (where LD 50% is the statistically derived Median Lethal Dose). The maternal mice were immunized according to the Active Maternal Immunization Assay schedule described above with the fusion proteins of a GBS 80 antigen with GBS 322 antigen in the GBS strains set forth in Table 14 below. Survival % was observed with the GBS fusion proteins. As shown in Table 14, in this particular challenge study, the survival rates for the fusion proteins in all of the GBS strains achieved up to 79%.

**Table 14: Active Maternal Immunization Assay using fusion proteins of GBS 80 with GBS 322**

| | **COH1 (III)** | | **CJB111 (V)** | | **515 (Ia)** | | **DK21 (II)** | | **2603 (V)** | |
|---|---|---|---|---|---|---|---|---|---|---|
| **GBS** | **Dead/ treated** | **Survival %** | **Dead/ treated** | **Survival %** | **Dead/ treated** | **Survival %** | **Dead/ treated** | **Survival %** | **Dead/ treated** | **Survival %** |
| 80N-322 | **16/40** | **60** | **8/39** | **79** | **12/28** | **57** | **7/19** | **63** | **8/37** | **78** |
| 80 | **4/24** | **83** | | | | | | | | |
| PBS | **35/40** | **12** | **27/35** | **23** | **32/39** | **18** | **31/40** | **22** | **33/40** | **17** |
| | | | | | | | | | | |
| 80-322 | **12/27** | **55** | | | | | | | **12/38** | **68** |
| | | | | | | | | | | |
| 80 | **0/33** | **100** | **28/40** | **30** | | | | | | |
| 322 | | | | | | | | | **1/16** | **94** |
| | | | | | | | | | | |
| PBS | **19/20** | **5** | **38/39** | **2** | **25/29** | **14** | | | **19/26** | **27** |

### Nucleic Acids

The invention also provides nucleic acid encoding the GBS antigens and/or the hybrid fusion polypeptides of the invention. Furthermore, the invention provides nucleic acid which can hybridise to these nucleic acids, preferably under "high stringency" conditions (*e.g*. 65°C in a 0.1xSSC, 0.5% SDS solution).

Polypeptides of the invention can be prepared by various means (*e.g*. recombinant expression, purification from cell culture, chemical synthesis, *etc*.) and in various forms (*e.g*. native, fusions, non-glycosylated, lipidated, *etc*.). They are preferably prepared in substantially pure form (*i.e*. substantially free from other GAS or host cell proteins).

Nucleic acid according to the invention can be prepared in many ways (*e.g*. by chemical synthesis, from genomic or cDNA libraries, from the organism itself, *etc.*) and can take various forms (*e.g*. single stranded, double stranded, vectors, probes, *etc*.). They are preferably prepared in substantially pure form (*i.e*. substantially free from other GBS or host cell nucleic acids).

The term "nucleic acid" includes DNA and RNA, and also their analogues, such as those containing modified backbones (*e.g*. phosphorothioates, *etc*.), and also peptide nucleic acids (PNA), *etc.* The invention includes nucleic acid comprising sequences complementary to those described above (*e.g*. for antisense or probing purposes).

The invention also provides a process for producing a polypeptide of the invention, comprising the step of culturing a host cell transformed with nucleic acid of the invention under conditions which induce polypeptide expression.

The invention provides a process for producing a polypeptide of the invention, comprising the step of synthesising at least part of the polypeptide by chemical means.

The invention provides a process for producing nucleic acid of the invention, comprising the step of amplifying nucleic acid using a primer-based amplification method (*e.g*. PCR).

The invention provides a process for producing nucleic acid of the invention, comprising the step of synthesising at least part of the nucleic acid by chemical means.

### Purification and Recombinant Expression

The GBS antigens of the invention may be isolated from *Streptococcus agalactiae,* or they may be recombinantly produced, for instance, in a heterologous host. Preferably, the GBS antigens are prepared using a heterologous host. The heterologous host may be prokaryotic (*e.g*. a bacterium) or eukaryotic. It is preferably *E.coli,* but other suitable hosts include *Bacillus subtilis, Vibrio cholerae, Salmonella typhi, Salmonella typhimurium, Neisseria lactamica, Neisseria cinerea, Mycobacteria* (*e.g. M. tuberculosis*), yeasts, *etc.*

Recombinant production of polypeptides is facilitated by adding a tag protein to the GBS antigen to be expressed as a fusion protein comprising the tag protein and the GBS antigen. Such tag proteins can facilitate purification, detection and stability of the expressed protein. Tag proteins suitable for use in the invention include a polyarginine tag (Arg-tag), polyhistidine tag (His-tag), FLAG-tag, Strep-tag, c-myc-tag, S-tag, calmodulin-binding peptide, cellulose-binding domain, SBP-tag,, chitin-binding domain, glutathione S-transferase-tag (GST), maltose-binding protein, transcription termination anti-terminiantion factor (NusA), *E. coli* thioredoxin (TrxA) and protein disulfide isomerase I (DsbA). Preferred tag proteins include His-tag and GST. A full discussion on the use of tag proteins can be found at Ref. 3.

After purification, the tag proteins may optionally be removed from the expressed fusion protein, i.e., by specifically tailored enzymatic treatments known in the art. Commonly used proteases include enterokinase, tobacco etch virus (TEV), thrombin, and factor Xₐ.

### GBS polysaccharides

The compositions of the invention may be further improved by including GBS polysaccharides. Preferably, the GBS antigen and the saccharide each contribute to the immunological response in a recipient. The combination is particularly advantageous where the saccharide and polypeptide provide protection from different GBS serotypes.

The combined antigens may be present as a simple combination where separate saccharide and polypeptide antigens are administered together, or they may be present as a conjugated combination, where the saccharide and polypeptide antigens are covalently linked to each other.

Thus the invention provides an immunogenic composition comprising (i) one or more GBS polypeptide antigens and (ii) one or more GBS saccharide antigens. The polypeptide and the polysaccharide may advantageously be covalently linked to each other to form a conjugate.

Between them, the combined polypeptide and saccharide antigens preferably cover (or provide protection from) two or more GBS serotypes (*e.g*. 2, 3, 4, 5, 6, 7, 8 or more serotypes). The serotypes of the polypeptide and saccharide antigens may or may not overlap. For example, the polypeptide might protect against serogroup II or V, while the saccharide protects against either serogroups Ia, Ib, or III. Preferred combinations protect against the following groups of serotypes: (1) serotypes Ia and Ib, (2) serotypes Ia and II, (3) serotypes Ia and III, (4) serotypes Ia and IV, (5) serotypes Ia and V, (6) serotypes Ia and VI, (7) serotypes Ia and VII, (8) serotypes Ia and VIII, (9) serotypes Ib and II, (10) serotypes Ib and III, (11) serotypes Ib and IV, (12) serotypes Ib and V, (13) serotypes Ib and VI, (14) serotypes Ib and VII, (15) serotypes Ib and VIII, 16) serotypes II and III, (17) serotypes II and IV, (18) serotypes II and V, (19) serotypes II and VI, (20) serotypes II and VII, (21) serotypes II and VII, (22) serotypes III and IV, (23) serotypes III and V, (24) serotypes III and VI, (25) serotypes III and VII, (26) serotypes III and VIII, (27) serotypes IV and V, (28) serotypes IV and VI, (29) serotypes IV and VII, (30) serotypes IV and VIII, (31) serotypes V and VI, (32) serotypes V and VII, (33) serotypes V and VIII, (34) serotypes VI and VII, (35) serotypes VI and VIII, and (36) serotypes VII and VIII.

Still more preferably, the combinations protect against the following groups of serotypes: (1) serotypes Ia and II, (2) serotypes Ia and V, (3) serotypes Ib and II, (4) serotypes Ib and V, (5) serotypes III and II, and (6) serotypes III and V. Most preferably, the combinations protect against serotypes III and V.

Protection against serotypes II and V is preferably provided by polypeptide antigens. Protection against serotypes Ia, Ib and/or III may be polypeptide or saccharide antigens.

In one embodiment, the immunogenic composition comprises a GBS saccharide antigen and at least two GBS polypeptide antigens or fragments thereof, wherein said GBS saccharide antigen comprises a saccharide selected from GBS serotype Ia, Ib, and III, and wherein said GBS polypeptide antigens comprise a combination of at least two polypeptide or a fragment thereof selected from the antigen group consisting of GBS 80, GBS 91, GBS 104, GBS 184, GBS 276, GBS 305, GBS 322, GBS 330, GBS 338, GBS 361, GBS 404, GBS 690, and GBS 691. Preferably, the combination includes one or more of GBS 80, GBS 104 and GBS 322. Still more preferably, the combination includes GBS 80 or a fragment thereof.

In certain embodiments, the compositions of the invention do not include a GBS polysaccharide. In certain embodiments, the combination does not include one or more of the GBS antigens selected from the group consisting of GBS 4, GBS 22, GBS 85, GBS 338 and GBS 361.

### Immunogenic compositions and medicaments

Compositions of the invention are preferably immunogenic compositions, and are more preferably vaccine compositions. The pH of the composition is preferably between 6 and 8, preferably about 7. The pH may be maintained by the use of a buffer. The composition may be sterile and/or pyrogen-free. The composition may be isotonic with respect to humans.

Vaccines according to the invention may either be prophylactic (i.e. to prevent infection) or therapeutic (i.e. to treat infection), but will typically be prophylactic. Accordingly, the invention includes a method for the therapeutic or prophylactic treatment of a *Streptococcus agalactiae* infection in an animal susceptible to streptococcal infection comprising administering to said animal a therapeutic or prophylactic amount of the immunogenic compositions of the invention.

The invention also provides a composition of the invention for use as a medicament. The medicament is preferably able to raise an immune response in a mammal (i.e. it is an immunogenic composition) and is more preferably a vaccine.

The invention also provides the use of the compositions of the invention in the manufacture of a medicament for raising an immune response in a mammal. The medicament is preferably a vaccine.

The invention also provides for a kit comprising a first component comprising a combination of GBS antigens.

The invention also provides a delivery device pre-filled with the immunogenic compositions of the invention.

The invention also provides a method for raising an immune response in a mammal comprising the step of administering an effective amount of a composition of the invention. The immune response is preferably protective and preferably involves antibodies and/or cell-mediated immunity. The method may raise a booster response.

The mammal is preferably a human. Where the vaccine is for prophylactic use, the human is preferably a female (either of child bearing age or a teenager). Alternatively, the human may be elderly (*e.g*., over the age of 50, 55, 60, 65, 70 or 75) and may have an underlying disease such as diabetes or cancer. Where the vaccine is for therapeutic use, the human is preferably a pregnant female or an elderly adult.

These uses and methods are preferably for the prevention and/or treatment of a disease caused by *Streptococcus agalactiae.* The compositions may also be effective against other streptococcal bacteria.

One way of checking efficacy of therapeutic treatment involves monitoring GBS infection after administration of the composition of the invention. One way of checking efficacy of prophylactic treatment involves monitoring immune responses against the GBS antigens in the compositions of the invention after administration of the composition.

Compositions of the invention will generally be administered directly to a patient. Direct delivery may be accomplished by parenteral injection (*e.g*. subcutaneously, intraperitoneally, intradermally, intravenously, intramuscularly, or to the interstitial space of a tissue), or by rectal, oral (*e.g*. tablet, spray), vaginal, topical, transdermal {*e.g*. see ref. 4} or transcutaneous {*e.g*. see refs. 5 & 6}, intranasal {*e.g*. see ref. 7}, ocular, aural, pulmonary or other mucosal administration.

The invention may be used to elicit systemic and/or mucosal immunity.

Dosage treatment can be a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.*

The compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g*. a lyophilised composition). The composition may be prepared for topical administration *e.g*. as an ointment, cream or powder. The composition may be prepared for oral administration *e.g*. as a tablet or capsule, as a spray, or as a syrup (optionally flavoured). The composition may be prepared for pulmonary administration *e.g*. as an inhaler, using a fine powder or a spray. The composition may be prepared as a suppository or pessary. The composition may be prepared for nasal, aural or ocular administration *e.g*. as drops. The composition may be in kit form, designed such that a combined composition is reconstituted just prior to administration to a patient. Such kits may comprise one or more antigens in liquid form and one or more lyophilised antigens.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

### Further Components of the Composition

The composition of the invention will typically, in addition to the components mentioned above, comprise one or more 'pharmaceutically acceptable carriers', which include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, and lipid aggregates (such as oil droplets or liposomes). Such carriers are well known to those of ordinary skill in the art. The vaccines may also contain diluents, such as water, saline, glycerol, *etc*. Additionally, auxiliary substances, such as wetting or emulsifying agents, pH buffering substances, and the like, may be present. A thorough discussion of pharmaceutically acceptable excipients is available in reference 8.

Vaccines of the invention may be administered in conjunction with other immunoregulatory agents. In particular, compositions will usually include an adjuvant.

Preferred further adjuvants include, but are not limited to, one or more of the following set forth below:

### A. Mineral Containing Compositions

Mineral containing compositions suitable for use as adjuvants in the invention include mineral salts, such as aluminium salts and calcium salts. The invention includes mineral salts such as hydroxides (*e.g*. oxyhydroxides), phosphates (*e.g*. hydroxyphoshpates, orthophosphates), sulphates, *etc.* {*e.g.* see chapters 8 & 9 of ref. 9}), or mixtures of different mineral compounds, with the compounds taking any suitable form (*e.g*. gel, crystalline, amorphous, *etc*.), and with adsorption being preferred. The mineral containing compositions may also be formulated as a particle of metal salt. See ref. 10.

### B. Oil-Emulsions

Oil-emulsion compositions suitable for use as adjuvants in the invention include squalene-water emulsions, such as MF59 (5% Squalene, 0.5% Tween 80, and 0.5% Span 85, formulated into submicron particles using a microfluidizer). See WO90/14837. See also, Frey et al., "Comparison of the safety, tolerability, and immunogenicity of a MF59-adjuvanted influenza vaccine and a non-adjuvanted influenza vaccine in non-elderly adults", Vaccine (2003) 21:4234 - 4237.

Particularly preferred adjuvants for use in the compositions are submicron oil-inwater emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyelthylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-*sn*-glycero-3-huydroxyphosphophoryloxy)-ethylamine (MTP-PE), for example, the submicron oil-in-water emulsion known as "MF59" (International Publication No. WO 90/14837; U.S. Patent Nos. 6,299,884 and 6,451,325, incorporated herein by reference in their entireties; and Ott et al., "MF59 -- Design and Evaluation of a Safe and Potent Adjuvant for Human Vaccines" in Vaccine Design: The Subunit and Adjuvant Approach (Powell, M.F. and Newman, M.J. eds.) Plenum Press, New York, 1995, pp. 277-296). MF59 contains 4-5% w/v Squalene (e.g., 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 µg/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an MF75 formulation that includes MTP, such as from 100-400 µg MTP-PE per dose.

Submicron oil-in-water emulsions, methods of making the same and immunostimulating agents, such as muramyl peptides, for use in the compositions, are described in detail in International Publication No. WO 90114837 and U.S. Patent Nos. 6,299,884 and 6,45 1,325, incorporated herein by reference in their entireties.

Complete Freund's adjuvant (CFA) and incomplete Freund's adjuvant (IFA) may also be used as adjuvants in the invention.

### C. Saponin Formulations

Saponin formulations, may also be used as adjuvants in the invention. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using High Performance Thin Layer Chromatography (HP-LC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in U.S. Patent No. 5,057,540. Saponin formulations may also comprise a sterol, such as cholesterol (see WO 96/33739).
Combinations of saponins and cholesterols can be used to form unique particles called Immunostimulating Complexs (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP 0 109 942, WO 96/11711 and WO 96/33739. Optionally, the ISCOMS may be devoid of additional detergent. See ref. 11.

A review of the development of saponin based adjuvants can be found at ref. 12.

### C. Virosomes and Virus Like Particles (VLPs)

Virosomes and Virus Like Particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO 03/024480, WO 03/024481, and Refs. 13, 14, 15 and 16. Virosomes are discussed further in, for example, Ref. 17

### D. Bacterial or Microbial Derivatives

Adjuvants suitable for use in the invention include bacterial or microbial derivatives such as:

### (1) Non-toxic derivatives ofenterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g*. RC-529. See Ref. 18.

### (2) Lipid A Derivatives

Lipid A derivatives include derivatives of lipid A from *Escherichia coli* such as OM-174. OM-174 is described for example in Ref. 19 and 20.

### (3) Immunostimulatory oligonucleotides

Immunostimulatory oligonucleotides suitable for use as adjuvants in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpG's can include nucleotide modifications/analogs such as phosphorothioate modifications and can be double-stranded or single-stranded. Optionally, the guanosine may be replaced with an analog such as 2'-deoxy-7-deazaguanosine. See ref. 21, WO 02/26757 and WO 99/62923 for examples of possible analog substitutions. The adjuvant effect of CpG oligonucleotides is further discussed in Refs. 22, 23, WO 98/40100, U.S. Patent No. 6,207,646, U.S. Patent No. 6,239,116, and U.S. Patent No. 6,429,199.

The CpG sequence may be directed to TLR9, such as the motif GTCGTT or TTCGTT. See ref. 24. The CpG sequence may be specific for inducing a Th1 immune response, such as a CpG-A ODN, or it may be more specific for inducing a B cell response, such a CpG-B ODN. CpG-A and CpG-B ODNs are discussed in refs. 25, 26 and WO 01/95935. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 27, 28,29 and WO 03/035836.

### (4) ADP-ribosylating toxins and detoxified derivatives thereof.

Bacterial ADP-ribosylating toxins and detoxified derivatives thereof may be used as adjuvants in the invention. Preferably, the protein is derived from *E. coli* (i.e., E. coli heat labile enterotoxin "LT), cholera ("CT"), or pertussis ("PT"). The use of detoxified ADP-ribosylating toxins as mucosal adjuvants is described in WO 95/17211 and as parenteral adjuvants in WO 98/42375. Preferably, the adjuvant is a detoxified LT mutant such as LT-K63.

### E. Human Immunomodulators

Human immunomodulators suitable for use as adjuvants in the invention include cytokines, such as interleukins (*e.g*. IL-1, IL-2, IL-4, IL-5, IL-6, IL-7, IL-12, *etc*.), interferons (*e.g*. interferon-y), macrophage colony stimulating factor, and tumor necrosis factor.

### F. Bioadhesives and Mucoadhesives

Bioadhesives and mucoadhesives may also be used as adjuvants in the invention. Suitable bioadhesives include esterified hyaluronic acid microspheres (Ref. 30) or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention. E.g., ref. 31.

### G. Microparticles

Microparticles may also be used as adjuvants in the invention. Microparticles (i.e. a particle of ∼100nm to ∼150µm in diameter, more preferably -200nm to ∼30µm in diameter, and most preferably ∼500nm to ∼10µm in diameter) formed from materials that are biodegradable and non-toxic (*e.g*. a poly(α-hydroxy acid), a polyhydroxybutyric acid, a polyorthoester, a polyanhydride, a polycaprolactone, *etc*.), with poly(lactide-co-glycolide) are preferred, optionally treated to have a negatively-charged surface (*e.g*. with SDS) or a positively-charged surface (*e.g*. with a cationic detergent, such as CTAB).

### H. Liposomes

Examples of liposome formulations suitable for use as adjuvants are described in U.S. Patent No. 6,090,406, U.S. Patent No. 5,916,588, and EP 0 626 169.

### I. Polyoxyethylene ether and Polyoxyethylene Ester Formulations

Adjuvants suitable for use in the invention include polyoxyethylene ethers and polyoxyethylene esters. Ref. 32. Such formulations further include polyoxyethylene sorbitan ester surfactants in combination with an octoxynol (Ref. 33) as well as polyoxyethylene alkyl ethers or ester surfactants in combination with at least one additional non-ionic surfactant such as an octoxynol (Ref. 34).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### J. Polyphosphazene (PCPP)

PCPP formulations are described, for example, in Ref. 35 and 36.

### K. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE).

### L. Imidazoguinolone Compounds.

Examples of imidazoquinolone compounds suitable for use adjuvants in the invention include Imiquamod and its homologues, described further in Ref. 37 and 38.

The invention may also comprise combinations of aspects of one or more of the adjuvants identified above. For example, the following adjuvant compositions may be used in the invention:
(1) a saponin and an oil-in-water emulsion (ref. 39);
(2) a saponin (e.g.., QS21) + a non-toxic LPS derivative (e.g., 3dMPL) (see WO 94/00153);
(3) a saponin (e.g.., QS21) + a non-toxic LPS derivative (e.g., 3dMPL) + a cholesterol;
(4) a saponin (*e.g*. QS21) + 3dMPL + IL-12 (optionally + a sterol) (Ref. 40);
(5) combinations of 3dMPL with, for example, QS21 and/or oil-in-water emulsions (Ref. 41);
(6) SAF, containing 10% Squalane, 0.4% Tween 80, 5% pluronic-block polymer L121, and thr-MDP, either microfluidized into a submicron emulsion or vortexed to generate a larger particle size emulsion.
(7) Ribi^{™} adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and
(8) one or more mineral salts (such as an aluminum salt) + a non-toxic derivative of LPS (such as 3dPML).

Aluminium salts and MF59 are preferred adjuvants for parenteral immunisation. Mutant bacterial toxins are preferred mucosal adjuvants.

The composition may include an antibiotic.

### Further antigens

The compositions of the invention may further comprise one or more additional non-GBS antigens, including additional bacterial, viral or parasitic antigens.

In another embodiment, the GBS antigen combinations of the invention are combined with one or more additional, non-GBS antigens suitable for use in a vaccine designed to protect elderly or immunocomprised individuals. For example, the GBS antigen combinations may be combined with an antigen derived from the group consisting of *Enterococcus faecalis, Staphylococcus aureus, Staphylococcus epidermis, Pseudomonas aeruginosa, Legionella pneumophila, Listeria monocytogenes, Neisseria meningitides,* influenza, and Parainfluenza virus ('PIV').

Where a saccharide or carbohydrate antigen is used, it is preferably conjugated to a carrier protein in order to enhance immunogenicity {*e.g*. refs. 42 to 51}. Preferred carrier proteins are bacterial toxins or toxoids, such as diphtheria or tetanus toxoids. The CRM₁₉₇ diphtheria toxoid is particularly preferred {52}. Other carrier polypeptides include the *N.meningitidis* outer membrane protein {53}, synthetic peptides {54, 55}, heat shock proteins {56, 57}, pertussis proteins {58, 59}, protein D from *H.influenzae* {60}, cytokines {61}, lymphokines, hormones, growth factors, toxin A or B from *C.difficile* {62}, iron-uptake proteins {63}, *etc.* Where a mixture comprises capsular saccharides from both serogroups A and C, it may be preferred that the ratio (w/w) of MenA saccharide:MenC saccharide is greater than 1 (*e.g.* 2:1, 3:1, 4:1, 5:1, 10:1 or higher). Different saccharides can be conjugated to the same or different type of carrier protein. Any suitable conjugation reaction can be used, with any suitable linker where necessary.

Toxic protein antigens may be detoxified where necessary *e.g*. detoxification of pertussis toxin by chemical and/or genetic means.

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens in the composition will typically be present at a concentration of at least 1µg/ml each. In general, the concentration of any given antigen will be sufficient to elicit an immune response against that antigen.

As an alternative to using protein antigens in the composition of the invention, nucleic acid encoding the antigen may be used {*e.g*. refs. 64 to 72}. Protein components of the compositions of the invention may thus be replaced by nucleic acid (preferably DNA *e.g*. in the form of a plasmid) that encodes the protein.

### Definitions

The term "comprising" means "including" as well as "consisting" *e.g*. a composition "comprising" X may consist exclusively of X or may include something additional *e.g*. X + Y.

The term "about" in relation to a numerical value x means, for example, *x*±1 0%.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 73. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in reference 74.

### REFERENCES (the contents of which are hereby incorporated by reference)

[1] Tettelin et al. (2002) Proc. Natl. Acad. Sci. USA*,*10.1073/pnas.182380799*.*
[2] International patent application WO02/34771.
3 Terpe et al., "Overview of tag protein fusions: from molecular and biochemical fundamentals to commercial systems", Appl Microbiol Biotechnol (2003) 60:523 - 533.
4. WO99/27961.
5. WO02/074244.
6. WO02/064162.
7. WO03/028760.
8. Gennaro (2000) Remington: The Science and Practice of Pharmacy. 20th ed., ISBN: 0683306472.
*9.* Vaccine design:the subunit and adjuvant approach (1995) Powell & Newman. ISBN 0-306-44867-X.
10. WO00/23105.
11. WO00/07621.
12. Barr, et al., "ISCOMs and other saponin based adjuvants", Advanced Drug Delivery Reviews (1998) 32:247-271. See also Sjolander, et al., "Uptake and adjuvant activity of orally delivered saponin and ISCOM vaccines", Advanced Drug Delivery Reviews (1998) 32:321-338.
13. Niikura et al., "Chimeric Recombinant Hepatitis E Virus-Like Particles as an Oral Vaccine Vehicle Presenting Foreign Epitopes", Virology (2002) 293:273 - 280.
14. Lenz et al., "Papillomarivurs-Like Particles Induce Acute Activation of Dendritic Cells", Journal of Immunology (2001) 5246 - 5355.
15. Pinto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327 - 338.
16. Gerber et al., "Human Papillomavrisu Virus-Like Particles Are Efficient Oral Immunogens when Coadministered with Escherichia coli Heat-Labile Entertoxin Mutant R192G or CpG", Journal of Virology (2001) 75(10):4752 - 4760.
17. Gluck et al., "New Technology Platforms in the Development of Vaccines for the Future", Vaccine (2002) 20:B10 -B16.
18. Johnson et al. (1999) Bioorg Med Chem Lett 9:2273-2278.
19. Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21:2485 - 2491.
20. Pajak, et al., "The Adjuvant OM-174 induces both the migration and maturation of murine dendritic cells in vivo", Vaccine (2003) 21:836 - 842.
21. Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393 - 2400.
22. Krieg, "CpG motifs: the active ingredient in bacterial extracts?", Nature Medicine (2003) 9(7): 831- 835.
23. McCluskie, et al., "Parenteral and mucosal prime-boost immunization strategies in mice with hepatitis B surface antigen and CpG DNA", FEMS Immunology and Medical Microbiology (2002) 32:179-185.
24. Kandimalla, et al., "Toll-like receptor 9: modulation of recognition and cytokine induction by novel synthetic CpG DNAs", Biochemical Society Transactions (2003) 31 (part 3): 654 - 658.
25. Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061 - 4068.
26. Krieg, "From A to Z on CpG", TRENDS in Immunology (2002) 23(2): 64 - 65.
27. Kandimalla, et al., "Secondary structures in CpG oligonucleotides affect immunostimulatory activity", BBRC (2003) 306:948 - 953.
28. Kandimalla, et al., "Toll-like receptor 9: modulation of recognition and cytokine induction by novel synthetic GpG DNAs", Biochemical Society Transactions (2003) 31(part 3):664 - 658.
29. Bhagat et al., "CpG penta- and hexadeoxyribonucleotides as potent immunomodulatory agents" BBRC (2003) 300:853 - 861.
30. Singh et al. (2001) J. Cont. Rele. 70:267-276.
31. WO99/27960.
32. WO99/52549.
33. WO01/21207.
34. WO01/21152.
35. Andrianov et al., "Preparation of hydrogel microspheres by coacervation of aqueous polyphophazene solutions", Biomaterials (1998) 19(1 - 3):109 -115.
36. Payne et al., "Protein Release from Polyphosphazene Matrices", Adv. Drug. Delivery Review (1998) 31(3):185 -196.
37. Stanley, "Imiquimod and the imidazoquinolones: mechanism of action and therapeutic potential" Clin Exp Dermatol (2002) 27(7):571 - 577.
38. Jones, "Resiquimod 3M", Curr Opin Investig Drugs (2003) 4(2):214 - 218.
39. WO99/11241.
40. WO98/57659.
41. European patent applications 0835318, 0735898 and 0761231.
42. Ramsay et al. (2001) Lancet 357(9251):195-196.
43. Lindberg (1999) Vaccine 17 Suppl 2:S28-36.
44. Buttery & Moxon (2000) JR Coll Physicians Lond 34:163-168.
45. Ahmad & Chapnick (1999) Infect Dis Clin North Am 13:113-133, vii.
46. Goldblatt (1998) J. Med. Microbiol. 47:563-567.
47. European patent 0 477 508.
48. US Patent No. 5,306,492.
49. International patent application WO98/42721.
*50.* Conjugate Vaccines (eds. Cruse et al.) ISBN 3805549326, particularly vol. 10:48-114.
51. Hermanson (1996) Bioconjugate Techniques ISBN: 0123423368 or 012342335X.
*52.* Research Disclosure, 453077 (Jan 2002)
53. EP-A-0372501
54. EP-A-0378881
55. EP-A-0427347
56. WO93/17712
57. WO94/03208
58. WO98/58668
59. EP-A-0471177
60. WO00/56360
61. WO91/01146
62. WO00/61761
63. WO01/72337
64. Robinson & Torres (1997) Seminars in Immunology 9:271-283.
65. Donnelly et al. (1997) Annu Rev Immunol 15:617-648.
66. Scott-Taylor & Dalgleish (2000) Expert Opin Investig Drugs 9:471-480.
67. Apostolopoulos & Plebanski (2000) Curr Opin Mol Ther 2:441-447.
68. Ilan (1999) Curr Opin Mol Ther 1:116-120.
69. Dubensky et al. (2000) Mol Med 6:723-732.
70. Robinson & Pertmer (2000) Adv Virus Res 55:1-74.
71. Donnelly et al. (2000) Am J Respir Crit Care Med 162(4 Pt 2):S 190-193.
72. Davis (1999) Mt. Sinai J. Med. 66:84-90.
*73.* Current Protocols in Molecular Biology (F.M. Ausubel et al., eds., 1987) Supplement 30.
74. Smith & Waterman (1981) Adv. Appl. Math. 2: 482-489.

## Claims

1. A composition that is isotonic with respect to humans and/or lyophilised, said composition comprising a combination of two or more GBS antigens, wherein said combination includes:
i) GBS 80 comprising the amino acid sequence of SEQ ID NO:2 or an immunogenic fragment thereof or a polypeptide sequence having 80% or greater sequence identity thereto; and
ii) at least one of
GBS 91 comprising the amino acid sequence of SEQ ID NO:11, an immunogenic fragment thereof or a polypeptide sequence having 80% or greater sequence identity thereto
GBS 104 comprising the amino acid sequence of SEQ ID NO:19, an immunogenic fragment thereof or a polypeptide sequence having 80% or greater sequence identity thereto
GBS 184 comprising the amino acid sequence of SEQ ID NO:24, an immunogenic fragment thereof or a polypeptide sequence having 80% or greater sequence identity thereto
GBS 276 comprising the amino acid sequence of SEQ ID NO:27, an immunogenic fragment thereof or a polypeptide sequence having 80% or greater sequence identity thereto
GBS 305 comprising the amino acid sequence of SEQ ID NO:32, an immunogenic fragment thereof or a polypeptide sequence having 80% or greater sequence identity thereto
GBS 322 comprising the amino acid sequence of SEQ ID NO:37, an immunogenic fragment thereof or a polypeptide sequence having 80% or greater sequence identity thereto,
GBS 330 comprising the amino acid sequence of SEQ ID NO:40, an immunogenic fragment thereof or a polypeptide sequence having 80% or greater sequence identity thereto,
GBS 338 comprising the amino acid sequence of SEQ ID NO:42, an immunogenic fragment thereof or a polypeptide sequence having 80% or greater sequence identity thereto,
GBS 361 comprising the amino acid sequence of SEQ ID NO:45, an immunogenic fragment thereof or a polypeptide sequence having 80% or greater sequence identity thereto,
GBS 404 comprising the amino acid sequence of SEQ ID NO:48, an immunogenic fragment thereof or a polypeptide sequence having 80% or greater sequence identity thereto,
GBS 690 comprising the amino acid sequence of SEQ ID NO:50, an immunogenic fragment thereof or a polypeptide sequence having 80% or greater sequence identity thereto,
GBS 691 comprising the amino acid sequence of SEQ ID NO:53, an immunogenic fragment thereof or a polypeptide sequence having 80% or greater sequence identity thereto.

2. The composition of claim 1, wherein said combination of GBS antigens demonstrates improved immunogenicity as measured by the Active Maternal Immunization Assay, wherein said Active Maternal Immunization Assay measures serum titers of female mice during an immunization schedule and percent survival rate of pups after challenge.

3. The composition of claim 2, wherein the percent survival rate of challenged pups is at least 2 percentage points higher than the percent survival rate of challenged pups from female mice immunized with a single non-GBS 80 antigen.

4. The composition of any one of claims 1-3, wherein said combination consists of two to thirteen GBS antigens selected from GBS 80, GBS 91, GBS104, GBS184, GBS276, GBS305, GBS322, GBS330, GBS338, GBS361, GBS404, GBS690 and GBS691, immunogenic fragments of said GBS antigens, or polypeptide sequences having 80% or greater sequence identity to said GBS antigens.

5. The composition of claim 4, wherein said combination consists of two, three, four or five of said GBS antigens.

6. The composition of any one of claims 1-5, wherein the fragment of GBS 80 comprises the amino acid sequence selected from the group consisting of SEQ ID NOS: 3, 4, 5, 6, 7, 8, and 9.

7. The composition of anyone of claims 1-6, said combination including GBS 80, GBS 104 and GBS 322, or immunogenic fragments of said GBS antigens, or polypeptide sequences having 80% or greater sequence identity to said GBS antigens.

8. The composition of any one of claims 1-7, wherein the GBS80 antigen, immunogenic fragment or polypeptide sequence having 80% or greater identity to GBS80 is expressed as a fusion polypeptide with the at least one GBS antigen selected from GBS 91, GBS 104, GBS 184, GBS 276, GBS 305, GBS 322, GBS 330, GBS 338, GBS 361, GBS 404, GBS 690, or GBS 691, immunogenic fragments thereof or polypeptide sequences having 80% or greater identity to said GBS antigens.

9. The composition of claim 8, comprising a fusion polypeptide consisting essentially of the GBS 80 antigen and the GBS 322 antigen, or immunogenic fragments thereof or polypeptide sequences having 80% or greater identity to said GBS antigens.

10. A composition according to any one of claims 1-9 for use in therapy.

11. A composition according to any one of claims 1-9 for the therapeutic or prophylactic treatment of GBS infection.

12. Use of a composition of any one of claims 1-9 for the manufacture of a medicament for treatment or prevention of GBS infection.

## Patentansprüche

1. Zusammensetzung, die in Bezug auf Menschen isotonisch und/oder lyophilisiert ist, wobei die Zusammensetzung eine Kombination von zwei oder mehreren GBS-Antigenen umfasst, wobei die Kombination folgendes umfasst:
i) GBS 80, das die Aminosäuresequenz von SEQ ID NO:2, eines immunogenen Fragments davon oder eine Polypeptidsequenz, die 80% oder mehr Sequenzidentität zu dieser aufweist, umfasst; und
ii) mindestens eines der folgenden:
GBS 91, das die Aminosäuresequenz von SEQ ID NO:11, ein immunogenes Fragment davon oder eine Polypeptidsequenz mit 80% oder mehr Sequenzidentität dazu umfasst;
GBS 104, das die Aminosäuresequenz von SEQ ID NO:19, ein immunogenes Fragment davon oder eine Polypeptidsequenz mit 80% oder mehr Sequenzidentität dazu umfasst;
GBS 184, das die Aminosäuresequenz von SEQ ID NO:24, ein immunogenes Fragment davon oder eine Polypeptidsequenz mit 80% oder mehr Sequenzidentität dazu umfasst;
GBS 276, das die Aminosäuresequenz von SEQ ID NO:27, ein immunogenes Fragment davon oder eine Polypeptidsequenz mit 80% oder mehr Sequenzidentität dazu umfasst;
GBS 305, das die Aminosäuresequenz von SEQ ID NO:32, ein immunogenes Fragment davon oder eine Polypeptidsequenz mit 80% oder mehr Sequenzidentität dazu umfasst;
GBS 322, das die Aminosäuresequenz von SEQ ID NO:37, ein immunogenes Fragment davon oder eine Polypeptidsequenz mit 80% oder mehr Sequenzidentität dazu umfasst;
GBS 330, das die Aminosäuresequenz von SEQ ID NO:40, ein immunogenes Fragment davon oder eine Polypeptidsequenz mit 80% oder mehr Sequenzidentität dazu umfasst;
GBS 338, das die Aminosäuresequenz von SEQ ID NO:42, ein immunogenes Fragment davon oder eine Polypeptidsequenz mit 80% oder mehr Sequenzidentität dazu umfasst;
GBS 361, das die Aminosäuresequenz von SEQ ID NO:45, ein immunogenes Fragment davon oder eine Polypeptidsequenz mit 80% oder mehr Sequenzidentität dazu umfasst;
GBS 404, das die Aminosäuresequenz von SEQ ID NO:48, ein immunogenes Fragment davon oder eine Polypeptidsequenz mit 80% oder mehr Sequenzidentität dazu umfasst;
GBS 690, das die Aminosäuresequenz von SEQ ID NO:50, ein immunogenes Fragment davon oder eine Polypeptidsequenz mit 80% oder mehr Sequenzidentität dazu umfasst;
GBS 691, das die Aminosäuresequenz von SEQ ID NO:53, ein immunogenes Fragment davon oder eine Polypeptidsequenz mit 80% oder mehr Sequenzidentität dazu umfasst.

2. Zusammensetzung nach Anspruch 1, wobei die Kombination von GBS-Antigenen eine verbesserte Immunogenität zeigt, wie sie durch den Assay auf aktive mütterliche Immunisierung (Active Maternal Immunization Assay) gemessen wird, wobei der Assay auf aktive mütterliche Immunisierung die Serum-Titer von weiblichen Mäusen während eines Immunisierungsprotokolls und die prozentuale Überlebensrate von Jungtieren nach Challenge bestimmt.

3. Zusammensetzung nach Anspruch 2, wobei die prozentuale Überlebensrate von Jungtieren, die einen Challenge durchlaufen haben, mindestens 2 Prozentpunkte höher ist als die prozentuale Überlebensrate von Jungtieren, die einen Challenge durchlaufen haben und von weiblichen Mäusen stammen, welche mit einem einzelnen Antigen immunisiert wurden, bei dem es sich nicht um GBS 80 handelt.

4. Zusammensetzung nach einem der Ansprüche 1-3, wobei die Kombination aus zwei bis dreizehn GBS-Antigenen besteht, die ausgewählt sind aus der Gruppe bestehend aus GBS 80, GBS 91, GBS 104, GBS184, GBS 276, GBS 305, GBS 322, GBS 330, GBS 338, GBS 361, GBS 404, GBS 690 und GBS 691, immunogenen Fragmenten dieser GBS-Antigene oder Polypeptidsequenzen mit 80% oder mehr Sequenzidentität zu diesen GBS-Antigenen.

5. Zusammensetzung nach Anspruch 4, wobei die Kombination aus zwei, drei, vier oder fünf der GBS-Antigene besteht.

6. Zusammensetzung nach einem der Ansprüche 1-5, wobei das Fragment von GBS80 die Aminosäuresequenz umfasst, die ausgewählt ist aus der Gruppe bestehend aus SEQ ID NO:3, 4, 5, 6, 7, 8 und 9.

7. Zusammensetzung nach einem der Ansprüche 1-6, wobei die Kombination GBS 80, GBS 104 und GBS 322 oder immunogene Fragmente dieser GBS-Antigene oder Polypeptidsequenzen mit 80% oder mehr Sequenzidentität zu den GBS-Antigenen umfasst.

8. Zusammensetzung nach einem der Ansprüche 1-7, wobei das GBS 80-Antigen, das immunogene Fragment oder die Polypeptidsequenz mit 80% oder mehr Sequenzidentität zu GBS 80 als Fusions-Polypeptid mit mindestens einem GBS-Antigen exprimiert wird, das ausgewählt ist aus GBS 91, GBS 104, GBS 184, GBS 276, GBS 305, GBS 322, GBS 330, GBS 338, GBS 361, GBS 404, GBS 690 und GBS 691, immunogenen Fragmenten davon oder Polypeptidsequenzen mit 80% oder mehr Identität zu diesen GBS-Antigenen.

9. Zusammensetzung nach Anspruch 8, die ein Fusions-Polypeptid umfasst, welches im Wesentlichen aus dem GBS 80-Antigen und dem GBS 322-Antigen oder aus immunogenen Fragmenten davon oder aus Polypeptidsequenzen mit 80% oder mehr Identität zu diesen GBS-Antigenen besteht.

10. Zusammensetzung nach einem der Ansprüche 1-9 zur Verwendung in der Therapie.

11. Zusammensetzung nach einem der Ansprüche 1-9 zur therapeutischen oder prophylaktischen Behandlung einer GBS-Infektion.

12. Verwendung einer Zusammensetzung nach einem der Ansprüche 1-9 zur Herstellung eines Medikaments zur Behandlung oder Vermeidung einer GBS-Infektion.

## Revendications

1. Composition qui est isotonique par rapport aux êtres humains et/ou lyophilisée, ladite composition comprenant une combinaison de deux antigènes GBS ou plus, où ladite combinaison comprend :
i) GBS 80 comprenant la séquence d'acides aminés de SÉQ ID NO : 2 ou un fragment immunogène de celle-ci ou une séquence polypeptidique présentant 80 % ou plus d'identité de séquence avec celle-ci ; et
ii) au moins l'un des
GBS 91 comprenant la séquence d'acides aminés de SÉQ ID NO : 11, un fragment immunogène de celle-ci ou une séquence polypeptidique présentant 80 % ou plus d'identité de séquence avec celle-ci
GBS 104 comprenant la séquence d'acides aminés de SÉQ ID NO : 19, un fragment immunogène de celle-ci ou une séquence polypeptidique présentant 80 % ou plus d'identité de séquence avec celle-ci
GBS 184 comprenant la séquence d'acides aminés de SÉQ ID NO : 24, un fragment immunogène de celle-ci ou une séquence polypeptidique présentant 80 % ou plus d'identité de séquence avec celle-ci
GBS 276 comprenant la séquence d'acides aminés de SÉQ ID NO : 27, un fragment immunogène de celle-ci ou une séquence polypeptidique présentant 80 % ou plus d'identité de séquence avec celle-ci
GBS 305 comprenant la séquence d'acides aminés de SÉQ ID NO : 32, un fragment immunogène de celle-ci ou une séquence polypeptidique présentant 80 % ou plus d'identité de séquence avec celle-ci
GBS 322 comprenant la séquence d'acides aminés de SÉQ ID NO : 37, un fragment immunogène de celle-ci ou une séquence polypeptidique présentant 80 % ou plus d'identité de séquence avec celle-ci
GBS 330 comprenant la séquence d'acides aminés de SÉQ ID NO : 40, un fragment immunogène de celle-ci ou une séquence polypeptidique présentant 80 % ou plus d'identité de séquence avec celle-ci
GBS 338 comprenant la séquence d'acides aminés de SÉQ ID NO : 42, un fragment immunogène de celle-ci ou une séquence polypeptidique présentant 80 % ou plus d'identité de séquence avec celle-ci
GBS 361 comprenant la séquence d'acides aminés de SÉQ ID NO : 45, un fragment immunogène de celle-ci ou une séquence polypeptidique présentant 80 % ou plus d'identité de séquence avec celle-ci
GBS 404 comprenant la séquence d'acides aminés de SÉQ ID NO : 48, un fragment immunogène de celle-ci ou une séquence polypeptidique présentant 80 % ou plus d'identité de séquence avec celle-ci
GBS 690 comprenant la séquence d'acides aminés de SÉQ ID NO : 50, un fragment immunogène de celle-ci ou une séquence polypeptidique présentant 80 % ou plus d'identité de séquence avec celle-ci
GBS 691 comprenant la séquence d'acides aminés de SÉQ ID NO : 53, un fragment immunogène de celle-ci ou une séquence polypeptidique présentant 80 % ou plus d'identité de séquence avec celle-ci.

2. Composition selon la revendication 1, où ladite combinaison d'antigènes GBS démontre une amélioration de l'immunogénicité mesurée par le test d'immunisation maternelle active, où ledit test d'immunisation maternelle active mesure les titres sériques de souris femelles au cours d'un programme d'immunisation et le pourcentage du taux de survie des souriceaux après provocation.

3. Composition selon la revendication 2, où le pourcentage du taux de survie des souriceaux provoqués est d'au moins 2 points de pourcentage supérieur au pourcentage du taux de survie des souriceaux provoqués provenant de souris femelles immunisées avec un seul antigène qui n'est pas le GBS 80.

4. Composition selon l'une quelconque des revendications 1 à 3, dans laquelle ladite combinaison consiste en deux à treize antigènes GBS choisis parmi GBS 80, GBS 91, GBS 104, GBS 184, GBS 276, GBS 305, GBS 322, GBS 330, GBS 338, GBS 361, GBS 404, GBS 690 et GBS 691, des fragments immunogènes desdits antigènes GBS ou des séquences polypeptidiques présentant 80 % ou plus d'identité de séquence avec lesdits antigènes GBS.

5. Composition selon la revendication 4, dans laquelle ladite combinaison consiste en deux, trois, quatre ou cinq desdits antigènes GBS.

6. Composition selon l'une quelconque des revendications 1 à 5, dans laquelle le fragment de GBS 80 comprend la séquence d'acides aminés choisie dans le groupe constitué des SÉQ ID NO : 3, 4, 5, 6, 7, 8 et 9.

7. Composition selon l'une quelconque des revendications 1 à 6, ladite combinaison comprenant GBS 80, GBS 104 et GBS 322 ou des fragments immunogènes desdits antigènes GBS ou des séquences polypeptidiques présentant 80 % ou plus d'identité de séquence avec lesdits antigènes GBS.

8. Composition selon l'une quelconque des revendications 1 à 7, dans laquelle l'antigène GBS 80, un fragment immunogène ou une séquence polypeptidique présentant 80 % ou plus d'identité avec GBS 80 est exprimé sous la forme d'un polypeptide de fusion avec le au moins un antigène GBS choisi parmi GBS 91, GBS 104, GBS 184, GBS 276, GBS 305, GBS 322, GBS 330, GBS 338, GBS 361, GBS 404, GBS 690 et GBS 691, des fragments immunogènes de ceux-ci ou des séquences polypeptidiques présentant 80 % ou plus d'identité de séquence avec lesdits antigènes GBS.

9. Composition selon la revendication 8, comprenant un polypeptide de fusion consistant essentiellement en l'antigène GBS 80 et l'antigène GBS 322 ou des fragments immunogènes de ceux-ci ou des séquences polypeptidiques présentant 80 % ou plus d'identité de séquence avec lesdits antigènes GBS.

10. Composition selon l'une quelconque des revendications 1 à 9, en vue d'une utilisation en thérapie.

11. Composition selon l'une quelconque des revendications 1 à 9, destinée au traitement thérapeutique ou prophylactique d'une infection GBS.

12. Utilisation d'une composition selon l'une quelconque des revendications 1 à 9, pour la fabrication d'un médicament destiné au traitement ou à la prévention d'une infection à GBS.
